(19) Europäisches Patentamt
European Patent Office
Office européen des brevets

(11) Veröffentlichungsnummer: **0 129 051**
**B1**

(12) **EUROPÄISCHE PATENTSCHRIFT**

(45) Veröffentlichungstag der Patentschrift: **04.07.90**

(21) Anmeldenummer: **84105450.5**

(22) Anmeldetag: **14.05.84**

(51) Int. Cl.$^5$: **C 07 D 401/04,**
C 07 D 403/04,
C 07 D 401/14,
C 07 D 491/056,
A 61 K 31/44, A 61 K 31/415
// A61K31/405

# (54) Substituierte Indole.

(30) Priorität: **17.05.83 US 495367**

(43) Veröffentlichungstag der Anmeldung:
**27.12.84 Patentblatt 84/52**

(45) Bekanntmachung des Hinweises auf die Patenterteilung:
**04.07.90 Patentblatt 90/27**

(84) Benannte Vertragsstaaten:
**AT BE CH DE FR GB IT LI LU NL SE**

(56) Entgegenhaltungen:
**EP-A-0 073 663**
**EP-A-0 080 154**
**CH-A- 511 838**

**DIE PHARMAZIE, 23. Jahrgang, Heft 10, Oktober 1968, Seiten 557-560, VEB Verlag Volk und Gesundheit, Berlin, DE; G. BUCHMANN et al.: "Zur Synthese von 2-Pyridylindolen"**

(73) Patentinhaber: **CIBA-GEIGY AG**
**Klybeckstrasse 141**
**CH-4002 Basel (CH)**

(72) Erfinder: **Renfroe, Harris Burt, Dr.**
**12 Stonehedge Drive**
**West Nyack New York 10994 (US)**

(74) Vertreter: **Zumstein, Fritz, Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 129 051 B1

**Beschreibung**

Die Erfindung betrifft neue 2-Heteroarylindole und Verfahren zu ihrer Herstellung.

Die Erfindung betrifft insbesondere 2-(Pyridyl und Imidazolyl)-Indole der Formel I

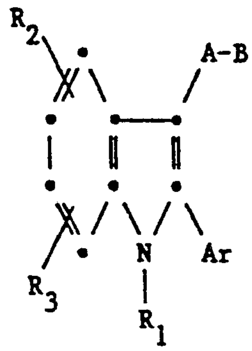

(I),

worin $R_1$ Wasserstoff oder Niederalkyl bedeutet, Ar jeweils für unsubstituiertes oder durch Niederalkyl, Carboxy, Niederalkoxycarbonyl oder Carbamoyl substituiertes 3-Pyridyl oder 1-Imidazolyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeuten, oder $R_2$ und $R_3$ zusammen, an benachbarten Kohlenstoffatomen, Niederalkylendioxy sind;

A Alkylen mit 3 bis 12 Kohlenstoffatomen, wobei die Anzahl der zwischen dem Indolkern und der Gruppe B stehenden Kohlenstoffatome 3 bis 12 beträgt, Alkenylenen oder Alkinylen mit jeweils 2 bis 12 Kohlenstoffatomen, Niederalkylenphenylen-nieder-(alkylen oder alkenylen), Niederalkylen-phenylen, Niederalkylen-(thio oder oxy)-niederalkylen, Niederalkylen-(thio oder oxy)-phenylen oder Niederalkylenphenylen-(thio oder oxy)-niederalkylen bedeutet und

B für Carboxy, verestertes Carboxy in Form von pharmazeutisch verwendbaren Estern, Carbamoyl, Mono- oder Diniederalkylcarbamoyl, Hydroxymethyl, Cyan, Hydroxycarbamoyl, 5-Tetrazolyl oder Formyl steht; ihre Imidazolyl- oder Pyridyl-N-Oxide, wobei die mit "nieder" bezeichneten Gruppen höhstens 7 Kohlenstoffatome enthalten, und ihre Salze, insbesondere ihre pharmazeutisch verwendbaren Salze, sowie Verfahren zu ihrer Herstellung, pharmazeutische Präparate enthaltend diese Verbindungen und ihre Verwendung zur Herstellung pharmazeutischer Präparate oder als pharmakologisch wirksame Verbindungen.

Die vor-, sowie nachstehend verwendeten Allgemeinbegriffe haben im Rahmen der vorliegenden Erfindung die folgenden Bedeutungen:

Ein Alkylenrest bedeutet Alkylen mit 3 bis 12 Kohlenstoffatomen, das geradkettig oder verzweigt sein kann, und vorzugsweise für Propylen, Butylen, Pentylen, Hexylen oder Heptylen steht, wobei die genannten Reste unsubstituiert oder durch eine oder mehrere Niederalkylgruppen substituiert sind, mit der Massgabe, dass die Summe der Kohlenstoffatome nicht grösser als 12 ist.

Der Ausdruck Alkenylen bedeutet einen Alkenylenrest mit 2 bis 12 Kohlenstoffatomen, der geradkettig oder verzweigt sein kann, und vorzugsweise für Propenylen, 1- oder 2-Butenylen, 1- oder 2-Pentenylen, 1-, 2- oder 3-Hexenylen oder 1-, 2-, 3- oder 4-Heptenylen steht. Die genannten Reste sind unsubstituiert oder durch eine oder mehrere Niederalkylgruppen substituiert, mit der Massgabe, dass die Summe der Kohlenstoffatome 12 nicht übersteigt.

Der Begriff Alkinylen bezeichnet einen Alkinylenrest mit 2 bis 12 Kohlenstoffatomen, der geradkettig oder verzweigt sein kann, und vorzugsweise für Propinylen, 1- oder 2-Butinylen, 1- oder 2-Pentinylen, 1-, 2- oder 3-Hexinylen oder 1-, 2-, 3- oder 4-Heptinylen steht. Diese Reste sind unsubstituiert oder durch eine oder mehrere Niederalkylgruppen substituiert, wobei die Summe der Kohlenstoffatome 12 nicht übersteigt.

Der Ausdruck Phenylen bedeutet 1,2-, 1,3- und vorzugsweise 1,4-Phenylen.

Der Ausdruck "nieder" umfasst in den vor- oder nachstehend genannten organischen Gruppen, Resten oder Verbindungen insbesondere solche mit höchstens 7, vorzugsweise 4 und vor allem 1,2 oder 3 Kohlenstoffatomen.

Niederalkylen-phenylen, Niederalkylen-phenylen-nieder-(alkylen oder alkenylen) oder Niederalkylen-(thio oder oxy)-phenylen enthalten in jedem Alkylenrest vorzugsweise 1 bis 4 und insbesondere 1 oder 2 Kohlenstoffatome, und in jedem Alkenylenrest vorzugsweise 2 bis 4 Kohlenstoffatome. Die Niederalkeylen- oder Niederalkenylenreste können geradkettig oder verzweigt sein.

Eine Niederalkylen-(thio oder oxy)-niederalkylengruppe kann geradkettig oder verzweigt sein und insgesamt 2 bis 12, vorzugsweise 2 bis 8, Kohlenstoffatome haben.

Niederalkyl enthält vorzugsweise 1 bis 4 Kohlenstoffatome und steht z.B. für Aethyl, Propyl oder Butyl und insbesondere für Methyl.

Niederalkylen enthält vorzugsweise 1 bis 4 Kohlenstoffatome und steht z.B. für Methylen, Aethylen, 1,2- oder 1,3-Propylen oder 1,2-, 1,3- oder 1,4-Butylen.

Niederalkylendioxy bedeutet vorzugsweise Aethylendioxy oder Methylendioxy.

Niederalkoxy enthält vorzugsweise 1 bis 4 Kohlenstoffatome und ist z.B. Aethoxy, Propoxy oder insbesondere Methoxy. Eine Niederalkyl(thio, -sulfinyl oder -sulfonyl)-Gruppe ist vorzugsweise Methylthio, Methylsulfinyl bzw. Methylsulfonyl.

Niederalkoxycarbonyl enthält vorzugsweise 1 bis 4 Kohlenstoffatome im Alkoxyteil und bedeutet z.B. Methoxycarbonyl, Propoxycarbonyl oder Isopropoxycarbonyl und insbesondere Aethoxycarbonyl. Mono-(niederalkyl)-carbamoyl hat vorzugsweise 1 bis 4 Kohlenstoffatome im Alkylteil und ist z.B. N-Methylcarbamoyl, N-Propylcarbamoyl oder insbesondere N-Aethylcarbamoyl. Di-(niederalkyl)-carbamoyl enthält vorzugsweise 1 bis 4 Kohlenstoffatome in jeder Niederalkylgruppe und steht z.B. für N,N-Dimethylcarbamoyl, N-Methyl-N-äthylcarbamoyl und insbesondere für N,N-Diäthylcarbamoyl.

Halogen ist vorzugsweise Fluor oder Chlor, kann aber auch Brom oder Jod sein.

Unter verestertem Carboxy sind vorzugsweise solche Ester zu verstehen, die pharmazeutisch verwendbar sind, insbesondere solche, die durch Solvolyse oder unter physiologischen Bedingungen in die entsprechenden freien Säuren überführbar sind, z.B. Niederalkoxycarbonyl, (Amino-, Mono- oder Di-niederalkylamino)-substituiertes Niederalkoxycarbonyl, Carboxy-substituiertes Niederalkoxycarbonyl, z.B. α-Carboxy-substituiertes Niederalkoxycarbonyl, Niederalkoxycarbonyl-substituiertes Niederalkoxycarbonyl, z.B. α-Niederalkoxycarbonyl-substituiertes Niederalkoxycarbonyl, Aryl-substituiertes Niederalkoxycarbonyl, z.B. gegebenenfalls substituiertes Benzyloxycarbonyl oder Pyridylmethoxycarbonyl, (Hydroxy-, Niederalkanoyloxy- oder Niederalkoxy)-substituiertes Niederalkoxycarbonyl, z.B. Pivaloyloxymethoxcarbonyl, (Hydroxy-, Niederalkanoyloxy- oder Niederalkoxy)-substituiertes Niederalkoxymethoxycarbonyl, Bicycloalkoxycarbonyl-substituiertes Niederalkoxycarbonyl, z.B. Bicyclo[2,2,1]-heptyloxycarbonyl-substituiertes Niederalkoxycarbonyl, insbesondere Bicyclo[2,2,1]-heptyloxycarbonyl-substituiertes Methoxy, z.B. Bornyloxycarbonylmethoxycarbonyl, 3-Phthalidoxycarbonyl, (Niederalkyl-, Niederalkoxy-, Halogen)-substituiertes 3-Phthalidoxycarbonyl, Niederalkoxycarbonyloxy-niederalkoxycarbonyl, z.B. 1-(Methoxy- oder Aethoxycarbonyloxy)-äthoxycarbonyl, Aryloxycarbonyl, z.B. Phenoxycarbonyl oder Phenoxycarbonyl, das vorteilhaft in der ortho-Position durch Carboxy oder Niederalkoxycarbonyl substituiert ist.

Salze sind vorzugsweise pharmazeutisch verwendbare Salze, insbesondere Metall- oder Ammoniumsalze der genannten Verbindungen der Formel I, welche eine freie Carboxygruppe besitzen, vor allem Alkalimetall- oder Erdalkalimetallsalze, z.B. Natrium-, Kalium-, Magnesium- oder Calciumsalze; in erster Linie leicht kristallisierende Ammoniumsalze, die abgeleitet sind von Ammoniak oder organischen Aminen, z.B. Mono-, Di- oder Tri-nieder-(alkyl oder cycloalkyl)-aminen, Tri(hydroxyniederalkyl)-aminen, Niederalkylendiaminen oder (Hydroxy-niederalkyl- oder Aryl-niederalkyl)-niederalkylammonium-Hydroxiden, z.B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten Verbindungen der Formel I bilden Säureadditionssalze. Diese werden vorzugsweise mit solchen anorganischen oder organischen Säuren, die pharmazeutisch verwendbare Säureadditionssalze ergeben, hergestellt. Solche Säuren sind z.B. starke Mineralsäuren, wie Halogenwasserstoffsäuren, z.B. Chlorwasserstoff- oder Bromwasserstoffsäure, Schwefel-, Phosphor-, Salpeter- oder Perchlorsäure; oder organische Säuren, insbesondere aliphatische oder aromatische Carbon- oder Sulfonsäuren, z.B. Ameisen-, Essig-, Pripion-, Bernstein-, Glykol-, Milch-, Aepfel-, Wein-, Glucon-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Benzolsulfon-, p-Toluolsulfon-, Naphthalinsulfon-, Sulfanil- oder Cyclohexylsulfaminsäure; oder andere saure organische Substanzen, wie z.B. die Ascorbinsäure.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt sind.

Die Verbindungen der Erfindung zeigen wertvolle pharmakologische Eigenschaften, z.B. kardiovaskuläre Effekte, indem sie die Thromboxan-Auschüttung in Säugern selektiv hemmen. Diese Hemmung kommt durch selektive Herabsetzung des Thromboxan-Synthetase-Spiegels zustande. Die Verbindungen sind daher nützlich zur Behandlung von solchen Krankheiten bei Säugern, die auf eine Hemmung der Thromboxan-Synthetase ansprechen. Solche Krankheiten sind in erster Linie kardiovaskuläre Störungen wie Thrombose, Arteriosklerose, Koronarkrämpfe, cerebrale ischämische Anfälle, Migräne und andere vaskuläre Kopfschmerzen, Myokardinfarkt, Angina pectoris und Hypertension.

Diese Wirkungen können durch in vito-Versuche oder in vivo-Tierversuche, vorzugsweise an Säugetieren, z.B. an Meerschweinchen, Mäusen, Ratten, Katzen, Hunden oder Affen, nachgewiesen werden. Die genannten Verbindungen können den Versuchstieren enteral oder parenteral, vorzugsweise oral oder subkutan, intravenös oder intraperitoneal, z.B. mittels Gelatine-Kapseln oder in Form von Stärke enthaltenden Suspension oder wässerigen Lösungen, verabreicht werden. Die verwendete Dosis kann in einem Bereich von ungefähr 0,01 bis 100 mg/kg/Tag, vorzugsweise von ungefähr 0,05 bis 50 mg/kg/Tag und insbesondere von ungefähr 0,1 bis 25 mg/kg/Tag liegen.

Die in vitro-Hemmung des Thromboxan-Synthetase-Enzyms kann analog der Methode von

Sun[Biochem. Biophys. Res. Comm. *74*, 1432 (1977)] nachgewiesen werden. Das Testverfahren wird wie folgt durchgeführt:

$^{14}C$-Arachidonsäure wird mit einem Enzymgemisch-Präparat, bestehend aus solubilisierter und partiell gereinigter Prostaglandin-cyclooxygenase von Schaf-Samenblasen und aus einem rohen Mikrosomen-Präparat von Thromboxan-Synthetase, das von lysierten menschlichen Blutplättchen stammt, inkubiert. Die Testverbindung (gelöst in einem Puffer, oder, falls nötig, in wenig Aethanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch aus Prostaglandin $F_2\alpha$ und $F_2\beta$ [$PGF_2\alpha+\beta$] reduziert. Die radioaktiven Produkte und das überschüssige Substrat werden mit Essigsäureäthylester extrahiert und der extrakt zur Trockene eingedampft. Der Rückstand wird in Aceton gelöst, auf Dünnschichtplatten aufgetragen und mit dem Lösungsmittelsystem Toluol/Aceton/Eisessig [100:100:3 (Volumen)] chromatographiert. Die radioaktiven Zonen werden lokalisiert. Die Zonen von Thromboxan $B_2$ ($TxB_2$) und $PGF_2\alpha+\beta$ werden in Szintillationsröhrchen für Flüssigkeiten übertragen und gezählt. Der Quotient aus den Zahlenwerten von $TxB_2$ und $PGF_2\alpha+\beta$ wird für jede Konzentration der Testverbindung berechnet und daraus die $IC_{50}$-Werte graphisch ermittelt. Dieser Wert entspricht der Konzentration an Testverbindung, bei welcher der Quotient $TxB_2/PGF_2\alpha+\beta$ auf 50% des Kontrollwertes reduziert wird.

Die in vitro-Wirkung der Verbindungen auf Prostaglandin-cyclooxygenase wird gemäss einer Modifikation der Methode von Takeguchi et al.[Biochemistry *10*, 2372 (1971)] gemessen. Das Testverfahren läuft wie folgt ab:

Lyophilisierte Samenblasen-Mikrosomen von Schafen werden als Prostaglandin-synthetisierendes Enzympräparat verwendet. Es wird die Umwandlung von $^{14}C$-Arachidonsäure in $PGE_2$ gemessen. Die Testverbindungen (gelöst in einem Puffer, oder, wenn nötig, in wenig Aethanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen in Szintillationsröhrichen für Flüssigkeiten übertragen und ihre Radioaktivität gezählt. Zur Bestimmung der $IC_{50}$-Werte für die Hemmung wird graphische die Konzentration an Testverbindung ermittelt, bei welcher die Menge des synthetisierten $PGE_2$ um 50% reduziert wird.

Die in-vitro-Wirkung auf Prostacyclin-($PGI_2$)-Synthetase wird analog der Methode von Sun et al., Prostaglandins *14*, 1055 (1977) bestimmt. Das Testverfahren läuft wie folgt ab:

$^{14}C$-Arachidonsäure wird mit einem Enzymgemisch inkubiert, das aus solubilisierter und partiell gereinigter Prostaglandin-cyclooxy-genase von Schaf-Samenblasen sowie aus roher $PGI_2$-Synthetase besteht, die in Form einer Mirkosomen-Fraktion, gewonnen aus Rinderaorten, vorliegt.

Die Testverbindung (gelöst in einem Puffer, oder, wenn nötig, in wenig Aethanol) wird in das Inkubationsmedium gegeben. Das Reaktionsgemisch wird in 100 mMolarem Tris HCl (pH 7,5) 30 Minuten bei 37°C inkubiert, auf den pH-Wert 3 angesäuert und mit Essigsäureäthylester extrahiert. Der Extrakt wird zur Trockene eingedampft, der Rückstand in Aceton gelöst, auf Dünnschicht-Platten aufgetragen und mit dem von Sun et al. beschriebenen Lösungsmittelsystem chromatographiert. Die radioaktiven Zonen werden mit einem Detektor lokalisiert. Die dem 6-Keto-$PGF_1\alpha$ (einem stabilen Endprodukt der Prostacyclin-Biotransformation) und $PGE_2$ entsprechenden Zonen werden in Szintillationsröhrchen für Flüssigkeiten übertragen und gezählt. Der Quotient aus den Zahlenwerten von 6-Keto-$PGF_1\alpha$ und $PGE_2$ wird für jede Konzentration der verwendeten Testverbindung berechnet und daraus die $IC_{50}$-Werte der Hemmung graphisch ermittelt. Dieser Wert entspricht der Konzentration an Testverbindung, bei welcher der Quotient 6-Keto-$PGF_1\alpha/PGE_2$ auf 50% des Kontrollwertes reduziert wird.

Die Hemmung der Thromboxan-Synthese und die Verminderung des Thromboxan-Plasmaspiegels wird in vivo durch Verabreichung der Testverbindung an Ratten [analog zu den von Tai et al. in Anual. Biochem. *87*, 343 (1978) und von Salmon in Prostaglandins *15*, 383 (1978) beschriebenen Verfahren] wie folgt bestimmt:

Ratten werden mit der Testsubstanz oder einem Trägermaterial behandelt; nach 2 h wir ihnen Ionophor A 23187 (0,5 mg/kg) intravenös injiziert. Zwei Minuten nach der Ionophor-Verabreichung wird den Tieren für die Analyse Blut entnommen. Eine bestimmte Einzelmenge jeder Plasmaprobe wird mittels Radioimmunoassay auf Thromboxan $B_2$, und eine weitere auf 6-Keto-$PGF_1\alpha$, die stabilen Metaboliten des Thromboxans $A_2$ bzw. des Prostacyclins ($PGI_2$), untersucht.

Die Verbindungen der Formel I sind sehr wirksame und selektive Thromboxan-Synthetase-Inhibitoren. Bei wirksamen Dosis-Spiegeln und darüber wird weder das vorteilhafte Prostacyclin-Synthetase- noch das Prostaglandin-cyclooxygenase-Enzymsystem wesentlich gehemmt. Ueberraschendereweise wird der Prostacyclin-Spiegel signifikant erhöht.

Bei einer oralen Dosis von 0,10 mg/kg oder weniger senken Verbindungen der Erfindung bei der Ratte die Plasmakonzentration von Thromboxan $B_2$ um über 50%; gleichzeitig wird überraschend ein Anstieg des Plasmaspiegels von Prostacyclin beobachtet.

Aufgrund der genannten vorteilhaften Eigenschaften sind die Verbindungen der Erfindung für Säuger, einschliesslich Menschen, als spezifische therapeutische Mittel sehr wertvoll.

Die Nützlichkeit der Verbindungen der Erfindung bei Thromboembolien ist daraus zu ersehen, dass Verbindungen der Erfindung eine auf unterschiedliche Weise herbeigeführte Aggregation der Blutplättchen und auch Thrombozytopenie aufheben. Experimentell wird eine nützliche antithrombotische

Wirkung z.B. durch die Verlängerung der Blutungszeit bei der Ratte angezeigt. Verbindungen der vorliegenden Erfindung verlängern diese Blutungszeit bei einer oralen Dosis von ungefähr 10 mg/kg oder weniger.

Ausser den oben genannten pharmazeutisch verwendbaren Salzen bilden auch alle pharmakologischen Vorstufen der erfindungsgemässen Carbonsäuren, sogenannte "prodrugs", z.B. ihre pharmazeutisch verwendbaren Ester und Amide, die durch Solvolyse oder unter physiologischen Bedingungen in genannten Carbonsäuren übergehen können, einen weiteren Gegenstand dieser Erfindung.

Solche Ester sind vorzugsweise z.B. geradkettige oder verzweigte Niederalkylester, die unsubstituiert oder geeignet substituiert sein können, wie etwa Pivaloyloxymethyl-, 2-Diäthylaminoäthyl-, Bornyloxycarbonylmethyl-, α-Carboxyäthyl- oder in geeigneter Weise veresterte α-Carboxyäthylester und dergleichen, welche in an sich bekannter Weise hergestellt werden können.

Die genannten Amide sind vorzugsweise z.B. einfache primäre oder sekundäre Amide und solche, welche von Aminosäuren oder deren Derivaten, Z.B. von Alanin oder Phenylalanin, abgeleitet sind.

Bevorzugt sind Verbindungen der Formel I, worin $R_1$ Wasserstoff oder Niederalkyl bedeutet, Ar jeweils unsubstituiertes oder durch Niederalkyl substituiertes 3-Pyridyl oder 1-Imidazolyl bedeutet, $R_2$ und $R_3$ unabhängig voneinander Wasserstoff, Niederalkyl, Halogen, Trifluormethyl, Hydroxy, Niederalkoxy oder Niederalkylthio bedeuten, oder $R_2$ und $R_3$ zusammen, an benachbarten Kohlenstoffatomen, Niederalkylendioxy sind;

A für Alkylen mit 4 bis 12 Kohlenstoffatomen, Nieder-(alkylenphenylen, akylen-thio-phenylen oder alkylen-oxy-phenylen) mit 7 bis 10 Kohlenstoffatomen steht;

B Carboxy, Niederalkoxycarbonyl, Carbamoyl, Cyan, Hydroxycarbamoyl, 5-Tetrazolyl oder Hydroxymethyl bedeutet; und ihre Salze, insbesondere ihre pharmazeutisch verwendbaren Salze.

Ferner sind die Verbindungen der Formel I bevorzugt, worin der Rest $R_2$ in 5-Stellung des Indolkerns angeknüpft ist und $R_3$ Wasserstoff bedeutet.

Besonders bevorzugt sind die Verbindungen der Formel I, worin B Carboxy, Niederalkoxycarbonyl, Carbamoyl, 5-Tetrazolyl oder Hydroxycarbamoyl bedeutet.

Ganz besonders sind solche Verbindung der Formel I bevorzugt, worin A Alkylen mit 4 bis 10 Kohlenstoffatomen, Niederalkylenphenylen mit 7 bis 10 Kohlenstoffatomen, Niederalkylen-thio-phenylen mit 7 bis 10 Kohlenstoffatomen oder Niederalkylen-oxy-phenylen mit 7 bis 10 Kohlenstoffatomen bedeutet, B für Carboxy oder Niederalkoxycarbonyl steht, Ar 3-Pyridyl oder 1-Imidazolyl bedeutet und $R_2$ und $R_3$ unabhängig voneinander Wasserstoff, Niederalkyl, Halogen, Trifluormethyl, Hydroxy, Niederalkylthio oder Niederalkoxy bedeuten; und ihre Salze, insbesondere ihre pharmazeutisch verwendbaren Salze.

In erster Linie sind Verbindungen der Formel I bevorzugt, worin A Alkylen mit 4 bis 8 Kohlenstoffatomen bedeutet.

Von besonderem Interesse sind Verbindungen der Formel II

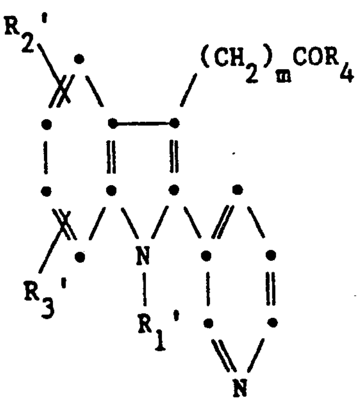

(II),

worin $R_1'$ Wasserstoff oder Niederalkyl bedeutet, $R_2'$ und $R_3'$ unabhängig voneinander für Wasserstoff, Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 4 bis 12 steht und $R_4$ Hydroxy, Niederalkoxy oder Amino bedeutet; und ihre Salze, insbesondere ihre pharmazeutisch verwendbaren Salze.

Ferner sind die Verbindungen der Formel II bevorzugt, worin $R_3'$ Wasserstoff bedeutet.

Ferner sind solche Verbindungen der Formel II bevorzugt, worin $R_1'$ Methyl, Aethyl oder Propyl bedeutet, $R_2'$ für Wasserstoff, Methyl, Chlor, Fluor, Trifluormethyl, Hydroxy, Methylthio oder Methoxy steht, $R_3'$ Wasserstoff bedeutet, m für eine ganze Zahl von 4 bis 8 steht und $R_4$ Hydroxy, Aethoxy, Methoxy oder Amino bedeutet; und ihre Salze, insbesondere ihre pharmazeutisch verwendbaren Salze.

Besonders bevorzugt sind Verbindungen der Formel II, worin $R_1'$ Wasserstoff oder Niederalkyl bedeutet, $R_2'$ für Wasserstoff oder Halogen steht, $R_3'$ Wasserstoff ist, m für eine ganz Zahl von 4 bis 8 steht

und $R_4$ Hydroxy, Niederalkoxy oder Amino bedeutet, und ihre Salze, insbesondere ihre pharmazeutisch verwendbaren Salze.

In erster Linie sind die Verbindungen der Formel II bevorzugt, worin $R_1'$ Wasserstoff oder Methyl bedeutet, $R_2'$ für Wasserstoff oder Chlor steht, $R_3'$ Wasserstoff ist, m für 5 steht und $R_4$ Hydroxy bedeutet, und ihre Salze, insbesondere ihre pharmazeutisch verwendbaren Salze.

Bevorzugt sind ferner Verbindungen der Formel III

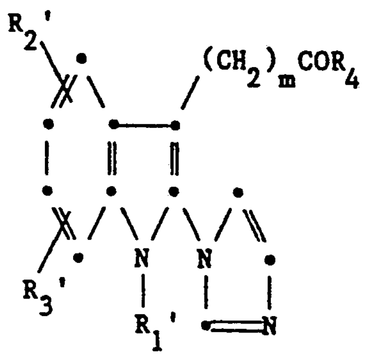

(III),

worin $R_1'$ Wasserstoff oder Niederalkyl bedeutet, $R_2'$ und $R_3'$ unabhängig voneinander für Wasserstoff, Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 3 bis 12 steht und $R_4$ Hydroxy, Niederalkoxy oder Amino bedeutet, und ihre Salze, insbesondere ihre pharmazeutisch verwendbaren Salze.

Bevorzugt sind Verbindungen der Formel III, worin $R_3'$ Wasserstoff bedeutet.

Besonders bevorzugt sind Verbindungen der Formel III, worin $R_1'$ Methyl, Aethyl oder Propyl bedeutet, $R_2'$ für Wasserstoff, Methyl, Chlor, Fluor, Trifluormethyl, Hydroxy, Methylthio oder Methoxy steht, $R_3'$ Wasserstoff ist, m für eine ganze Zahl von 3 bis 8 steht und $R_4$ Hydroxy, Aethoxy, Methoxy oder Amino bedeutet, und ihre Salze, insbesondere ihre pharmazeutisch verwendbaren Salze.

Ganz besonders bevorzugt sind Verbindungen der Formel III, worin $R_1'$ Wasserstoff oder Niederalkyl bedeutet, $R_2'$ für Wasserstoff oder Halogen steht, $R_3'$ Wasserstoff ist, m für eine ganze Zahl von 4 bis 8 steht und $R_4$ Hydroxy, Niederalkoxy oder Amino bedeutet, und ihre Salze, insbesondere ihre pharmazeutisch verwendbaren Salze.

In erster Linie sind solche Verbindungen der Formel III bevorzugt, worin $R_1'$ Wasserstoff oder Methyl bedeutet, $R_2'$ für Wasserstoff oder Chlor steht, $R_3'$ Wasserstoff ist, m für 5 steht und $R_4$ Hydroxy bedeutet, und ihre Salze, insbesondere ihre phrmazeutisch verwendbaren Salze.

Vor allen Dingen bevorzugt sind die in den Beispielen beschriebenen Verbindungen der Formel I und ihre pharmazeutisch verwendbaren Salze.

Die Verbindungen der vorliegenden Erfindung werden nach an sich bekannten Methoden, vorzugsweise dadurch hergestellt, dass man z.B.

1) eine Verbindung der Formel IV

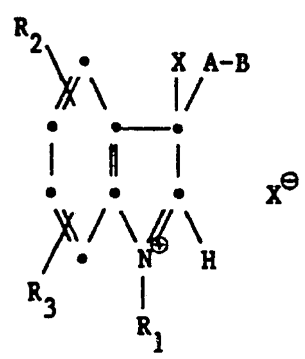

(IV),

worin $R_1$, $R_2$, $R_3$, A und B die unter Formel I angegebene Bedeutung haben und X für Halogen steht, mit einer Verbindung der Formel Ar-H oder einem reaktiven metallierten Derivat davon, worin Ar die unter Formel I angegebene Bedeutung hat, umsetzt, und, falls gewünscht, eine erhaltene Verbindung der Formel

I, worin $R_1$ Wasserstoff ist, mit einem Alkylierungsmittel in eine Verbindung der Formel I überführt, worin $R_1$ Niederalkyl bedeutet, oder

2) eine Verbindung der Formel V

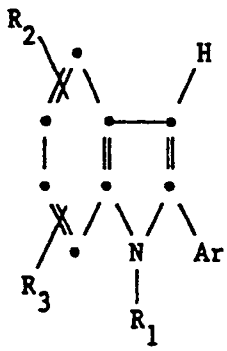

(V),

worin $R_1$, $R_2$, $R_3$ und Ar die unter Formel I angegebene Bedeutung haben, als reaktives metallorganisches Derivat mit einem reaktiven funktionellen Derivat einer Verbindung der Formel VI

$$HO—A—B \quad (VI),$$

worin A und B die unter Formel I angegebene Bedeutung haben, umsetzt, und, falls gewünscht, eine erhaltene Verbindung der Formel I, worin $R_1$ Wasserstoff ist, mit einem Alkylierungsmittel in eine Verbindung der Formel I umsetzt, worin $R_1$ Niederalkyl ist, oder

3) eine Verbindung der Formel VII

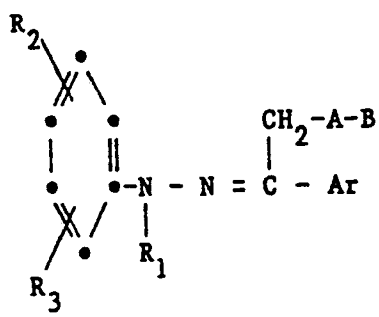

(VII),

worin $R_1$, $R_2$, $R_3$, A und B die unter Formel I angegebene Bedeutung haben und Ar für unsubstituiertes oder wie unter Formel I angegeben substituiertes 3-Pyridyl steht, ringschliesst, oder

4) eine Verbindung der Formel VIII

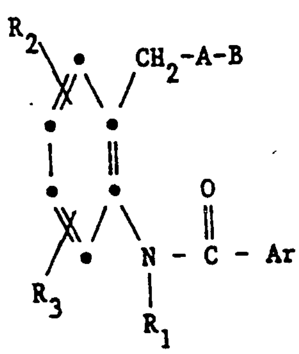

(VIII),

worin Ar, $R_1$, $R_2$, $R_3$, A und B die unter Formel I angegebene Bedeutung haben, cyclisiert, oder

5) zur Herstellung einer Verbindung der Formel I, worin A Alkenylen bedeutet, unter den Bedingungen einer Wittig-Reaktion eine Verbindung der Formel Va

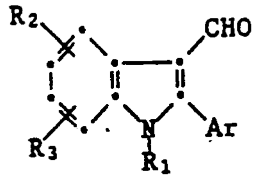

(Va),

die einem 3-Formylderivat einer Verbindung der Formel V entspricht, mit dem Ylid einer Verbindung der Formel XII

$$R_5—A'—B \quad (XII)$$

worin B die unter Formel I angegebene Bedeutung hat, A' Alkylen wie oben für A in Verbindungen der Formel I definiert, jedoch mit um 1 Kohlenstoffatom verkürzter Kettenlänge, ist und $R_5$ einen Dialkylphosphono- oder Triarylphosphonium-Rest bedeutet, umsetzt oder

6) zur Herstellung einer Verbindung der Formel I, worin A Niederalkyl-(thio oder oxy)-niederalkylen oder Niederalkylen-(thio oder oxy)-phenylen bedeutet, eine Verbindung der Formel Vb

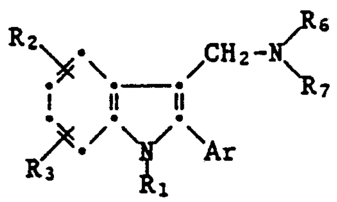

(Vb),

worin Ar, $R_1$, $R_2$ und $R_3$ die unter Formel I angegebene Bedeutung haben und $R_6$ und $R_7$ jeweils Niederalkyl bedeuten — also eine 3-(disubstituiertes Aminomethyl)-Derivat einer Verbindung der Formel V — mit einer Verbindung der Formel XIII

$$R_5'—A''—B \quad (XIII)$$

oder einem reaktiven Alkalimetall- oder Ammonium-Derivat davon, worin B wie unter Formel I angegeben definiert ist, $R_5'$ Hydroxy oder Thol bedeutet und A'' für Niederalkylen oder Phenylen steht; oder mit einem Lacton oder Thiolacton einer Verbindung der Formel XIII, worin $R_5'$ Hydroxy oder Thiol bedeutet, A'' Niederalkylen ist und B für Carboxy steht, umsetzt, oder

7) eine Verbindung der Formel Ia

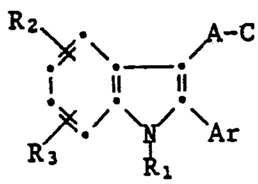

(Ia),

worin A, Ar, $R_1$, $R_2$ und $R_3$ die unter Formel I angegebene Bedeutung haben und C eine von B verschiedene und in B überführbare Gruppe, wie Trialkoxymethyl, verestertes Hydroxymethyl, veräthertes Hydroxymethyl, Halogenmethyl, 2-Oxazolinyl, Dihydro-2-oxazolinyl, Niederalkanoyloxymethyl, Acetyl, Methyl, Carboxycarbonyl, Trihalogenacetyl, Di-niederalkoxymethyl, Alkylendioxymethyl, Vinyl, Alkinyl, verestertes Carboxy oder amidiertes Carboxy, bedeutet, gegebenenfalls unter Verlängerung der Kette A im Rahmen ihrer Definition, oder eine Verbindung der Formel I* oder Ia*, die derjenigen der Formel I beziehungsweise Ia gleicht, worin jedoch abweichend A für Alkylen mit 1 oder 2 Kohlenstoffatomen steht, unter Verlängerung der Alkylenkette A zu Alkylen mit einer solchen Zahl von Kohlenstoffatomen wie sie unter Formel I definiert ist, in eine Verbindung der Formel I umwandelt; wobei störende reaktionsfähige Gruppen im Molekül gegebenenfalls vorübergehend geschützt sind, und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

Die zur Durchführung des Verfahrens 1) notwendigen Verbindungen der Formel IV werden z.B. dadurch hergestellt, vorzugsweise *in situ*, dass man eine Verbindung der Formel IVa

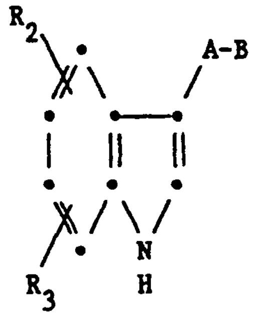

(IVa),

worin $R_2$, $R_3$, A und B die oben angegebene Bedeutung haben, mit einem Halogen, vorzugsweise Brom, in einem inerten Lösungsmittel wie z.B. Dioxan, vorteilhaft bei Raumptemperatur umsetzt.

Die anschliessende Reaktion mit einer Verbindung der Formel Ar-H oder einem reaktiven metallorganischen Derivat davon, z.B. einem Alkalimetall-Derivat, wird in einem Temperaturbereich von 0° bis 100°C, vorteilhaft bei Raumtemperatur, durchgeführt.

Das Verfahren 1) ist besonders vorteilhaft für die Herstellung von Verbindungen der Formel I, worin Ar unsubstituiertes oder durch Niederalkyl substituiertes 1-Imidazolyl bedeutet, wobei eine Verbindung der Formel Ar-H unsubstituiertem oder an einem der Kohlenstoffatome mit Niederalkyl substituiertem Imidazol entspricht.

Die als Ausgangsmaterialien verwendeten Indole der Formel IVa sind entweder bekannt oder, wenn neu, werden sie nach an sich bekannten Methoden hergestellt.

Zur Durchführung des Verfahrens 2) werden die Verbindungen der Formel V zunächst in reaktive metallorganische Derivate überführt, z.B. in Alkalimetall- oder Halogenmagnesium-(Grignard)-Derivate mittels eines zur Metallierung geeigneten Agens, z.B. einem Grignard-Reagens, einer Alkalimetallbase oder einer quaternären Ammoniumbase. Vorzugsweise werden die Verbindungen der Formel V *in situ* in die reaktiven metallorganischen Zwischenprodukte unter Verwendung eines reaktiven metallierenden Agens umgewandelt, vorzugsweise mit einem molaren Aequivalent von z.B. einer starken Alkalimetallbase, wie z.B. Lithium-diisopropylamid, Natriumhydrid, Kalium-tert.-butoxid, einem Grignard-Reagens, z.B. einem Niederalkylmagnesiumhalogenid wie Methyl- oder Aethylmagnesiumbromid, in einem inerten Lösungsmittel, wie z.B. Dimethylformamid, Diäthyläther oder Tetrahydrofuran, in einem Temperaturbereich von −50° bis +75°C, vorzugsweise von −25° und +50°C. Die Umsetzung der erhaltenen reaktiven metallorganischen Verbindungen der Formel V mit einem reaktiven veresterten Derivat einer Verbindung der Formel VI wird in einem Temperaturbereich von ungefähr −25° bis +50°C vorzugsweise in einem Bereich von 0° bis 30°C durchgeführt. In den Fällen, wo B für Carboxy, Carbamoyl, Hydroxycarbamoyl oder Mono-niederalkylcarbamoyl steht, sind eines oder mehrere weitere molare Aequivalente des Metallierrungsmittels erforderlich.

Die Zwischenprodukte der Formel V sind bekannt (z.B. US-Patent 3.468.894; J. Chem. Soc. *1955*, 2865; Bull. Soc. Chim. France *1969*, 4154) oder werden analog zu den bekannten hergestellt, z.B. durch Reaktion der entsprechenden gegebenenfalls substituierten Phenylhydrazine mit Ketonen der Formel $ArCOCH_3$ in Gegenwart eines Kondensationsmittels, z.B. äthanolischer HCl-Lösung oder Polyphosphorsäure, gemäss der bekannten Fischer-Indolsynthese.

Die Ausgangsverbindungen der Formel VI sind bekannt oder, wenn neu, werden sie nach herkömmlichen Methoden hergestellt, z.B. gemäss den im US-Patent 4.256.757 und in der Britischen Patentanmeldung 2.016.452 A angegebenen Verfahrensweisen.

Der Ringschluss des Ausgangsstoffes der Formel VII gemäss dem Verfahren 3) wird gemäss der wohlbekannten Fischer-Indolsynthese durchgeführt, die z.B. in "Heterocyclic Compounds, Indoles Part I" (Herausgeber: W. J. Houlihan) S. 232—317, beschrieben ist, und zwar thermisch oder vorzugsweise in Gegenwart eines sauren Kondensationsmittels. Im letzeren Fall arbeitet man vorzugsweise in Gegenwart von Halogenwasserstoffen, z.B. äthanolischem Chlorwasserstoff, oder Polyphosphorsäure, gegebenenfalls in einem inerten Lösungsmittel, vorzugsweise bei Temperaturen zwischen ungefähr 50° und 100°C.

Die Hydrazon-Zwischenprodukte der Formel VII werden — in isolierter Form oder vorzugsweise in situ — durch Kondensation eines Ketons der Formel $ArCOCH_2—A—B$, worin Ar, A und B die oben für Verbindungen der Formel I angegebene Bedeutung haben, mit einem Hydrazin der Formel IX

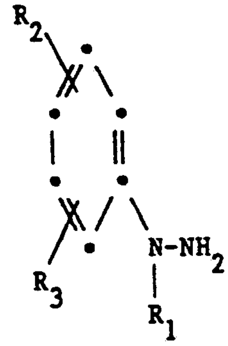

(IX),

worin $R_1$, $R_2$ und $R_3$ die oben für Verbindungen der Formel I angegebenen Bedeutung haben, vorteilhaft in Gegenwart eines sauren Katalysators, hergestellt.

Die Hydrazin-Ausgangsstoffe der Formel IX sind bekannt oder werden vorzugsweise z.B. durch Nitrosierung von entsprechend substituierten Anilinen der Formel X

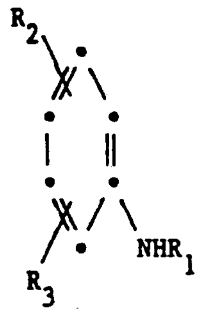

(X),

worin $R_1$, $R_2$ und $R_3$ die oben angegebene Bedeutung haben, und nachfolgende Reduktion der N-Nitroso-Derivate, z.B. mit Zink in Essigsäure oder nach anderen an sich bekannten Methoden, hergestellt.

Die Cyclisierung gemäss dem Verfahren 4) wird unter den Bedingungen der Indolsynthese nach Madelung, wie in "Heterocyclic Compounds, Indoles Part I" (Herausgeber W. J. Houlihan) S. 385—396 beschrieben, vorgenommen. Die intramolekulare Cyclisierung wird vorzugsweise in Gegenwart einer starken Base, z.B. Natriumäthoxid, Natriumamid oder Kalium-tert.-butoxid, vorteilhaft bei erhöhter Temperatur, z.B. bei ungefähr 300°C, oder in einem inerten hochsiedenden Lösungsmittel, z.B. Tetrahydronaphthalin, durchgeführt.

Die Ausgangsstoffe der Formel VIII, worin Ar vorzugsweise unsubstituiertes oder wie oben angegeben substituiertes 3-Pyridyl bedeutet, werden durch Acylierung von substituierten Anilinen der Formel XI

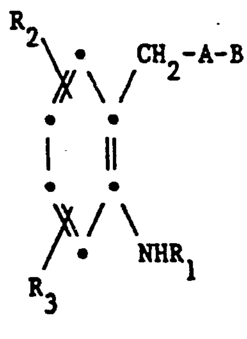

(XI),

worin A, B, $R_1$, $R_2$ und $R_3$ die oben angegebene Bedeutung haben, mit einer Verbindung der Formel ArCOOH, worin Ar vorzugsweise unsubstituiertes oder wie oben definiert substituiertes 3-Pyridyl bedeutet, oder einem reaktiven funktionellen Derivat davon, erhalten.

Die zur Durchführung des Verfahrens 5) benötigten Ausgangsverbindungen der Formel Va können z.B. dadurch hergestellt werden, dass man eine Verbindung der Formel V unter den Bedingungen der Vilsmeier-Haack-Reaktion mit einem N,N-disubstituierten Formamid, z.B. mit Dimethylformamid, in Gegenwart von Phosphoroxychlorid umsetzt.

Dialkylphosphono bedeutet z.B. Diäthylphosphono, und ein Triarylphosphonium-Rest ist z.B. Triphenylphosphonium.

Die zur Durchführung des Verfahrens 6) benötigten Ausgangsverbindungen der Formel Vb können z.B.

dadurch erhalten werden, dass man eine Verbindung der Formel V unter den Bedingungen der Mannich-Reaktion z.B. mit Formaldehyd und einem sekundären Amin, z.B. Dimethylamin, umsetzt.

Die Umwandlung einer Verbindung der Formel Ia, worin sich C von B unterscheidet, gemäss dem Verfahren 7) in eine Verbindung der Formel I, und die fakultative Umwandlung eines erhaltenen Produktes der Formel I in eine andere Verbindung dieser Erfindung, werden gemäss an sich bekannten chemischen Methoden und/oder wie hier beschrieben vergenommen.

Umwandelbare Gruppen C sind vorzugsweise Trialkoxymethyl, verestertes Hydroxymethyl, veräthertes Hydroxymethyl, Halogenmethyl, 2-Oxazolinyl, Dihydro-2-oxazolinyl, Niederalkanoyloxymethyl, Acetyl, Methyl, Carboxycarbonyl, Trihalogenacetyl, Di-niederalkoxymethyl, Alkylendioxymethyl, Vinyl, Alkinyl, verestertes Carboxy und amidiertes Carboxy.

Die Zwischenprodukte der Formel Ia werden z.B. in strenger Analogie zu den Verfahren 1) bis 6) oder wie hier beschrieben, jeweils unter Verwendung von an sich bekannten chemischen Methoden, hergestellt.

Die einzelnen Definitionen in den vorher beschriebenen Verfahren haben folgende Bedeutung:

Reaktive funktionelle Derivate eines Alkohols der Formel VI sind z.B. solche, in denen die Hydroxygruppe mit einer starken anorganischen oder organischen Säure, vor allem mit einer Halogenwasserstoffsäure, z.B. Chlorwasserstoff-, Bromwasserstoff- oder Jodwasserstoffsäure, einer aliphatischen oder aromatischen Sulfonsäure, z.B. Methansulfon- oder p-Toluolsulfonsäure, verestert ist. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Veräthertes Hydroxymethyl bedeutet vorzugsweise tertiäres Niederalkoxymethyl, niederes Alkoxyalkoxymethyl, z.B. Methoxymethoxymethyl, 2-Oxa- oder 2-Thiacycloalkoxymethyl, insbesondere 2-Tetrahydropyranyloxymethyl.

Verestertes Hydroxymethyl bedeutet vorzugsweise Niederalkanoyloxymethyl, vorteilhaft Acetoxymethyl, Niederalkylsulfonyloxymethyl, z.B. Methylsulfonyloxymethyl, oder Arylsulfonyloxymethyl, z.B. p-Tolylsulfonyloxymethyl.

Halogenmethyl steht insbesondere für Chlormethyl, kann aber auch Brommethyl oder Jodmethyl sein.

Ein Alkalimetall bedeutet vorzugsweise Lithium, kann aber auch Kalium oder Natrium sein.

Die Umwandlung von Verbindungen der Formel Ia in Verbindungen der Formel I sowie die Umwandlung von Verbindungen der Formel I in eine andere Verbindung der Erfindung werden nach an sich bekannten chemischen Methoden durchgeführt.

Zwischenprodukte der Formel Ia, in welchen C Halogenmethyl bedeutet, können vorzugsweise mit einem Metallcyanid, z.B. Kaliumcyanid, in an sich bekannter Weise umgesetzt werden. Man erhalt dabei Verbindungen der Formel I, in welchen die Kette um ein Kohlenstoffatomen verlängert ist und B für Cyan steht. Dies können ihrerseits nach an sich bekannten Methoden in Verbindungen der Formel I, worin B Carboxy, Niederalkoxycarbonyl oder Carbamoyl bedeutet, überführt werden.

So können Verbindungen der Formel I, in welchen B Cyan bedeutet (Nitrile), in Verbindungen der Formel I, worin B für Carboxy steht, durch Hydrolyse mit anorganischen Säuren, z.B. mit Halogenwasserstoffsäuren, wie Chlorwasserstoffsäure, oder Schwefelsäure in wässeriger Lösung, oder vorzugsweise durch Hydrolyse mit wässerigen Alkalimetallhydroxiden, z.B. Kaliumhydroxid, bei Rückflusstemperatur umgewandelt werden.

Die Ueberführung der genannten Nitrile in Verbindungen der Formel I, worin B Niederalkoxycarbonyl bedeutet, wird vorzugsweise zunächst durch Behandlung mit einem Niederalkanol, z.B. wasserfreiem Aethanol, in Gegenwart einer starken Säure, z.B. Chlorwasserstoffsäure, vorzugsweise unter Rückfluss, und nachfolgende vorsichtige Hydrolyse mit Wasser vorgenommen.

Die Ueberführung der genannten Nitrile in Verbindungen der Formel I, worin B für Carbamoyl steht, wird vorzugsweise durch Behandlung mit einem Alkalimetallhydroxid, z.B. verdünnter Natronlauge, und Wasserstoffperoxid, vorzugsweise bei Raumtemperatur, durchgeführt.

Zwischenprodukte der Formel Ia, in welchen C Halogenmethyl, z.B. Chlormethyl, bedeutet, können in Verbindungen der Formel I, worin B für Carboxy steht und die Kette um 2 Kohlenstoffatome verlängert ist, umgewandelt, werden, indem sie zunächst z.B. mit einem Diniederalkyl-malonat, z.B. Diäthylmalonat, in Gegenwart einer Base, z.B. Kaliumcarbonat oder Natriumäthoxid, in einem Lösungsmittel wie Dimethylformamid, vorzugsweise bei einer Temperatur zwischen 50° und 100°C behandelt werden. Die entstandenen substituierten Di-niederalkylmalonate werden dann vorzugsweise mit einer wässerigen Base, z.B. verdünnter Natronlauge, zur entsprechenden Malonsäure hydrolysiert, welche unter Standardbedingungen, z.B. durch Erhitzen in Xylol, zu einer Verbindung der Formel I, worin B Carboxy bedeutet, decarboxyliert wird. Ersetzt man den Malonsäure-di-n-niederalkylester durch einen Cyanessigsäure-niederalkylester, so erhält man die entsprechenden Verbindungen der Formel I, in welchen B Cyan bedeutet.

Verbindungen der Erfindung, in denen A ein geradkettiger oder verzweigter Alkenylenrest mit einer terminalen Doppelbindung ist, können auch aus den Zwischenprodukten der Formel Ia, worin C Halogenmethyl bedeutet, hergestellt werden. So können z.B. diese Zwischenprodukte zuerst mit einem Niederalkylester einer α-(Aryl- oder Alkyl)-thioessigsäure, z.B. α-(Phenylthio)-essigsäureäthylester, in Gegenwart einer starken Base, z.B. Natriumhydrid, behandelt werden. Die nachfolgende Oxidation des erhaltenen α-Aryl-thio- oder α-Alkylthio-substituierten Esters zum entsprechenden α-Arylsulfinyl- oder α-

Alkylsulfinyl-ester z.B. mit Natriumperjodat, gerfolgt von einer durch Hitze ausgelösten Eliminierung, z.B. durch Erhitzen in Xylol, ergibt eine Verbindung der allgemeinen Formel I (einen α,β-ungesättigten Ester), in der A Alkenylen bedeutet und B z.B. Niederalkoxycarbonyl ist. Die Kette wird gleichzeitig um zwei Kohlenstoffatome verlängert. Die gleiche Umwandlung kann auch unter Verwendung z.B. von α-(Phenylseleno)-essigsäure-äthylester, wie in J. Am. Chem. Soc. *95*, 6137 (1973) beschrieben, durchgeführt werden. Auch können die Verbindungen der Formel Ia, in denen C Halogenmethyl bedeutet, zuerst in die entsprechenden Carboxyaldehyde umgewandelt werden, z.B. mit Dimethylsulfoxid in Gegenwart von Triäthylamin und Silbertetrafluoroborat, oder mit Chromtrioxid und Pyridin in Methylenchlorid. Die nachfolgende Wittig-Kondensation z.B. mit Trimethylphosphonoacetat oder (Triphenylphosphoranyliden)-essigsäure-äthylester ergibt ebenfalls die obengenannten α,β-ungesättigten Ester.

Verbindungen der Formel I, worin B Niederalkoxycarbonyl ist, können mit Ammoniak, Mono- oder Di-niederalkylaminen, z.B. Methylamin oder Dimethylamin, in einem inerten Lösungsmittel, z.B. einem Niederalkanol wie Butanol, gegebenenfalls bei erhöhten Temperaturen, zu Vebindungen der Formel I, in welchen B unsubstituiertes, mono- oder di-niederalkyl-substituiertes Carbamoyl bedeutet, amidiert werden.

Verbindungen der Formel I, worin A einen geradkettigen oder verzweigten Alkenylenrest mit einer terminalen Doppelbindung bedeutet, z.B. α,β-ungesättigte Ester, können auch aus den entsprechenden α,β-gesättigten Verbindungen durch Behandlung z.B. mit Phenylselenylchlorid in Gegenwart einer starken Base gemäss der in J. Am. Chem. Soc. *95*, 6137 (1973) beschriebenen Methode erhalten werden.

Die Umwandlung von Verbindungen der Formel I, worin B Niederalkoxycarbonyl, Cyan, unsubstituiertes, mono- oder ni-niederalkyl-substituiertes Carbamoyl bedeutet, in Verbindungen der Formel I, in denen B Carboxy ist, wird vorteilhaft durch Hydrolyse mit anorganischen Säuren, z.B. mit Halogenwasserstoffsäuren oder Schwefelsäure, oder mit wässerigen Alkalien, vorzugsweise mit Alkalimetallhydroxiden, z.B. Lithium- oder Natriumhydroxid, durchgeführt.

Verbindungen der Formel I, in welchen B Carboxy oder Niederalkoxycarbonyl bedeutet, können mit einfachen oder komplexen Leichtmetallhydriden, z.B. mit Lithiumaluminiumhydrid, Alan oder Diboran, zu Verbindungen der Formel I, in denen B für Hydroxymethyl steht, reduziert werden. Diese Alkohole können auch durch geeignete Solvolyse von Zwischenprodukten der Formel Ia, worin C Halogenmethyl bedeutet, durch Behandlung z.B. mit einem Alkalimetallhydroxid, z.B. Lithium- oder Natriumhydroxid, erhalten werden.

Die vorher genannten Alkohole können ihrerseits mit konventionellen Oxidationsmitteln, vorzugsweise mit Pyridin-dichromat in Dimethylformamid bei Raumtemperatur, in Verbindungen der Formel I, worin B Carboxy bedeutet, umgewandelt werden.

Freie Carbonsäuren können mit Niederalkanolen, z.B. Aethanol in Gegenwart einer starken Säure, z.B. Schwefelsäure, vorzugsweise bei erhöhter Temperatur, oder mit Diazo-niederalkanen, z.B. Diazomethan in einem Lösungsmittel, z.B. Diäthyläther, vorzugsweise bei Raumtemperatur, zu den entsprechenden Estern, nämlich zu solchen Verbindungen der Formel I, worin B Niederalkoxycarbonyl bedeutet, umgesetzt werden.

Weiter können die freien Carbonsäuren durch Behandlung eines ihrer reaktionsfähigen Zwischenprodukte, z.B. eines Acylhalogenids, etwa eines Säurechlorids oder gemischten Anhydrids, z.B. eines solchen, das von einem Halogenkohlensäure-niederalkylester, z.B. Chlorameisensäure-äthylester abgeleitet ist, mit Ammoniak, Mono- oder Di-niederalkylaminen, in einem inerten Lösungsmittel, z.B. Methylenchlorid, vorzugsweise in Gegenwart eines basischen Katalysators, z.B. Pyridin, in Verbindungen der Formel I, in denen B für unsubstituiertes, mono- oder di-niederalkyl-substituiertes Carbamoyl steht, umgewandelt werden.

Verbindungen der Formel I, worin B Mono-niederalkyl-carbamoyl bedeutet, können in Verbindungen der Formel I, in denen B für Di-niederalkyl-carbamoylsteht, durch Behandlung mit einer starken Base, z.B. Natriumhydrid, gefolgt von einem Alkylierungsmittel, z.B. einem Niederalkylhalogenid, in einem inerten Lösungsmittel, z.B. Dimethylformamid, überführt werden.

Weiter können Verbindungen der Formel I, in denen A einen geradkettigen oder verzweigten Alkinylen- oder Alkenylenrest bedeutet, durch katalytische Hydrierung, vorzugsweise unter neutralen Bedingungen, z.B. einem Palladium-Katalysator bei atmosphärischem Druck, in einem inerten Lösungsmittel, z.B. Aethanol, in Verbindungen der Formel I, worin A für geradkettiges oder verzweigtes Alkylen steht, umgewandelt werden.

Carboxaldehyde, nämlich Verbindung der Formel I, in denen B Formyl bedeutet, können durch Oxidation von Verbindungen der Formeln I bzw. Ia, worin B bzw. C Hydroxymethyl bzw. Halogenmethyl bedeuten, z.B. mit Dimethylsulfoxid und einem Katalysator, z.B. einem Gemisch aus Triäthylamin und Silbertetrafluoroborat, oder mit Chromtrioxid und Pyridin oder mit an sich bekannten anderen geeigneten Oxidationsmitteln hergestellt werden. Die genannten Carboxaldehyde können in die entsprechenden Acetale, d.h. Verbindungen der Formel Ia, worin C Di-(niederalkoxy)-methyl oder Alkylendioxymethyl bedeutet, z.B. das Dimethylacetal, durch säurekatalysierte Kondensation mit einem Alkohol, z.B. Methanol, umgewandelt werden.

Verbindungen der Formel I, worin B Carboxy bedeutet, können durch die wohlbekannte Arndt-Eistert-Synthese zu Verbindungen der Formel I, worin B Carboxy ist und die Kette A ein Kohlenstoffatomen mehr enthält, umgewandelt werden. Insbesondere kann man ein reaktionsfähiges funktionelles Derivat der als Ausgangsstoff verwendeten Carbonsäure, z.B. ein Säurechlorid, mit Diazomethan z.B. in Diäthyläther

behandeln, wobei man eine Verbindung der Formel Ia, worin C Diazoazetyl bedeutet, erhält. Nach Behandlung z.B. mit Silberoxid erhält man die genannte Carbonsäure der Formel I, worin die Kette A um ein Kohlenstoffatom verlängert ist.

Eine besondere Ausführungsform des Verfahrens 7) betrifft die Herstellung von Verbindungen der Formel I, worin B Carboxy bedeutet. Sie besteht darin, dass man in einer Verbindung der Formel Ia, worin C einen in die Carboxygruppe überführbaren Rest bedeutet, die Gruppe C, gegebenenfalls unter Verlängerung der Kette A im Rahmen ihrer Definition, in Carboxy überführt.

In eine Carboxygruppe überführbare Reste sind z.B. verestertes Carboxy, anhydriertes Carboxy einschliesslich entsprechender asymmetrischer und innerer Anhydride, amidiertes Carboxy, Cyan, Amidin-Strukturen, einschliesslich cyclischer Amidine, wie z.B. 5-Tetrazolyl, Iminoäther, einschliesslich cyclischer Iminoäther, wie z.B. unsubstituierter oder durch Niederalkyl substituierter 2-Oxazolinyl- oder Dihydro-2-oxazolinyl-Reste, ferner Methyl, Hydroxymethyl, veräthertes Hydroxymethyl, Niederalkanoyloxymethyl, Trialkoxymethyl, Acetyl, Trihalogenacetyl, Halogenmethyl, Carboxycarbonyl (—COCOOH), Formyl, Di-niederalkoxymethyl, Alkylendioxymethyl, Vinyl, Aethinyl, oder Diazoacetyl. Gleichzeitig mit der Umwandlung von C in die Carboxygruppe kann die Kette A im Rahmen ihrer Definition verlängert werden.

Verestertes Carboxy ist vorzugsweise Carboxy in Form seiner Niederalkylester, z.B. des Methyl-, Aethyl-, n- oder Iso-(Propyl oder Butyl)-esters. Ferner in Form von substituierten Niederalkylestern, z.B. dem ω-Amino-, ω-Monomethylamino- oder ω-Dimethylamino-, α-Carboxy- oder α-Aethoxycarbonyl-(äthyl, propyl oder butyl)-ester. Weitere Ester sind Aryl-niederalkylester, z.B. Benzyl-, (Methyl, Methoxy, Chlor)-substituierte Benzyl- und Pyridylmethyl-Ester; Niederalkanoyloxy-niederalkylester, z.B. Pivaloyloxymethylester; unsubstituierte und durch Methyl, Methoxy oder Chlor substituierte 3-Phthalidylester, die von den entsprechenden 3-Hydroxy-phthaliden abgeleitet sind, (Hydroxy, Niederalkanoyloxy, Niederalkoxy)-substituierte Niederalkoxy-methylester, z.B. β-(Hydroxy, Acetyloxy, Methoxy)-äthoxymethylester; Bicycloalkyloxycarbonyl-niederalkylester, z.B. solche, die von bicyclischen Monoterpenoid-Alkoholen, z.B. unsubstituierten oder durch Niederalkyl substituierten Bicyclo[2,2,1]heptyloxycarbonyl-niederalkylestern, vorteilhaft Bornyloxycarbonylmethylestern; oder Halogen-substituierten Niederalkylestern, z.B. Trichloräthyl- oder Jodäthylestern, abgeleitet sind.

Amidiertes Carboxy ist vorzugsweise Carboxy in Form seiner unsubstituierten Amide, N-Mono- oder N,N-Di-niederalkylamide, z.B. Mono- oder Di-methylamide; in Form von tertiären Amiden, die z.B. von Pyrrolidin, Piperidin oder Morpholin abgeleitet sein können; ferner α-Niederalkoxycarbonyl- oder α-Carboxy-substituierte Niederalkylamide, z.B. Mono-N-äthoxycarbonylmethyl)-amide und Mono-N-(carboxymethyl)-amide; α-Niederalkoxycarbonyl- oder α-Carboxy-substituierte Aryl-niederalkylamide, z.B. Aethoxycarbonyl- oder Carboxy-substituierte Phenyläthylamide; Amino-niederalkylamide, z.B. β-Aminoäthylamide und β-(Benzyloxycarbonyl-amino)-äthylamide.

Die Ueberführung in die Carboxygruppe wird nach an sich bekannten Methoden, z.B. wie hier und in den Beispielen beschrieben, vorgenommen, z.B. solvolytisch, etwa hydrolytisch oder acidolytisch wie oben bescrieben, oder bei veresterten Carboxygruppen auch reduktiv. So werden z.B. 2,2,2-Trichloräthyl- oder 2-Jodäthylester durch Reduktion z.B. mit Zink und einer Carbonsäure in Gegenwart von Wasser in die Carbonsäure überführt. Benzylester oder Nitro-benzylester können durch katalytische Hydrierung oder auch durch Hydrierung mit chemischen Reduktionsmitteln, wie z.B. Natriumdithionit oder Zink und einer Carbonsäure, z.B. Essigsäure, in die Carboxygruppe umegwandelt werden. Ferner können tert-Butylester z.B. auch mit Trifluoressigsäure gespalten werden.

Während der Reduktion des Restes C kann gleichzeitig eine Alkenylen- oder Alkinylenkette A in die entsprechende Alkylenkette umgewandelt werden.

Weiter können Verbindungen der Formel Ia, worin C Acetyl bedeutet, oxidativ zu den entsprechenden Verbindungen der Formel I, worin B Carboxy bedeutet, gespalten werden. Dazu wird die Acetylverbindung zunächst in eine Verbindung der Formel Ia, worin C Trihalogenacetyl, z.B. Tribrom- oder Trijodacetyl bedeutet, überführt, z.B. durch Behandlung mit Natrium-hypobromit, und nachfolgend z.B. mit einer wässerigen Base, etwa natronlauge, gespalten.

Ausgangsstoffe der Formel Ia, in welchen C Acetyl bedeutet, können ausgehend von Verbindungen der Formel Ia, worin C Halogenmethyl bedeutet, durch Behandlung mit einem Acetessigsäure-niederalkylester, z.B. Acetessigsäure-äthylester, in Gegenwart einer Base, z.B. Natriumhydrid, und nachfolgende Hydrolyse mit einer starken Base, z.B. mit wässeriger Natronlauge, hergestellt werden.

Die genannten Verbindungen könnnen auch durch Kondensation einer Verbindung der Formel Ia, worin C Cyan bedeutet, z.B. mit einem Grignard- oder einem anderen metallorganischen Reagens, z.B. Methylmagnesium-bromid, unter Standardbedingungen hergestellt werden.

Augangsstoffe der Formel Ia, worin C Carboxycarbonyl (—COCOOH) bedeutet, können durch thermische Behandlung oder mittels Oxidation in Verbindungen der Formel I, worin B Carboxy bedeutet, überführt werden. Der Ausgangsstoff wird dazu bei erhöhter Temperatur, z.B. ungefähr 200°C, in Gegenwart von Glaspulver erhitzt, oder z.B. mit Wasserstoffperoxid in Gegenwart einer Base, z.B. Natriumhydroxid, behandelt.

Die Ausgangsstoffe der Formel Ia, worin C Carboxycarbonyl bedeutet, können z.B. durch Kondensation einer Verbindung der Formel Ia, worin C Halogenmethyl bedeutet, z.B. mit 2-Aethoxycarbonyl-1,3-dithian, nachfolgende oxidative Hydrolyse, z.B. mit N-Bromsuccinimid in wässerigem Aceton, und schliesslich Behandlung mit z.B. verdünnter wässeriger Natronlauge erhalten werden.

Verbindungen der Formel Ia, worin C Formyl, Di-niederalkoxy-methyl oder Akylendioxymethyl — letzteres entspricht als Acetal geschütztem Formyl— z.B. Dimethylacetal, bedeutet, könnnen z.B mit Silbernitrat, Pyridiniumdichromat oder Ozon zu den entsprechenden Verbindungen der Formel I, worin B Carboxy bedeutet, oxidiert werden.

Verbindungen der Formel Ia, worin C Vinyl bedeutet, können in Verbindungen der Formel I, worin B Carboxy ist, umgewandelt werden. Dazu werden sie zuerst durch Ozonolyse in Verbindungen der Formel I, worin B Formyl bedeutet, überführt, die ihrerseits in an sich bekannter Weise zu Verbindungen der Formel I, worin B Carboxy bedeutet, oxidiert werden können.

Ausgangsstoffe der Formel Ia, worin C Vinyl bedeutet, können auch mit Nickelcarbonyl und Kohlenmonoxid unter erhöhtem Druck behandelt werden, wobei man Verbindungen der Formel I, worin B Carboxy bedeutet und die Kette A in Nachbarschaft zur Carboxygruppe eine Doppelbindung trägt, erhält.

Verbindungen der Formel Ia, worin C Aethinyl bedeutet, können mit einer starken Base, z.B. Butyllithium, bei einer Temperatur von $-70°$ bis $+50°C$ behandelt, dann mit Kohlendioxid oder einem Halogenameisensäure-niederalkylester, z.B. Chlorameisensäureäthylester, kondensiert und nachfolgend hydrolysiert werden. Man erhält Verbindungen der Formel I, worin B Carboxy bedeutet und die Kette A in Nachbarstellung zur Carboxygruppe eine Dreifachbindung enthält.

Verbindungen der Formel Ia, worin C Halogenmethyl bedeutet, können in die entsprechenden metallorganischen Zwischenprodukte, z.B. Kupfer- oder Magnesiumderivate, nach an sich bekannten Methoden umgewandelt werden.

Die Umsetzung z.B. eines erhaltenen organischen Magnesium-(Grignard)-Reagenses, z.B. einer Verbindung der Formel Ia, worin C in $CH_2MgCl$ umgewandelt ist, mit Kohlendioxid, ergibt eine Verbindung der Formel I, worin B Carboxy bedeutet und die Kette um eine Kohlenstoffatom verlängert ist.

Die Reaktion des genannten Grignard-Reagenses z.B. mit einem Halogenessigsäure-niederalkylester, z.B. Bromessigsäure-äthylester, und nachfolgende Hydrolyse ergibt eine Verbindung der Formel I, worin B Carboxy bedeutet und die Kette um 2 Kohlenstoffatome verlängert ist.

Das genannte Grignard-Reagens kann in Gengwart eines Kupfer-I-halogenids, z.B. Kupfer-I-chlorids, mit einer α,β-ungesättigten Säure, z.B. mit Propiolsäure oder Acrylsäure, kondensiert werden, wobei man einer Verbindung der Formel I erhält, worin B carboxy bedeutet und die Kette durch 3 Kohlenstoffatome verlängert ist.

Ferner können Verbindungen der Formel Ia, worin C Halogenmethyl bedeutet, z.B. mit dem 3-Lithio-Derivat der Propiolsäure (hergestellt in situ aus Propiolsäure und z.B. Lithium-diisopropylamid) kondensiert werden, wobei man eine Verbindung der Formel I erhält, in welcher A terminales Alkinylen enthält, B für Carboxy steht und die Kette um 3 Kohlenstoffatome verlängert ist.

Verbindungen der Formel I, worin A Niederalkylen bedeutet und B für Hydroxymethyl oder insbesondere ein reaktionsfähiges funktionelles Derivat davon steht, können mit einem Niederalkanol (oder Niederalkanthiol), oder einem Phenol (oder Thiophenol), die jeweils in geeigneter Weise durch B substituiert sind, vorzugsweise in Gegenwart einer starken Base, umgesetzt werden. Man erhält Verbindungen der Formel I, worin A Niederalkylen-(thio oder oxy)-phenylen oder Niederalkylen-(thio oder oxy)-niederalkylen bedeutet.

Wenn irgendwelche der genannten Zwischenprodukte störende reaktionsfähige Gruppen, z.B. Carboxy-, Hydroxy- oder Aminogruppen, enthalten, können solche vorzugsweise vorübergehend, auf jeder Stufe, durch leichtabspaltbare Schutzgruppen geschützt werden. Der Zweck der Einführung von Schutzgruppen besteht darin, funktionelle Gruppen vor unerwünschten Reaktionen mit Reaktionspartnern zu bewahren und dadurch zu verhindern, dass die abgespalten oder in Derivate umgewandelt werden. Auf der anderen Seite kann es geschehen, dass bei Anwesenheit ungeschützter funktioneller Gruppen Reaktionspartner in unerwünschter Weise durch Reaktion mit ersteren verbraucht oder gebunden werden und daher für die eigentlich vorgesehene Reaktion nicht zur Verfügung stehen. Die Wahl der Schutzgruppe für eine bestimmte Reaktionhängt von verschiedenen Punkten ab, nämlich von der Art der zu schützenden funktionellen Gruppe, der Struktur und Stabilität des Moleküls, an dem die funktionelle Gruppe sitzt, und den Reaktionsbedingungen. Schutzgruppen, die diese Bedingungen erfüllen, ihre Einführung und Entfernung sind an sich bekannt und beschrieben, z.B. in J. F. W. McOmie, "Protective Groups in Organic Chemistry", Plenum Press, London, New York 1973. So können Carboxygruppen z.B. als Ester geschützt werden, z.B als unsubstituierte oder substituierte Niederalkylester, wie etwa dem Methyl- oder Benzylester, wobei es möglich ist, solche Estergruppen unter milden Bedingungen, insbesondere alkalischen Bedingungen, wieder leicht abzuspalten. Amino- oder Hydroxy-Schutzgruppen, die unter schonenden Bedingungen wieder abgespalten werden können, sind z.B. Acylradikale, wie etwa gegebenenfalls durch Halogen substituiertes Nieder alkanoyl, z.B. Formyl oder Trichloracetyl, oder organische Silylgruppen, z.B. Triniederalkylsilyl, wie etwa Trimethylsilyl. In einer Verbindung der Formel I oder in irgendeinem Zwischenprodukt, die eine oder mehrere funktionelle Gruppen in geschützter Form enthalten, z.B. geschütztes Carboxy, Amino oder Hydroxy, können letztere in an sich bekannter Weise z.B. durch Solvolyse, etwa Hydrolyse, freigesetzt werden.

Die obengenannten Reaktionen werden nach an sich bekannten Methoden, in Gegenwart oder Abwesenheit von Verdünnungsmitteln, vorzugsweise in solchen, die gegenüber den Reagenzien inert sind und diese lösen, Katalysatoren, Kondensations- oder anderen obengenannten Mitteln, und/oder einer inerten Atmosphäre, unter Kühlung, bei Zimmertemperatur oder bei erhöhter Temperatur, vorzugsweise

beim Siedepunkt des verwendeten Lösungsmittels, bei normalem oder erhöhtem Druck, durchgeführt. Die bevorzugten Lösungsmittel, Katalysatoren und Reaktionsbedingungen sind in den beigefügten Beispielen offenbart.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin einer oder mehrere der Ausgangsstoffe in Form eines Salzes oder eines optisch reinen Antipoden verwendet wird.

Im Verfahren der vorliegenden Erfindung werden bevorzugt solche Ausgangsstoffe verwendet, die zu den im Vorstehenden als besonders wertvoll beschriebenen Verbindungen führen.

Die Erfindung betrifft auch neue Ausgangsstoffe und Verfahren zu ihrer Herstellung.

Abhängig von der Wahl der Ausgangsstoffe und dem verwendeten Verfahren können die neuen Verbindungen in Form eines der möglichen Isomeren oder als Isomerengemische vorliegen. So können sie, abhängig von der Gegenwart einer Doppelbindung und von der Anzahl der asymmetrischen Kohlenstoffatome, z.B. reine optische Isomeren, etwa Antipoden, oder Gemische von optischen Isomeren, z.B. Racemate, Gemische von Diastereomeren, Gemische von Racematen oder Gemische von geometrischen Isomeren sein. Die vorher genannten möglichen Isomeren und ihre Gemische gehören zum Umfang der vorliegenden Erfindung. Bestimmte spezifische Isomeren können bevorzugt sein.

Erhaltene Gemische von Diastereomeren, Gemische von Racematen oder geometrischen Isomeren können auf der Basis der physikochemischen Unterschiede ihrer Komponenten in an sich bekannter Weise in die reinen Isomeren, Diasteromeren, Racemate oder geometrischen Isomeren, z.B. durch Chromatographie und/oder fraktionierte Kristallisation, getrennt werden.

Erhaltene Racemate können weiter in die optischen Antipoden in an sich bekannter Weise, z.B. durch Umsetzung eines sauren Endproduktes mit einer optische aktiven Base, die mit der racemischen Säure Salze bildet, aufgetrennt werden. Diese Salze können z.B. durch fraktionierte Kristallisation in die diastereomeren Salze getrennt werden. Aus letzteren können die optisch aktiven Säure-Antipoden durch Ansäuren freigesetzt werden. Basische racemische produkte können in analoger Weise, z.B. durch Trennung ihrer diastereomeren Salze mit einer optisch aktiven Säure und Freisetzung der optisch aktiven basischen Endprodukte mit einer gewöhnlichen Base, getrennt werden. Racemische Produkte der Erfindung können auf diese Weise in ihre optische Antipoden gespalten werden, z.B. durch fraktionierte Kristallisation von d- oder 1-(tartraten, Mandelaten, Camphersulfonaten, oder von d- oder 1-(α-Methylbenzylamin, Cinchonidin, Cinchonin, Chinin, Chinidin, Ephedrin, Dehydroabietylamin, Brucin oder Strychin)-salzen. Vorzugsweise wird von zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz vorzugsweise mit Säuren, die therapeutisch verwendbare Säureadditionssalz ergeben, oder mit Anionenaustauschern überführt werden. Erhaltene Salze können in die entsprechenden freien Basen z.B. durch Behandlung mit einer stärkeren Base, wie einem Metall- oder Ammoniumhydroxid oder einem basischen Salz, z.B. einem Alkalimetallhydroxid oder -carbonat, oder mit einem Kationenaustauscher umgewandelt werden. Eine Verbindung der Formel I, in welcher B Carboxy bedeutet, kann auf diese Weise auch in die entsprechenden Metall- oder Ammoniumsalze überführt werden. Diese oder andere Salze, z.B. die Pikrate, können auch in der Reinigung von erhaltenen freien Basen verwendet werden. Die Basen werden in ihre Salze überführt, die Salze abgetrennt und die Basen aus den Salzen freigesetzt.

Infolge der engen Beziehung zwischen den neuen Verbindungen in freier Form und in Form ihrer Salze sind im Vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere, für die Kristallisation verwendet Lösungsmittel einschliessen.

Die erfindungsgemässen pharmazeutischen Präparate sind solche, die sich für enterale, z.B. orale oder rektale, und parenterale Verabreichung an Säuger, einschliesslich Menschen, eignen. Diese Präparate enthalten eine wirksame Dosis wenigstens einer pharmakologisch aktiven Verbindung der Formel I, oder eines ihrer pharmazeutisch verwendbaren Salze, allein oder in Kombination mit einem oder mehreren pharmazeutisch annehmbaren Trägermaterialien.

Die pharmakologisch wirksamen Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder ihren Salzen, wie Magnesium- oder Calciumstearat, und/oder Polyäthylenglykol, enthalten; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen und/oder

Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten etwa 0,1% bis etwa 75%, insbesondere etwa 1% bis etwa 50%, des Altivstoffes. Einzeldosen für Säuger mit einem Gewicht von ungefähr 50 bis 70 kg können ungefähr 10 bis 100 mg des aktiven Bestandteils enthalten.

Die folgenden Beispiele dienen zur Illustration der Erfindung und sind nicht als Einschränkung ihres Umfangs aufzufassen. Temperaturen werden in Celsiusgraden angegeben, und Angaben über Teile betreffen Gewichtsteile. Wenn nicht anders definiert, wird das Eindampfen von Lösungsmitteln unter vermindertem Druck, vorzugsweise zwischen ungefähr 20 und 130 mbar, durchgeführt.

## Beispiel 1

Ein Gemisch von 10,32 g Phenylhydrazin-Hydrochlorid und 16,8 g 7-Nikotinoylheptancarbonsäure in 750 ml Aethanol wird 6 h bei Rückflusstemperatur erhitzt. Nach dem Abkühlen in einem Eis-Wasserbad werden 250 ml etwa 2 n äthanolischen Chlorwasserstoffs zugegeben, und das Gemisch wird 16 h unter Rückfluss erhitzt. Das Gemisch wird filtriert und das Filtrat unter vermindertem Druck eingeengt. Das Rohprodukt wird mit Petroläther behandelt und aus Aethanol/Aether umkristallisiert; man erhält 3-[5-(Aethoxycarbonyl)-pentyl]-2-(3-pyridyl)-indol-Hydrochlorid, Fp. 145 bis 150°.

Die Ausgangsverbindung wird wie folgt hergestellt:

Zu einer Suspension von 16 g 50-proz. Natriumhydrid in 450 ml Aether werden tropfenweise 92,2 g Korksäure-diäthylester und 27,3 ml (30,2 g) Nikotinsäure-äthylester bei Raumtemperatur und unter Stickstoff zugegeben. Das Reaktionsgemisch wird bei Rückflusstemperatur über Nacht erhitzt und, nach Abkühlen, mit 400 ml Eiswasser versetzt. Die Aetherphase wird abgetrennt und verworfen, die wässerige Phase mit 1 n Salzsäure auf pH 5 eingestellt und mit Aether extrahiert (3 × 250 ml). Die Aetherphase wird getrocknet und konzentriert; man erhält 7-Aethoxycarbonyl-7-nikotinoylheptancarbonsäure-äthylester als Oel. Eine Lösung dieses Esters in 300 ml 1 m Schwefelsäure wird 12 Stunden bei Rückflusstemperatur erhitzt. Die Reaktionslösung wird in einem Eisbad abgekühlt und auf pH 4,5 bis 5,0 mit gesättigter Natriumhydrogencarbonatlösung eingestellt. Ein weisser Feststoff wird gesammelt und nach dem Waschen mit Wasser und dem Trocknen an der Luft aus Aethanol umkristallisiert; man erhält 7-Nikotinoylheptancarbonsäure, Fp 113 bis 115°.

## Beispiel 2

Eine Lösung von 7,46 g 3-[5-(Aethoxycarbonyl)-pentyl]-2-(3-pyridyl)-indol in 2 n wässeriger Salzsäure (275 ml) wird 20 h unter Rückfluss erhitzt. Die heisse Reaktionsmischung wird filtriert; das Filtrat gibt beim Abkühlen gelbe Kristalle, die durch Filtrieren gesammelt und getrocknet werden; man erhält 3-(5-Carboxypentyl)-2-(3-pyridyl)-indol-Hydrochlorid, Fp. 220 bis 222°.

Eine wässerige Lösung von 10,5 g 3-(5-Carboxypentyl)-2-(3-pyridyl)-indol-Hydrochlorid wird auf einen pH-Wert von etwa 7 mit gesättigter Natriumhydrogencarbonatlösung neutralisiert und mit Aether extrahiert. Der Aetherextrakt wird getrocknet und im Vakuum eingeengt; man erhält 3-(5-Carboxypentyl)-2-(3-pyridyl)-indol.

## Beispiel 3

Zu einer Suspension von 1,2 g Natriumhydrid (60% in Mineralöl) in 50 ml Dimethylformamid wird unter Stickstoffschutz tropfenweise eine Lösung von 8,8 g 3-(5-Carboxypentyl)-2-(3-pyridyl)-indol in 50 ml Dimethylformamid bei Raumtemperatur gegeben. Nach der Zugabe wird das Gemisch 0,5 h gerührt, auf 0 bis 5° abgekühlt und mit 1,62 ml Methyliodid behandelt. Das Gemisch wird über Nacht gerührt, in Eiswasser gegossen und mit 1 n Salzsäure angesäuert. Nach der Extraktion mit Aether wird die wässerige Phase auf einen pH-Wert von 9 bis 10 mit gesättigter Natriumhydrogen-carbonatlösung eingestellt und zweimal mit je 300 ml Aether extrahiert. Die Aetherphase wird mit Natriumchloridlösung gewaschen, getrocknet ($MgSO_4$) und zu einem Oel eingeengt.

Eine Lösung des Rückstands in 300 ml 2 n Salzsäure wird 2 h bei Rückflusstemperatur erhitzt. Nach dem Einengen im Vakuum wird eine Natriumhydrogencarbonatlösung zugegeben, um den pH-Wert auf 5,5 bis 6,5 einzustellen. Der sich bildende gummiartige Feststoff wird mit Methylenchlorid extrahiert. Die organische Phase wird getrocknet und eingedampft und der Rückstand aus Acetonitril kristallisiert; man erhält 3-(5-Carboxypentyl)-1-methyl-2-(3-pyridyl)-indol, Fp. 128 bis 130°.

## Beispiel 4

a) Gemäss Beispiel 1 erhält man durch Kondensation von p-Methoxyphenylhydrazin mit 7-Nikotinoylheptancarbonsäure 2-(3-Pyridyl)-3-[5-(äthoxycarbonyl)-pentyl]-5-methoxyindol-Hydrochlorid, Fp. 152 bis 154°.

b) Die folgende Hydrolyse gemäss Beispiel 2 gibt 2-(3-Pyridyl)-3-(5-carboxypentyl)-5-methoxyindol, Fp. 185 bis 187°.

Beispiel 5

Eine Lösung von 1,5 g 2-(3-Pyridyl)-3-[5-(äthoxycarbonyl)-pentyl]-5-methoxyindol in 15 ml 48-proz. Bromwasserstoffsäure wird 2,5 h bei Rückflusstemperatur erhitzt. Die Reaktionslösung wird abgekühlt und mit Natriumhydrogencarbonatlösung auf pH 6 bis 7 neutralisiert und dann mit Aethylacetat extrahiert. Der organische Extrakt wird getrocknet ($MgSO_4$), filtriert und zu einem Feststoff eingeengt, der aus Acetonitril umkristallisiert wird; man erhält 5-Hydroxy-2-(3-pyridyl)-3-(5-carboxypentyl)-indol, Fp. 185 bis 187°.

Beispiel 6

Ein Gemisch von 9,60 g 7-Nikotinoylheptancarbonsäure und 7,34 g 4-Chlorphenylhydrazin in 100 ml Aethanol wird gerührt und unter Stickstoffschutz über Nacht unter Rückfluss erhitzt. Das Reaktionsgemisch wird im Vakuum zu einem gummiartigen Produkt eingeengt, das in 100 ml Aethanol suspendiert, mit 40 ml 6 n äthanolischem Chlorwasserstoff behandelt und unter Stickstoff 22 h unter Rückfluss erhitzt wird. Die Suspension wird abgekühlt und mit zusätzlichen 50 ml 6 n äthanolischem Chlorwasserstoff versetzt. Das Erhitzen unter Rückfluss wird wieder aufgenommen und weitere etya 20 h fortgesetzt. Die Suspension wird in einem Eis-Wasserbad gekühlt und filtriert. Das Filtrat wird im Vakuum zu einem teilweise festen Rückstand eingeengt, der mit Aether/Aethanol (10:1) behandelt wird; der gebildete gelbe Feststoff wird gesammelt; man erhält 3-[5-(Aethoxycarbonyl)-pentyl]-5-chlor-2-(3-pyridyl)-indol-Hydrochlorid, Fp. 141 bis 145°.

Die Hydrolyse mit 2 n wässeriger Salzsäure gemäss Beispiel 2 gibt 3-(5-Carboxypentyl)-5-chlor-2-(3-pyridyl)-indol-Hydrochlorid, Fp. 255 bis 257°.

Beispiel 7

Zu einer Suspension von Natriumhydrid in 10 ml trockenem Dimethylformamid wird unter Rühren und Stickstoffschutz bei 0° tropfenweise eine Lösung von 2,12 g 5-Chlor-3-[5-(äthoxycarbonyl)-pentyl]-2-(3-pyridyl)-indol in Dimethylformamid gegeben. Nach vollständiger Zugabe wird die orangefarbene Suspension bei 0° 0,5 h gerührt. Zu der Suspension wird 0,42 ml Jodmethan gegeben. Man lässt die Suspension auf Raumtemperatur erwärmen. Nch dem Rühren über Nacht bei Raumtemperatur wird das Reaktionsgemisch in Wasser (80 ml) gegossen und mit Aether extrahiert (3 × 50 ml). Der Aetherextrakt wird mit Wasser und einer gesättigten Natriumchloridlösung gewaschen, getrocknet ($MgSO_4$), filtriert und im Vakuum eingeengt; man erhält 5-Chlor-3-[5-(äthoxycarbonyl)-pentyl]-1-methyl-2-(3-pyridyl)-indol.

Beispiel 8

Ein Gemisch von 1,0 g 5-Chlor-3-[5-(äthoxycarbonyl)-pentyl]-1-methyl-2-(3-pyridyl)-indol in 20 ml 2 n wässeriger Salzsäure wird über Nacht gerührt und unter Rückfluss erhitzt. Das Gemisch wird abgekühlt; der gebildete Feststoff wird gesamelt und im Vakuum getrocknet; man erhält 3-(5-Carboxypentyl)-5-chlor-1-methyl-2-(3-pyridyl)-indol-Hydrochlorid, Fp. 186 bis 189°.

Beispiel 9

a) Eine Lösung von 3-(4-Cyanbenzyl)-2-(3-pyridyl)-indol (5,8 g) in 100 ml eines 1:1-Gemisches von 20-proz. wässeriger Salzsäure und Eisessig wird unter Rückfluss 20 h erhitzt. Nach dem Abkühlen wird die Lösung in Eiswasser (100 ml) gegossen und der pH-Wert auf 4,5 bis 5 mit gesättigter Natriumhydrogencarbonatlösung eingestellt. Der entstandene Niederschlag wird mit Aethylacetat extrahiert; der Aethylacetatextrakt wird mit Wasser gewaschen und zur Trockene eingedampft; man erhält 3-(4-Carboxybenzyl)-2-(3-pyridyl)-indol.

Das Ausgangsnitril wird wie folgt hergestellt:

Zu 30 ml einer 2 m Aethylmagnesiumbromidlösung in Tetrahydrofuran wird unter Stickstoffschutz bei 0 bis 5° tropfenweise im Verlauf von 20 min eine Lösung von 10,0 g 2-(3-Pyridyl)-indol in 60 ml Tetrahydrofuran gegeben. Das Reaktionsgemisch wird 0,5 h bei 0 bis 5° gerührt, dann tropfenweise mit 9,8 g p-Cyanbenzylbromid in 50 ml Tetrahydrofuran versetzt. Man rührt 1 h bei 0 bis 10°, 0,5 h bei Raumtemperatur und giesst dann das Reaktionsgemisch in Eiswasser (600 ml). Der entstandene Feststoff wird gesammelt, getrocknet, mit Petroläther gewaschen und in Aether (500 ml) wieder aufgelöst. Die Aetherlösung wird zuerst mit Wasser, dann mit gesättigter Natriumhydrogencarbonatlösung gewaschen, über $MgSO_4$ getrocknet, mit Kohle behandelt und filtriert. Nach dem Eindampfen des Aetherextrakts zur Trockene und anschliessender Reinigung erhält man 3-(4-Cyanbenzyl)-2-(3-pyridyl)-indol.

b) Entsprechend wird 3-(4-Carboxybenzyl)-5-chlor-2-(3-pyridyl)-indol hergestellt.

Beispiel 10

a) Zu einer Suspension von 0,49 g Lithiumaluminiumhydrid in 50 ml wasserfreiem Tetrahydrofuran wird unter Stickstoff tropfenweise bei Raumtemperatur eine Lösung von 4,09 g 3-(5-Aethoxycarbonylpentyl)-5-chlor-1-methyl-2-(3-pyridyl)-indol in 30 ml wasserfreiem Tetrahydrofuran gegeben. Nach beendeter Zugabe wird die Suspension 1 h bei Raumtemperatur gerührt und mit 50 ml einer gesättigten

Ammoniumchloridlösung versetzt. Das Reaktionsgemisch wird bei Raumtemperatur über Nacht stehengelassen, dann die organische Phase abgetrennt. Die wässerige Phase wird zur Entfernung der Salze filtriert und mit Aethylacetat extrahiert (2 × 50 ml). Die vereinigten organischen Extrakte werden mit gesättigter Natriumchloridlösung gewaschen, über Magnesiumsulfat getrocknet und im Vakuum eingeengt. Das Rohprodukt wird durch Behandeln mit Hexan/Aether gereinigt und in Aethanol gelöst. Die Lösung wird mit äthanolischer Salzsäure bis zur sauren Reaktion versetzt und mit wasserfreiem Aether verdünnt, um das Produkt auszukristallisieren. Man erhält 5-Chlor-3-(6-hydroxyhexyl)-1-methyl-2-(3-pyridyl)-indol-Hydrochlorid.

b) Entsprechend wird 3-(6-Hydroxyhexyl)-1-methyl-2-(3-pyridyl)-indol hergestellt.

Beispiel 11

Zu einer Suspension von 1,52 g 3-(5-Carboxypentyl)-5-chlor-1-methyl-2-(3-pyridyl)-indol in 50 ml Toluol werden unter Stickstoffschutz tropfenweise bei Raumtemperatur 0,31 ml Thionylchlorid zugegeben. Das entstandene Gemisch wird 1 h unter Rückfluss erhitzt. Die Lösung wird mit weiteren 0,10 ml Thionylchlorid versetzt und bei Raumtemperatur über Nacht gerührt. Die entstandene Suspension wird zur Trockene eingedampt; man erhält 3-(5-Chlorcarbonylpentyl)-5-chlor-1-methyl-2-(3-pyridyl)-indol. Eine Suspension dieses Säurechlorids in 20 ml konzentrierter Ammoniaklösung wie bei Raumtemperatur über Nacht gerührt. Der entstandene Feststoff wird abfiltriert; man erhält 3-(5-Carbamoylpentyl)-5-chlor-1-methyl-2-(3-pyridyl)-indol.

Beispiel 12

Eine Lösung von 4 g 3-(5-Aethoxycarbonylpentyl)-1-methyl-2-(3-pyridyl)-indol in 40 ml n-Butanol wird mit Methylamin gesättigt und erhitzt, und zwar in einem Wasserdampfbad in einer Druckflasche während drei Tagen. Das Reaktionsgemisch wird zur Trockene eingedampft und das Produkt aus Diäthyläther kristallisiert; man erhält 3-[5-(N-Methylcarbamoyl)-pentyl]-1-methyl-2-(3-pyridyl)-indol.

Beispiel 13

Eine Lösung von 50 mg 3-(5-Carbamoylpentyl)-5-chlor-1-methyl-2-(3-pyridyl)-indol in 1 ml 6 n Salzsäure wird 3 Stunden bei Rückflusstemperatur erhitzt. Beim Abkühlen fällt das Hydrochlorid (Salz) aus. Die Suspension wird zur Trockene eingeengt und der Rückstand mit gesättigter Natriumhydrogencarbonatlösung alkalisch gemacht. Diese Lösung wird mit Aether gewaschen und auf pH 6 bis 7 mit 2 n Salzsäure neutralisiert. Nach der Extraktion mit Methylenchlorid erhält man 3-(5-Carboxypentyl)-5-chlor-1-methyl-2-(3-pyridyl)-indol, das durch Behandlung mit 2 n Salzsäure in das entsprechende Hydrochlorid, Fp. 186 bis 189°, überführt wird.

Beispiel 14

Zu 30 ml einer 2 m Aethylmagnesiumbromidlösung in Tetrahydrofuran wird unter Stickstoff bei 0 bis 5° tropfenweise im Verlauf von 30 min eine Lösung von 10,0 g 2-(3-Pyridyl)-indol in 60 ml Tetrahydrofuran gegeben. Das Gemisch wird 0,5 h bei 0 bis 5° gerührt, dann tropfenweise mit 17,6 g 1-Tetrahydropyranyloxy-8-brom-oktan in 50 ml Tetrahydrofuran versetzt. Das Reaktionsgemisch wird bei 0 bis 10° 1 h und bei Raumtemperatur 0,5 h gerührt, in Eiswasser gegossen und mit Aether extrahiert. Der Aetherextrakt wird mit Wasser gewaschen, über $MgSO_4$ getrocknet und zur Trockene eingedampft. Der Rückstand wird in 100 ml 3 n Salzsäure gelöst, das entstandene Gemisch wird bei Raumtemperatur 0,5 h stehengelassen, mit Aether gewaschen, mit 3 n wässeriger Natriumhydroxidlösung alkalisch gemacht und mit Methylenchlorid extrahiert. Die Methylenchloridlösung wird zur Trockene eingedampft; man erhält 3-(8-Hydroxyoktyl)-2-(3-pyridyl)-indol.

Beispiel 15

Zu 12 ml einer 2 m Aethylmagnesiumbromidlösung in Tetrahydrofuran wird unter Stickstoffschutz bei 0 bis 5° tropfenweise im Verlauf von 20 min eine Lösung von 4,0 g 2-(3-Pyridyl)-indol in 20 ml Tetrahydrofuran gegeben. Das Gemisch wird 0,5 h bei 0 bis 5° gerührt und dann mit einer Lösung von 5,06 g p-(2-Bromäthoxy)-benzoesäure-äthylester [zur Herstellung siehe US-Patent 2,790,825 (1957)] in 30 ml Tetrahydrofuran versetzt. Die Suspension wird bei 0 bis 10° 1 h und bei Raumtemperatur 0,5 h gerührt, in Eiswasser gegossen und mit Aether extrahiert. Der Aetherextrakt wird abgetrennt, über Magnesiumsulfat getrocknet und eingeengt; man erhält 3-[2-(4-Aethoxycarbonylphenoxy)-äthyl]-2-(3-pyridyl)-indol.

Beispiel 16

Ein Gemisch von 4,5 g 3-[2-(4-Aethoxycarbonylphenoxy)-äthyl]-2-(3-pyridyl)-indol in 220 ml 2 n Salzsäure wird 6 h unter Rückfluss erhitzt. Nach dem Abkühlen wird die Lösung mit 3 n Natronlauge alkalisch gemacht und mit Aethylacetat extrahiert. Die basische Lösung wird filtriert und mit 3 n Salzsäure auf pH 6 bis 7 gebracht. Der Feststoff wird gesammelt und getrocknet; man erhält 3-[2-(4-Carboxyphenoxy)-äthyl]-2-(3-pyridyl)-indol.

Beispiel 17

Eine Lösung von 5,9 g p-Mercaptobenzoesäure-äthylester (hergestellt gemäss J. Chem. Soc. *1963,* Seiten 1947 bis 1954) in 30 ml Dimethylformamid wird tropfenweise zu einer Aufschlämmung von 1,55 g 50-proz. Natriumhydrid (Dispersion in Mineralöl) in 30 ml Dimethylformamid gegeben. Dieses Gemisch wird bei Raumtemperatur 0,5 h unter Stickstoffatmosphäre gerührt und tropfenweise zu einer Lösung von 9,78 g 3-(2-Methylsulfonyloxyäthyl)-2-(3-pyridyl)-indol in 60 ml Dimethylformamid bei −10° gegeben. Dieses Gemisch wird bei Raumtemperatur über Nacht gerührt, in 1 l Eiswasser gegossen und dann einige Male mit Aether extrahiert. Der Aetherextrakt wird mit Wasser gewaschen, über Magnesiumsulfat getrocknet und im Vakuum eingedampft; man erhält 3-[2-(4-Aethoxycarbonylphenylthio)-äthyl]-2-(3-pyridyl)-indol.

Die Ausgangsverbindung kann wie folgt hergestellt werden:

Zu 10,0 g 3-(2-Aethoxycarbonyläthyl)-2-(3-pyridyl)-indol in 400 ml trockenem tetrahydrofuran werden bei 0° 60 ml einer 1 m Lithiumaluminiumhydridlösung in Tetrahydrofuran gegeben. Das Gemisch wird bei Raumtemperatur 1 h gerührt, dann in einem Eisbad gekühlt und nacheinander mit 2,26 ml Wasser, 2,26 ml einer 15-proz. Natriumhydroxidlösung und 6,78 ml Wasser versetzt. Das Gemisch wird filtriert und im Vakuum eingeengt; der Rückstand wird in Aether gelöst, mit gesättigter Natriumhydrogencarbonatlösung gewaschen, über Magnesiumsulfat getrocknet und im Vakuum eingeengt; man erhält 3-(2-Hydroxyäthyl)-2-(3-pyridyl)-indol.

Methansulfonylchlorid (2,50 ml) wird tropfenweise zu einer Lösung von 7,5 g 3-(2-Hydroxyäthyl)-2-(3-pyridyl)-indol und 10,0 ml Triäthylamin in 150 ml Methylenchlorid bei −10° gegeben. Dieses Gemisch wird bei Raumtemperatur 0,5 h gerührt und in 600 ml Eiswasser gegossen. Die entstandene Aufschlämmung wird mit Methylenchlorid extrahiert; der Extrakt wird mit gesättigter Natriumhydrogencarbonatlösung gewaschen, über Magnesiumsulfat getrocknet und im Vakuum eingedampft; man erhält 3-(2-Methylsulfonyloxyäthyl)-2-(3-pyridyl)-indol.

Beispiel 18

Ein Gemisch von 6,0 g 3-[2-(4-Aethoxycarbonylphenylthio)-äthyl]-2-(3-pyridyl)-indol in 250 ml 2 n Salzsäure wird bei Rückflusstemperatur 6 h erhitzt. Nach dem Abkühlen wird der pH-Wert auf 6 bis 7 mit gesättigter wässeriger Natriumhydrogencarbonatlösung (etwa 500 ml) eingestellt. Das entstandene Produkt wird gesammelt, zuerst mit Wasser dann mit Aether gewaschen und in 100 ml heissem absoluten Aethanol gelöst. Die Lösung wird filtriert und noch heiss mit 1,68 ml 6,5 n äthanolischem Chlorwasserstoff behandelt. Die Lösung wird abgekühlt und mit etwa 100 ml Aether verdünnt. Das entstandene 3-[2-(4-Carboxyphenylthio)-äthyl]-2-(3-pyridyl)-indol-Hydrochlorid wird gesammelt.

Beispiel 19

Eine Lösung von Lithiumdiisopropylamid (LDA) wird hergestellt, indem man n-Butyllithium (7,66 mM, 1,6 m in Hexan) zu einer Lösung von Diisopropylamin (7,6 mM) in Tetrahydrofuran (12 ml) bei −20° gibt. Die LDA-Lösung wird auf −78° abgekühlt und mit 3-(5-Aethoxycarbonylpentyl)-1-methyl-2-(3-pyridyl)-indol (2,48 g) in Tetrahydrofuran (24 ml) tropfenweise im Verlauf von 5 min versetzt. Das Gemisch wird bei −78° 20 min gerührt, dann mit Phenylselenylchlorid (1,5 g) in Tetrahydrofuran (12 ml) versetzt. Nach 5 min entfernt man das Kühlbad und lässt das Gemisch auf 0° erwärmen. Man gibt wässerige Natriumhydrogencarbonatlösung (60 ml) zu und extrahiert dann mit Aether (3 × 50 ml). Die vereinigten organischen Extrakte werden mit gesättigter Natriumhydrogencarbonatlösung und Natriumchloridlösung gewaschen und über wasserfreiem Magnesiumsulfat getrocknet. Nach dem Einengen im Vakuum erhält man rohes 3-(5-Aethoxycarbonyl-5-phenylselenylpentyl)-1-methyl-2-(3-pyridyl)-indol. Das rohe Selenid wird in Dichlormethan (40 ml) gelöst und mit 30-proz. Wasserstoffperoxid (1,8 g, 16 mM) in Wasser (1,8 ml) tropfenweise versetzt. Nach der Zugabe von etwa 10% des Wasserstoffperoxids wird die Reaktion exotherm. Die Temperatur steigt auf 30° am Ende der Zugabe. Es wird weitere 30 min gerührt, dann wird 5-proz. wässerige Natriumcarbonatlösung (40 ml) zugegeben. Die Dichlormethanphase wird abgetrennt. Die wässerige Phase wird mit Dichlormethan (25 ml) extrahiert. Die vereinigten organischen Phasen werden mit 5-proz. wässeriger Natriumcarbonatlösung, Wasser und Natriumchlorid gewaschen und über wasserfreiem Magnesiumsulfat getrocknet. Nach dem Einengen im Vakuum erhält man 3-(5-Aethoxycarbonylpent-4-enyl)-1-methyl-2-(3-pyridyl)-indol.

Beispiel 20

Zu einer Lösung des α,β-ungesättigten Esters 3-(5-Aethoxycarbonylpent-4-enyl)-1-methyl-2-(3-pyridyl)-indol (84 mg) in Methanol (1 ml) wird eine 1 n wässerige Lithiumhydroxidlösung (1 ml) zugegeben. Das Gemisch wird bei Raumtemperatur über Nacht gerührt, dann zur Trockene im Vakuum eingedampft. Der Rückstand wird in Wasser (2 ml) gelöst und mit Diäthyläther (5 ml) gewaschen. Die wässerige Phase wird mit Säure auf pH 6,6 bis 7,0 gebracht und mit Dichlormethan extrahiert. Der organische Extrakt wird mit Natriumchloridlösung gewaschen, über Magnesiumsulfat getrocknet und dann im Vakuum zu 3-(5-Carboxypent-4-enyl)-1-methyl-2-(3-pyridyl)-indol eingeengt.

Beispiel 21

Zu einer Lösung des Collins-Reagenz, hergestellt aus Chromtrioxid (5,6 g) und Pyridin (8,86 g, 112 mM) in Dichlormethan (150 ml) bei 0 bis 5° unter einer Stickstoffatmosphäre, werden auf einmal 1,8 g 3-(6-Hydroxyhexyl)-1-methyl-2-(3-pyridyl)-indol in Dichlormethan (15 ml) zugegeben. Das Gemisch wird weitere 30 min gerührt, dann durch Cellit filtriert. Das Filtrat wird durch eine Kieselgelsäule geleitet. Das Produkt wird vom Kieselgel mit einem Gemisch von Aethylacetat/Dichlormethan (500 ml) eluiert. Nach dem Einengen im Vakuum erhält man 3-(5-Formylpentyl)-1-methyl-2-(3-pyridyl)-indol.

Beispiel 22

Phosphonessigsäuretrimethylester (328 mg) wird tropfenweise zu einer Lösung von Kalium-tert.-butoxid (220 mg) in Tetrahydrofuran (5 ml) bei 0° unter Stickstoffatmosphäre gegeben. Die Lösung wird bei 0° 20 min gerührt, dann auf −78° abgekühlt. Eine Lösung von 3-(5-Formylpentyl)-1-methyl-2-(3-pyridyl)-indol (450 mg) in Tetrahydrofuran (5 ml) wird tropfenweise im Verlauf von 15 min zugegeben. Das Gemisch wird bei −78° 15 min stehengelassen, dann wird das Kühlbad entfernt. Das Gemisch wird über Nacht bei Raumtemperatur gerührt, dann mit Wasser (25 ml) verdünnt und mit Diäthyläther extrahiert (3 × 25 ml). Die vereinigten Extrakte werden mit gesättigter Natriumhydrogencarbonatlösung, dann mit Natriumchloridlösung gewaschen und über wasserfreiem Magnesiumsulfat getrocknet. Nach dem Einengen in Vakuum erhält man den α,β-ungesättigten Ester 3-(7-Methoxycarbonylhept-6-enyl)-1-methyl-2-(3-pyridyl)-indol.

Beispiel 23

Durch Hydrolyse von 50 mg 3-(7-Methoxycarbonylhept-6-enyl)-1-methyl-2-(3-pyridyl)-indol mit 1 n wässeriger Lithiumhydroxidlösung erhält man 3-(7-Carboxyhept-6-enyl)-1-methyl-2-(3-pyridyl)-indol.

Beispiel 24

3-(7-Carboxyhept-6-enyl)-1-methyl-2-(3-pyridyl)-indol (100 mg) wird in 10 ml absolutem Aethanol gelöst und mit einer katalytischen Menge von 10% Palladium-auf-Kohle bei einem Druck von 1 bar hydriert. Nach der Aufnahme von 1 Mol Wasserstoff wird der Katalysator durch Filtrieren entfernt und mit wenigen ml Aethanol gewaschen. Die vereinigten Filtrate werden im Vakuum eingeengt; man erhält 3-(7-Carboxyheptyl)-1-methyl-2-(3-pyridyl)-indol.

Beispiel 25

3-(4-Cyanbutyl)-2-(3-pyridyl)-indol (540 mg) wird bei 185° 0,5 h mit 450 mg gepulvertem Natriumhydroxid und 5 ml Aethylenglykol erhitzt; die Reaktionslösung wird mit 50 ml Wasser verdünnt, mit Aether gewaschen und mit 2 n Salzsäure auf pH 6 eingestellt; man erhält 3-(4-Carboxybutyl)-2-(3-pyridyl)-indol.

Das Ausgangsmaterial wird wie folgt hergestellt:

Zu 6,0 ml einer 2 m Aethylmagnesiumbromidlösung in Tetrahydrofuran wird unter Stickstoffschutz bei 0° tropfenweise im Verlauf von 20 min eine Lösung von 2-(3-Pyridyl)-indol (1,9 g) in 12 ml Tetrahydrofuran gegeben. Das Gemisch wird bei 0° 0,5 h gerührt und dann mit einer Lösung von 1,78 g 5-Bromvaleronitril in 4 ml Tetrahydrofuran behandelt. Dieses Gemisch wird bei 0° 1 h, dann bei Raumtemperatur 1 h gerührt und in 125 ml Eiswasser gegossen. Dieses Gemisch wird zweimal mit je 50 ml Aether extrahiert; der Extrakt wird mit Wasser gewaschen, getrocknet, zur Trockene eingedampft und gereinigt, man erhält 3-(4-Cyanbutyl)-2-(3-pyridyl)-indol.

Beispiel 26

Ein Gemisch von 540 mg 3-(4-Cyanbutyl)-2-(3-pyridyl)-indol, 173 mg Natriumazid, 142 mg Ammoniumchlorid und 5 mg Lithiumchlorid in 2 ml Dimethylformamid wird bei 120° über Nacht erhitzt. Nach dem Abkühlen wird das Gemisch filtriert und das Filtrat mit etwa 25 ml Wasser verdünnt. Nach dem Einstellen des pH-Wertes auf 10 bis 11 mit 3 n Natronlauge wird die Lösung mit Aether gewaschen, um nicht umgesteztes Nitril zu entfernen. Die wässerige Phase wird auf pH 5 bis 6 mit 2 n Salzsäure eingestellt und mit Aether extrahiert. Der Aetherextrakt wird mit Wasser gewaschen, über Magnesiumsulfat getrocknet und im Vakuum eingeengt. Der Rückstand wird gereinigt; man erhält 3-[4-(5-Tetrazolyl)-butyl]-2-(3-pyridyl)-indol.

Beispiel 27

a) Zu 6,0 ml einer 2 m Aethylmagnesiumbromidlösung in Tetrahydrofuran wird unter Stickstoffschutz bei 0° tropfenweise im Verlauf von 20 min eine Lösung von 2-(3-Pyridyl)-indol (1,9 g) in 12 ml Tetrahydrofuran gegeben. Nach vollständiger Zugabe wird das Gemisch bei 0° 0,5 h gerührt und dann tropfenweise mit einer Lösung von 2,39 g 3-(p-Chlormethylphenyl)-2-methylacrylsäure-äthylester in 5 ml Tetrahydrofuran behandelt. Das entstandene Gemisch wird bei 0° 1 h, dann bei Raumtemperatur 1 h gerührt und in 100 ml Eiswasser gegossen. Das entstandene Gemisch wird mit Aether (2 × 50 ml) extrahiert; die organische Phase wird mit 100 ml Natriumchloridlösung gewaschen, über

Magenesiumsulfat getrocknet und eingedampft; man erhält 3-[p-(2-Aethoxycarbonylpropen-1-yl)-benzyl]-2-(3-pyridyl)-indol.

b) Durch Hydrolyse mit 2 n wässeriger Salzsäure erhält man 3-[p-(2-Carboxypropen-1-yl)-benzyl]-2-(3-pyridyl)-indol.

Die Ausgangsverbindung wird wie folgt hergestellt:

Zu einer Suspension von 10,0 g 50% Natriumhydrid (Dispersion in Mineralöl) in frisch destilliertem Dimethoxyäthan (350 ml), die unter Stickstoffschutz bei 10° gerührt wird, werden 53,6 ml 2-Phosphonpropionsäure-triäthylester im Verlauf von etwa 40 min gegeben. Das Gemisch wird gerührt, und zwar 0,5 h bei 10° und dann weitere 1,5 h, in denen man die Temperatur auf Raumtemperatur ansteigen lässt. Diese Lösung wird unter Stickstoffschutz mit einer Kanüle in einen 500 ml-Tropftrichter übergeführt und tropfenweise zu einer Lösung von Terephthalaldehyd (33,53 g) in trockenem Dimethoxyäthan (475 ml) zugegeben, und zwar im Verlauf von 1 h bei 22 bis 34°. Nach beendeter Zugabe wird das Reaktionsgemisch mechanisch bei Raumtemperatur 2 h gerührt, in 1 l Wasser gegossen und viermal mit je 500 ml Aether extrahiert. Der Aetherextrakt wird mit gesättigter Natriumchloridlösung (700 ml) gewaschen, über Magnesiumsulfat getrocknet, filtriert und im Vakuum zu einem gelben Oel eingeengt, das beim Stehenlassen teilweise kristallisiert. Dieses Rohgemisch wird durch Suspendieren in Petroläther und Aethylacetat (93:7) gereinigt. Das Filtrat wird nach Entfernen des nicht umgesetzten Dialdehyds im Vakuum eingeengt; man erhält ein Gemisch, das durch Hochdruck-Flüssigchromatographie [mit Petroläther/Aethylacetat (93:7)] weiter gereinigt wird. Man erhält den reinen 4-Formyl-α-methylzimtsäure-äthylester. Eine Lösung dieses Aldehyds (34,80 g) in 820 ml absolutem Aethanol wird mit 12,11 g granuliertem Natriumborhydrid bei Raumtemperatur und Stickstoffschutz behandelt. Das entstandene Gemisch wird bei Raumtemperatur 3 h gerührt (oder bis das gesamte Borhydrid aufgelöst ist), dann auf ein Volumen von etwa 200 ml eingeengt, mit 400 ml Wasser verdünnt und dreimal mit je 200 ml Aether extrahiert. Der Aetherextrakt wird mit 100 ml Wasser und Natriumchloridlösung (100 ml) gewaschen, über Magnesiumsulfat getrocknet und filtriert; das Filtrat wird im Vakuum zu 3-(p-Hydroxymethylphenyl)-2-methacrylsäure-äthylester eingeengt. Zu einer Lösung dieses Produkts in 350 ml Methylenchlorid werden bei Raumtemperatur 11,53 ml Thionylchlorid zugegeben, und zwar tropfenweise im Verlauf von 25 min. Die klare farblose Lösung wird 2 h gerührt. Die Lösung wird mit 100 ml Wasser, 200 ml gesättigter Natriumhydrogencarbonatlösung, 100 ml Wasser und 100 ml Natriumchloridlösung gewaschen. Die organische Phase gibt nach dem Trocknen und Entfernen des Lösungsmittels 3-(p-Chlormethylphenyl)-2-methacrylsäure-äthylester, der ohne weitere Reinigung verwendet werden kann.

Beispiel 28

3-(5-Formylpentyl)-1-methyl-2-(3-pyridyl)-indol (127 mg) wird in Dimethylformamid (0,66 ml) gelöst und mit Pyridiniumdichromat (298 mg) auf einmal versetzt. Das Gemisch wird über Nacht bei Raumtemperatur gerhrt, dann mit Aether verdünnt und filtriert. Der Feststoff wird mit Methylenchlorid gewaschen, und die vereingten Filtrate werden im Vakuum eingeengt; man erhält ein Produkt, das mit 0,1 n Natronlauge (2 ml) extrahiert wird. Der wässerige Extrakt wird auf pH 5,5 bis 6,0 angesäuert und mit Chloroform extrahiert. Der Chloroformextrakt wird getrocknet und im Vakuum eingeengt, wodurch man nach Reinigung durch Chromatographie das 3-(5-Carboxypentyl)-1-methyl-2-(3-pyridyl)-indol des Beispiels 3 erhält.

Beispiel 29

Eine Lösung von 3-(5-Carboxypentyl)-5-chlor-1-methyl-2-(3-pyridyl)-indol-Hydrochlorid (400 ml) in 7 ml Tetrahydrofuran wird erwärmt und mit 200 mg (0,27 ml) Triäthylamin behandelt. Diese Lösung wird tropfenweise zu einer Lösung von 108 mg (0,096 ml) Chlorameisensäure-äthylester in 1 ml Tetrahydrofuran zugegeben, die auf 0 bis 5° gekühlt ist. Das Reaktionsgemisch wird 1 h bei dieser Temperatur gerührt und filtriert, um Triäthylamin-Hydrochlorid zu entfernen. Das Filtrat wird mit einer Lösung von Hydroxylamin-Hydrochlorid (69 mg) und Natriumhydroxid (40 mg) in 10 ml Methanol behandelt. Das Gemisch wird 0,5 h gerührt und im Vakuum eingeengt. Der Rückstand wird mit 25 ml Aether/Methanol (10:1) behandelt und filtriert. Das Filtrat wird im Vakuum eingedampft; der Rückstand wird in Aceton gelöst und mit 6,5 n äthanolischem Chlorwasserstoff behandelt; man erhält 3-(5-Hydroxycarbamoylpentyl)-5-chlor-1-methyl-2-(3-pyridyl)-indol-Hydrochlorid.

---

Beispiel 30

3-[7,7-(Bis-methoxycarbonyl)-heptyl]-1-methyl-2-(3-pyridyl)-indol (273 mg) wird in Methanol (0,5 ml) gelöst und mit 1 n wässeriger Lithiumhydroxidlösung (1,95 ml) versetzt. Das Gemisch wird bei Raumtemperatur 1 h gerührt, dann 2,5 h unter Rückfluss erhitzt. Die Lösung wird zur Trockene eingeengt; der Rückstand wird in Wasser gelöst und der pH-Wert auf 6 bis 6,2 eingestellt. Das Gemisch wird mit Methylenchlorid extrahiert. Durch Einengen des Extrakts nach Trocknen über wasserfreiem Magnesiumsulfat erhält man rohes 3-[7,7-(Bis-carboxy)-heptyl]-1-methyl-2-(3-pyridyl)-indol.

Eine Probe der rohen Dicarbonsäure (30 mg) wird mit p-Xylol (3 ml), das 0,1 n Salzsäure (0,1 ml) enthält, 0,5 h erhitzt. Man lässt das Reaktionsgemisch auf Raumtemperatur abkühlen und extrahiert es mit

Natronlauge. Die wässerige Phase wird abgetrennt und nach Einstellen des pH-Wertes auf 6 bis 6,2 mit Aethylacetat extrahiert. Die organische Phase wird über wasserfreiem Mangnesiumsulfat getrocknet und eingeengt; man erhält 3-(7-Carboxyheptyl)-1-methyl-2-(3-pyridyl)-indol.

Die Ausgangsverbindung wird wie folgt hergestellt:

Thionylchlorid (0,36 ml) wird mit 3-(6-Hydroxyhexyl)-1-methyl-2-(3-pyridyl)-indol (0,37 g) bei 0° vereinigt Das Gemisch wird dann bei Raumtemperatur 1 h gerührt. Gesättigte wässerige Natriumhydrogencarbonatlösung wird zugegeben, und das Gemisch wird mit Dichlormethan extrahiert. Der Extrakt wird mit Natriumchloridlösung gewaschen, über wasserfreiem Magnesiumsulfat getrocknet und im Vakuum eingeengt; man erhält das rohe Chlorid 3-(6-Chlorhexyl)-1-methyl-2-(3-pyridyl)-indol.

3-(6-Chlorhexyl)-1-methyl-2-(3-pyridyl)-indol (0,5 g) wird mit Malonsäure-dimethylester (792 mg), Kaliumcarbonat (790 mg) und Dimethylformamid (11,6 ml) versetzt; das Gemisch wird bei 80 bis 90° 18 h unter Stickstoff erhitzt. Das Gemisch wird in Eiswasser (80 ml) gegossen, mit 1 n Salzsäure angesäure und mit Aether gewaschen. Die wässrige Phase wird auf pH 6 eingestellt und mit Aether extrahiert, der dann über wasserfreiem Magnesiumsulfat getrocknet und eingeengt wird; man erhält 3-[7,7-(Bis-methoxycarbonyl)-heptyl]-1-methyl-2-(3-pyridyl)-indol.

Beispiel 31

3-(6-Chlorhexyl)-1-methyl-2-(3-pyridyl)-indol (165 mg) in trockenem Tetrahydrofuran (2 ml) wird tropfenweise zu Magnesiumspänen (12 mg) in trockenem Tetrahydrofuran (2 ml) in einer Stickstoffatmosphäre gegeben. Ein Jodkristall wird während der Zugabe zugesetzt, um die Reaktion zu starten. Das Gemisch wird nach beendeter Zugabe 4 h unter Rückfluss erhitzt, dann auf 0° abgekühlt; trockenes Kohlendioxid wird in den Kolben unter Rühren 15 min lang eingeleitet. Das Gemisch wird in 5 ml 1 n Natronlauge gegossen und mit Aether extrahiert. Die wässerige Phase wird auf pH 6 bis 6,2 eingestellt und mit Aethylacetat extrahiert. Die organische Phase wird über wasserfreiem Magnesiumsulfat getrocknet und im Vakuum eingeengt; man erhält 3-(6-Carboxyhexyl)-1-methyl-2-(3-pyridyl)-indol.

Beispiel 32

Zu 30 ml einer 2 m Aethylmagnesiumbromidlösung in Tetrahydrofuran wird unter Stickstoff bei 0 bis 5° tropfenweise im Verlauf von 30 min eine Lösung von 10,0 g 2-(3-Pyridyl)-indol in 60 ml Tetrahydrofuran zugesetzt. Das Gemisch wird 0,5 h bei 0 bis 5° gerührt, dann tropfenweise mit 10,6 g Bromtetrolsäuremethylester (J. Chem. Soc. *1950,* 3646) in 50 ml Tetrahydrofuran versetzt. Das Gemisch wird weitere 2 h gerührt, bei 0° in Eiswasser gegossen und mit Aether extrahiert. Die wässerige Phase wird mit Aether extrahiert. Der Aetherextrakt wird mit Wasser und Natriumchloridlösung gewaschen und über wasserfreiem Magnesiumsulfat getrocknet. Durch Einengen im Vakuum erhält man 3-(3-Methoxycarbonyl-prop-2-inyl)-2-(3-pyridyl)-indol.

Beispiel 33

Durch Behandeln von 330 mg 3-(3-Methoxycarbonylprop-2-inyl)-2-(3-pyridyl)-indol in 10 ml Methanol mit 3,0 ml 1 n wässeriger Lithiumhydroxidlösung bei Raumtemperatur erhält man 3-(3-Carboxyprop-2-inyl)-2-(3-pyridyl)-indol.

Beispiel 34

a) Zu einer Lösung von 4-(3-Indolyl)-buttersäure-äthylester (3,00 g) und Imidazol (4,43 g) in Dioxan (75 ml), die bei 10° gerührt wird, wird tropfenweise unter Rühren eine Lösung von Brom (0,65 ml) in Dioxan (25 ml) im Verlauf von 2,5 h zugegeben. Sofort bei der Zugabe des Broms wird das Reaktionsgemisch eine dicke Suspension. Es wird weiteres Dioxan (30 ml) zugegeben, um das Reaktionsgemisch rührfähig zu halten. Nach beendeter Zugabe wird das Kühlbad entfernt, und das Reaktionsgemisch bei Raumtemperatur über Nacht gerührt. Der suspendierte Feststoff wird durch Vakuumfiltration entfernt und das Filtrat im Vakuum zu einem orangefarbenen Oel eingeengt. Dieses Oel wird in Aether (100 ml) suspendiert und mit 1 n Salzsäure (1 × 50 ml) extrahiert. Die wässerige Phase wird mit Aether (1 × 100 ml) gewaschen, mit 3 n Natronlauge (12 ml) alkalisch gemacht und mit Aether extrahiert (2 × 100 ml). Dieser Aetherextrakt wird mit Natriumchloridlösung gewaschen, über Magnesiumsulfat getrocknet, filtriert und im Vakuum zu einem orangefarbenen Oel eingeengt, das teilweise kristallisiert. Dieses Material (1,38 g) wird in 20 ml Aether gelöst; die Lösung wird mit 0,65 ml 7,1 m ethanolischem Chlorwasserstoff versetzt. Das Salz trennt sich als Oel ab, das bei Zugabe weniger ml Aethanol kristallisiert; man erhält 2-(1-Imidazolyl)-3-(3-äthoxycarbonyl-propyl)-indol-Hydrochlorid, Fp. 152 bis 155°.

b) Entsprechend wird 2-(1-Imidazolyl)-3-(5-äthoxycarbonylpentyl)-indol hergestellt; NMR ($CDCl_3$): 4,07 (q, 2H), 1,23 (t, 3H), 7,0 bis 7,8 (m, 7H).

Beispiel 35

a) Eine Suspension von 0,20 g 2-(1-Imidazolyl)-3-(3-äthoxycarbonylpropyl)-indol-Hydrochlorid in 5 ml 3 n Natronlauge wird bei Raumtemperatur 2 h gerührt. Absolutes Aethanol (5 ml) wird dann zugegeben; die entstandene Lösung wird weitere 2,5 h bei Raumtemperatur gerührt. Die Lösung wird im Vakuum

eingeengt, um das Aethanol zu entfernen. Die entstandene Lösung wird mit 1 n Salzsäure auf pH 3,5 angesäuert. Der Niederschlag wird gesammelt und getrocknet (50°/33 mbar); man erhält 2-(1-Imidazolyl)-3-(3-carboxypropyl)-indol, Fp. 205 bis 207°.

b) Entsprechend wird 2-(1-Imidazolyl)-3-(5-carboxypentyl)-indol, Fp. 146 bis 148°, hergestellt.

Beispiel 36

Zu einer Suspension von (4-Carboxybutyl)-triphenylphosponiumbromid (4,17 g) in Toluol (50 ml) wird unter Rühren und Stickstoffschutz bei Raumtemperatur Kalium-tert.-butoxid (2,11 g) gegeben. Die Suspension wird auf 90° erhitzt und 40 min bei dieser Temperatur gehalten. Die entstandene Suspension wird auf Raumtemperatur abgekühlt und mit einer Lösung von 1-Methyl-2-(3-pyridyl)-indol-3-carboxaldehyd (2,00 g) in einem Gemisch aus Toluol (20 ml) und Dimethylsulfoxid (4 ml) tropfenweise versetzt. Nach vollständiger Zugabe wird die Suspension über Nacht bei Raumtemperatur gerührt.

Das Reaktionsgemisch wird 3 h bei 60° erhitzt, auf Raumtemperatur abgekühlt und mit Wasser verdünnt (100 ml). Die organische Phase wird abgetrennt und verworfen. Die wässerige Phase wird zuerst mit Toluol (1 × 100 ml) und Aether (1 × 100 ml) extrahiert, auf pH 1 mit 1 n Salzsäure angesäuert und mit Aether extrahiert (2 × 100 ml). Der letztere Aetherextrakt wird getrocknet (Magnesiumsulfat), filtriert und im Vakuum zu einem Oel eingeengt. Die Reinigung durch Blitzchromatograhie über Kieselgel mit Methylenchlorid/Methanol (97:3) gibt im wesentlichen reines 3-(5-Carboxypent-1-enyl)-1-methyl-2-(3-pyridyl)-indol, das in das Hydrochlorid (Salz) umgewandelt wird. Dieses wird aus Methanol/Aether umkristallisiert; man erhält 3-(5-Carboxypent-1-enyl)-1-methyl-2-(3-pyridyl)-indol-Hydrochlorid, Fp. 163 bis 165°C.

Die Ausgangsverbindung wird wie folgt hergestellt:

Eine Lösung von 19,7 ml Phenylhydrazin (21,63 g) und 22 ml 3-Acetylpyridin (24,23 g) in Eisessig (100 ml) wird gerührt und 4,5 h unter Rückfluss erhitzt. Die Lösung wird in einem Eiswasserbad gekühlt und mit gesättigter Natriumhydrogencarbonatlösung (180 ml) neutralisiert. Der Niederschlag wird gesammelt, gewaschen und getrocknet; man erhält 3-Acetylpyridinphenylhydrazon.

Ein Gemisch aus diesem Hydrazon (8,0 g) und Polyphosphorsäure (74,0 g) wird gerührt und auf einem Wasserbad 1 h erhitzt. Das Gemisch wird dann bei 135 bis 140° 10 min erhitzt. Das Reaktionsgemisch wird in 840 ml Wasser gegossen; der Niederschlag wird gesammelt und mit Wasser gewaschen. Das Produkt wird wieder in 100 ml Eiswasser suspendiert und der pH-Wert mit 50-proz. wässeriger Natronlauge auf 8 eingestellt. Der Niederschlag wird gesammelt, mit kaltem Wasser gewaschen und getrocknet; man erhält 2-(3-Pyridyl)-indol, Fp. 176 bis 177° [Bull. Soc. Chem. France *1969,* 4154].

Phosphoroxychlorid (3,36 ml) wird tropfenweise im Verlauf von 20 min zu Dimethylformamid (10,6 g), welches bei 0 bis 3° gehalten wird, gegeben. Nach der vollständigen Zugabe wird eine Lösung von 2-(3-Pyridyl)-indol (6,50 g) in Dimethylformamid (20 ml) tropfenweise im Verlauf auf 30 min so zugegeben, dass die Reaktionstemperatur 10° nicht übersteigt. Das Kühlbad wird entfernt, die viskose Lösung auf 35 bis 40° erhitzt und bei dieser Temperatur 1 h gehalten. Das Reaktionsgemisch wird mit gestossenem Eis (50 g) versetzt. Eine Lösung von Natriumhydroxid (24,4 g) in 65 ml Wasser wird langsam zugegeben. Nach der Zugabe wird die entstandene rotorangefarbene Lösung schnell unter Rückfluss erhitzt und bei Rückflusstemperatur etwa 2 min gehalten. Man lässt die Lösung auf Raumtemperatur abkühlen und stellt sie für zwei Tage in einein Kühlschrank. Der entstandene Niederschlag wird gesammelt und gut mit Wasser gewaschen; man erhält einen orangefarbenen Feststoff, der aus Methanol (240 ml) umkristallisiert wird; man erhält 2-(3-Pyridyl)-indol-3-carboxaldehyd, Fp. 236 bis 238°.

Zu einer Lösung von 2-(3-Pyridyl)-indol-3-carboxaldehyd (2,22 g) in Dimethylformamid (20 ml) wird unter Stickstoffschutz bei Raumtemperatur Kaliumcarbonat (3,97 g) zugegeben. Die Suspension wird bei Raumtemperatur 5 min gerührt. Jodmethan (1,56 g) wird auf einmal zugegeben und die entstandene Suspension bei Raumtemperatur über Nacht gerührt. Die Suspension wird dann mit Wasser (100 ml) verdünnt; der Feststoff wird gesammelt, mit Wasser gewaschen und getrocknet; man erhält 1-Methyl-2-(3-pyridyl)-indol-3-carboxaldehyd, Fp. 148 bis 150°.

Beisiel 37

Eine Lösung von 3-(5-Carboxypent-1-enyl)-1-methyl-2-(3-pyridyl)-indol (0,51 g) in absolutem Aethanol (20 ml), der 10% Palladium-auf-Kohle (0,05 g) zugesetzt werden, wird bei einem Druck von 3 bar 2 h hydriert. Der Katalysator wird abfiltriert; das Filtrat wird zur Trockene eingedampft. Durch Behandeln des Rückstands mit absolutem Aethanol erhält man einen weissen Feststoff, Fp. 117 bis 120°. Durch Umkristallisieren aus Acetonitril erhält man 3-(5-Carboxypentyl)-1-methyl-2-(3-pyridyl)-indol (die Verbindung des Beispiels 3), Fp 128 bis 130°.

Beispiel 38

Gemäss den Methoden der vorhergehenden Beispiele werden weitere Verbindungen der Formeln II und III hergestellt, in denen $R_1' = CH_3$ und $R_4 = OH$ bedeutet:

| Verbindung | $R_2'$ | $R_3'$ | $(CH_2)_m$ |
|---|---|---|---|
| 1 | 5-Cl | H | $(CH_2)_7$ |
| 2 | H | H | $(CH_2)_4$ |
| 3 | 5-Cl | 6-Cl | $(CH_2)_5$ |
| 4 | 5-F | H | $(CH_2)_5$ |
| 5 | 5-$CH_3$ | H | $(CH_2)_5$ |
| 6 | 5-$CH_3$ | H | $(CH_2)_7$ |
| 7 | H | H | $(CH_2)_{10}$ |
| 8 | 5-O- $CH_2$ | -O-6 | $(CH_2)_5$ |
| 9 | 5-OH | H | $(CH_2)_5$ |
| 10 | 5-$SCH_3$ | H | $(CH_2)_5$ |
| 11 | H | H | $(CH_2)_{11}$ |
| 12 | H | H | $(CH_2)_9$ |

Beispiel 39

Gemäss den Methoden der vorhergehenden Beispiele werden weitere Verbindungen der Formel I hergestellt, in denen $R_1$ = $CH_3$, Ar = 3-Pyridyl oder 1-Imidazolyl und B = COOH bedeuten:

| Beispiel | $R_2$ | $R_3$ | A |
|---|---|---|---|
| 1 | H | H | $CH_2-C \equiv C-(CH_2)_2$ |
| 2 | H | H | $CH_2-S-(CH_2)_2$ |
| 3 | H | H | $(CH_2)_2-O-(CH_2)_2$ |
| 4 | H | H | $CH_2-O-(CH_2)_3$ |

Beispiel 40

Es werden 10.000 Tabletten hergestellt, die je 10 mg des Wirkstoffs enthalten:

Zusammensetzung:

| | |
|---|---|
| 3-(5-Carboxypentyl)-1-methyl-2-(3-pyridyl)-indol | 100,00 g |
| Milchzucker | 1.157,00 g |
| Maisstärke | 75,00 g |
| Polyäthylenglykol 6.000 | 75,00 g |
| Talkumpulver | 75,00 g |
| Magnesiumstearat | 18,00 g |
| Gereinigtes Wasser | quantum satis |

Verfahren:

Alle pulverigen Bestandteile werden durch ein Sieb mit einer Maschenweite von 0,6 mm gesiebt. Dann wird der Wirkstoff, Milchzucker, Talkum, Magnesiumstearat und die Hälfte der Stärke in einem geeigneten Mischer vermischt. Die andere Hälfte der Stärke wird in 40 ml Wasser suspendiert und die Suspension zur siedenden Lösung des Polyäthylenglykols in 150 ml Wasser gegeben. Die gebildete Paste wird den Pulvern zugesetzt und gegebenenfalls unter Zugabe von mehr Wasser granuliert. Das Granulat wird über Nacht bei 35° getrocknet, durch ein Sieb mit 1,2 mm Maschenweite getrieben und zu Tabletten von 6,4 mm Durchmesser, welche eine Bruchrille aufweisen, gepresst.

Beispiel 41

Es werden 10.000 Kapseln hergestellt, die je 25 mg Wirkstoff enthalten:

Zusammensetzung:

| | |
|---|---|
| 3-(5-Carboxypentyl)-1-methyl-2-(3-pyridyl)-indol | 250,0 g |
| Milchzucker | 1.650,0 g |
| Talkumpulver | 100,0 g |

Verfahren:

Alle pulverigen Bestandteile werden durch ein Sieb mit einer Maschenweite von 0,6 mm gesiebt. Dann wird der Wirkstoff in einem geeigneten Mischer zuerst mit Talkum und dann mit Milchzucker bis zur Homogenität vermischt. Kapseln Nr. 3 werden mit je 200 mg der erhaltenen Mischung unter Verwendung einer Kapselfüllmaschine gefüllt.

Entsprechend werden Tabletten und Kapseln hergestellt, die etwa 10 bis 100 mg anderer erfindungsgemässer Verbindungen enthalten, z.B. zon jeder anderen in den Beispielen beschriebenen Verbindung.

Beispiel 42

a) Zu einer Suspension von Natriumhydrid (50%, 0,106 g) in Dimethylformamid (3 ml), die unter Stickstoff bei Raumtemperatur gehalten wird, wird unter Rühren tropfenweise eine Lösung von p-Mercaptobenzoesäureäthylester (0,40 g) in Dimethylformamid (1 ml) gegeben. Das Gemisch wird eine halbe Stunde gerührt und dann tropfenweise eine Lösung von 3-(N,N-Dimethylaminomethyl)-2-(3-pyridyl)-indol (0,5 g) in Dimethylformamid (6 ml) gegeben, die bei −10° unter Stickstoff gehalten und gerührt wird. Das Reaktionsgemisch wird 18 h bei Raumtemperatur gerührt. Der Ansatz wird in Eiswasser gegossen und mit Aether extrahiert. Man wäscht den Aetherextrakt wiederholt mit Wasser, trocknet ihn über Magnesiumsulfat, filtriert und engt ihn ein, wobei man 3-[(p-Aethoxycarbonylphenylthio)-methyl]-2-(3-pyridyl)-indol erhält.

Die Ausgangsverbindung wird wird folgt hergestellt:

Zu einer Mischung aus 40% Dimethylamino-Lösung (4,5 ml), 37% wässeriger Formaldehyd-Lösung (2,6 ml) und Eisessig (6,4 ml), die bei 10° gerührt wird, wird portionsweise 2-(3-Pyridyl)-indol (5,0 g) hinzugegeben. Nach beendeter Zugabe wird das Reaktionsgemisch 3 h bei Raumtemperatur gerührt. Das Gemisch wird mit konzentrierter wässeriger Ammoniaklösung basisch gemacht, dann das produkt gesammelt, wobei man 3-(N,N-Dimethylaminomethyl)-2-(3-pyridyl)-indol erhält.

b) Durch Hydrolyse von 3-[(p-Aethoxycarbonylphenylthio)-methyl]-2-(3-pyridyl)-indol mit 2 n Salzsäure erhält man 3-[(p-Carboxyphenylthio)-methyl]-2-(3-pyridyl)-indol.

Beispiel 43

Eine Mischung aus 0,01 Mol 7-[o-(N-Nikotinoyl-N-methylamino)-phenyl]-heptansäure und 2 Moläquivalenten Kalium-tert.-butoxid in 50 ml Tetrahydronaphthalin wird über Nacht unter Stickstoff unter Rückfluss gekocht. Die Reaktion wird durch Zugabe von Wasser abgebrochen, und man dampft den Ansatz unter vermindertem Druck zur Trockene ein. Wasser und Methylenchlorid werden hinzugegeben, und der pH-Wert wird mit 2 n Salzsäure auf 5 eingestellt.

Die Methylenchloridphase wird abgetrennt, mit Wasser gewaschen und zur Trockene eingedampft. Nach Umkristallisation des Rückstands aus Acetonitril erhält man 3-(5-Carboxypentyl)-1-methyl-2-(3-pyridyl)-indol des Beispiels 3, Fp. 128—130°C.

Die Ausgangsverbindung wird die folgt hergestellt:

Zunächst wird 1-Aza-2,3-benzocyclodek-2-en-10-on durch eine Beckmann-Umlagerung aus 1,2-Benzocyclonon-1-en-3-on-Oxim hergestellt [Chem. Ber. *90*, 1946 (1957)].

Zu einer Suspension von 50% Natriumhydrid (0,50 g) in wasserfreiem Dimethylformamid (20 ml), die unter Stickstoff bei Raumtemperatur gerührt wird, wird tropfenweise eine Lösung von 1-Aza-2,3-benzocyclodek-2-en-10-on (2,03 g) in Dimethylformamid (10 ml) gegeben. Das Gemisch wird eine halbe Stunde bei Raumtemperatur gerührt, dann Methyliodid (1,56 g) tropfenweise hinzugegeben. Nach beendeter Zugabe wird das Reaktionsgemisch 5 h bei Raumtemperatur gerührt. Der Ansatz wird in Eiswasser gegossen und wiederholt mit Aether extrahiert. Der Aetherextrakt wird mit Wasser und mit gesättigter Kochsalzlösung gewaschen, über Magnesiumsulfat getrocknet, filtriert und bei vermindertem Druck eingeengt, wobei man N-Methyl-1-aza-2,3-benzocyclodek-2-en-10-on erhält.

Ein Gemisch aus N-Methyl-1-aza-2,3-benzocyclodek-2-en-10-on (1,09 g) und 3 n Natronlauge (20 ml) wird unter Rühren 18 h bei Rückfluss gekocht. Die Lösung wird abgekühlt und mit 3 n Salzsäure bis auf pH 4 angesäuert. Das Produkt wird mit Methylenchlorid extrahiert. Dieser Extrakt wird mit Wasser gewaschen, über Magnesiumsulfat getrocknet, filtriert und eingeengt, wobei man 7-[o-(N-Methylamino)-phenyl]-heptansäure erhält.

Zu einer Mischung aus 7-[o-(N-Methylamino)-phenyl]-heptansäure (1,18 g) und Triäthylamin (1,21 g) in

Toluol (50 ml), die bei Raumtemperatur gerührt wird, wird Nikotinoylchlorid-Hydrochlorid (0,89 g) portionsweise zugegeben. Nach beendeter Zugabe wird das Gemisch 6 h unter Rückfluss gekocht. Man kühlt die Suspension in Eiswasser ab und sammelt den Niederschlag. Dieser wird in Chloroform suspendiert, ernaut gesammelt, wobei man 7-[o-(N-Nikotonoyl-N-methylamino)-phenyl]-heptansäure erhält.

**Patentansprüche für die Vertragsstaaten: BE CH LI DE FR GB IT LU NL SE**

1. 2-(Pyridyl und Imidazolyl)-Indole der Formel I

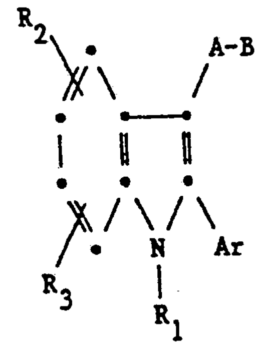

(I),

worin

$R_1$ Wasserstoff oder Niederalkyl bedeutet, Ar jeweils für unsubstituiertes oder durch Niederalkyl, Carboxy, Niederalkoxycarbonyl oder Carbamoyl substituiertes 3-Pyridyl oder 1-Imidazolyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeuten, oder $R_2$ und $R_3$ zusammen, an benachbarten Kohlenstoffatomen, Niederalkylendioxy sind;

A Alkylen mit 3 bis 12 Kohlenstoffatomen, wobei die Anzahl der zwischen dem Indolkern und der Gruppe B stehenden Kohlenstoffatome 3 bis 12 beträgt, Alkenylen oder Alkinylen mit jeweils 2 bis 12 Kohlenstoffatomen, Niederalkylenphenylen-nieder-(alkylen oder alkenylen), Niederalkylen-phenylen, Niederalkylen-(thio oder oxy)-niederalkylen, Niederalkylen-(thio oder oxy)-phenylen oder Niederalkylen-phenylen-(thio oder oxy)-niederalkylen bedeutet und

B für Carboxy, verestertes Carboxy in Form von pharmazeutisch verwendbaren Estern, Carbamoyl, Mono- oder Diniederalkylcarbamoyl, Hydroxymethyl, Cyan, Hydroxycarbamoyl, 5-Tetrazolyl oder Formyl steht: ihre Imidazolyl- oder Pyridyl-N-Oxide, wobei die mit "nieder" bezeichneten Gruppen höchstens 7 Kohlenstoffatome enthalten, und ihre Salze.

2. Verbindungen der Formel I gemäss Anspruch 1, worin $R_1$ Wasserstoff oder Niederalkyl bedeutet, Ar jeweils unsubstituiertes oder durch Niederalkyl substituiertes 3-Pyridyl oder 1-Imidazolyl bedeutet, $R_2$ und $R_3$ unabhängig voneinander Wasserstoff, Niederalkyl, Halogen, Trifluormethyl, Hydroxy, Niederalkoxy oder Niederalkylthio bedeuten, oder $R_2$ und $R_3$ zusammen, an benachbarten Kohlenstoffatomen, Niederalkylendioxy sind;

A für Alkylen mit 4 bis 12 Kohlenstoffatomen, Nieder-(alkylenphenylen, alkylen-thio-phenylen oder alkylen-oxy-phenylen) mit 7 bis 10 Kohlenstoffatomen steht;

B Carboxy, Niederalkoxycarbonyl, Carbamoyl, Cyan, Hydroxycarbamoyl, 5-Tetrazolyl oder Hydroxymethyl bedeutet; wobei die mit "nieder" bezeichneten Gruppen höchstens 7 Kohlenstoffatome enthalten, und ihre Salze.

3. Verbindung der Formel II gemäss Anspruch 1

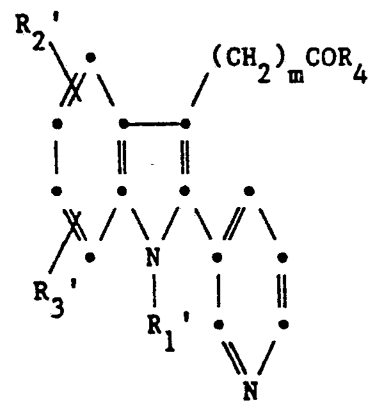

(II),

worin $R_1'$ Wasserstoff oder Niederalkyl bedeutet, $R_2'$ und $R_3'$ unabhängig voneinander für Wasserstoff,

Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 4 bis 12 steht und $R_4$ Hydroxy, Niederalkoxy oder Amino bedeutet; wobei die mit "nieder" bezeichneten Gruppen höchstens 7 Kohlenstoffatome enthalten, und ihre pharmazeutisch verwendbaren Salze.

4. Verbindungen der Formel II gemäss Anspruch 3, worin $R_1'$ Wasserstoff oder Niederalkyl bedeutet, $R_2'$ für Wasserstoff oder Halogen steht, $R_3'$ Wasserstoff ist, m für eine ganze Zahl von 4 bis 8 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze.

5. Verbindungen der Formel III gemäss Anspruch 1

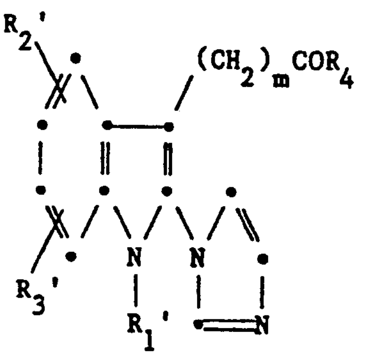

(III),

worin $R_1'$ Wasserstoff oder Niederalkyl bedeutet, $R_2'$ und $R_3'$ unabhängig voneinander für Wasserstoff, Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 3 bis 12 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze.

6. Verbindungen der Formel III gemäss Anspruch 5, worin $R_1'$ Wasserstoff oder Niederalkyl bedeutet, $R_2'$ für Wasserstoff oder Halogen steht, $R_3'$ Wasserstoff ist, m für eine ganze Zahl von 4 bis 8 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze.

7. 3-(5-Carboxypentyl)-2-(3-pyridyl)-indol und pharmazeutisch verwendbare Salze davon gemäss Anspruch 1.

8. 3-(5-Carboxypentyl)-1-methyl-2-(3-pyridyl)-indol und pharmazeutisch verwendbare Salze davon gemäss Anspruch 1.

9. 5-Hydroxy-2-(3-pyridyl)-3-(5-carboxypentyl)-indol und pharmazeutisch verwendbare Salze davon gemäss Anspruch 1.

10. 3-(5-Carboxypentyl)-5-chlor-2-(3-pyridyl)-indol und pharmazeutisch verwendbare Salze davon gemäss Anspruch 1.

11. 3-(5-Carboxypentyl)-5-chlor-1-methyl-2-(3-pyridyl)-indol und pharmazeutisch verwendbare Salze davon gemäss Anspruch 1.

12. 2-(1-Imidazolyl)-3-(3-carboxypropyl)-indol und pharmazeutisch verwendbare Salze davon gemäss Anspruch 1.

13. 2-(1-Imidazolyl)-3-(5-carboxypentyl)-indol und pharmazeutisch verwendbare Salze davon gemäss Anspruch 1.

14. Eine Verbindung gemäss Anspruch 1 und ihre pharmazeutisch verwendbaren Salze zur Anwendung in einem Verfahren zur therapeutischen Behandlung des menschlichen und tierischen Körpers.

15. Pharmazeutische Präparate enthaltend Verbindungen der Formel I gemäss Anspruch 1 oder pharmazeutisch verwendbare Salze davon.

16. Verwendung von Verbindungen der Formel I gemäss Anspruch 1 und von pharmazeutisch verwendbaren Salzen davon zur Herstellung von pharmazeutischen Präparaten.

17. Verbindungen der Formel I gemäss Anspruch 1 als Thromboxan-Synthetase-Hemmer.

18. Verfahren zur Herstellung von Verbindungen der Formel I gemäss Anspruch 1, dadurch gekennzeichnet, dass man 1) eine Verbindung der Formel IV

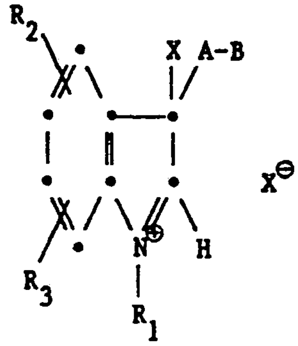

(IV),

worin $R_1$, $R_2$, $R_3$, A und B die unter Formel I angegebene Bedeutung haben und X für Halogen steht, mit einer Verbindung der Formel Ar—H oder einem reaktiven metallierten Derivat davon, worin Ar die unter Formel I angegebene Bedeutung hat, umsetzt, und, falls gewünscht, eine erhaltene Verbindung der Formel I, worin $R_1$ Wasserstoff ist, mit einem Alkylierungsmittel in eine Verbindung der Formel I überführt, worin $R_1$ Niederalkyl bedeutet, oder

2) eine Verbindung der Formel V

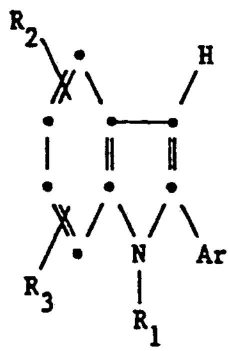

(V),

worin $R_1$, $R_2$, $R_3$ und Ar die unter Formel I angegebene Bedeutung haben, als reaktives metallorganisches Derivat mit einem reaktiven funktionellen Derivat einer Verbindung der Formel VI

HO—A—B (VI),

worin A und B die unter Formel I angegebene Bedeutung haben, umsetzt, und, falls gewünscht, eine erhaltene Verbindung der Formel I, worin $R_1$ Wasserstoff ist, mit einem Alkylierungsmittel in eine Verbindung der Formel I umsetzt, worin $R_1$ Niederalkyl ist,.oder

3) eine Verbindung der Formel VII

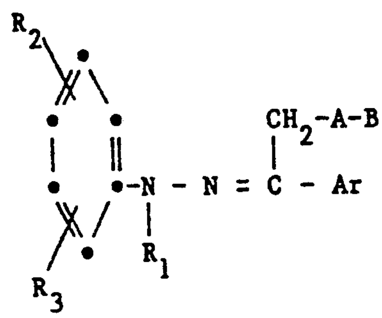

(VII),

worin $R_1$, $R_2$, $R_3$, A und B die unter Formel I angegebene Bedeutung haben und Ar für unsubstituiertes oder wie unter Formel I angegeben substituiertes 3-Pyridyl steht, ringschliesst, oder

4) eine Verbindung der Formel VIII

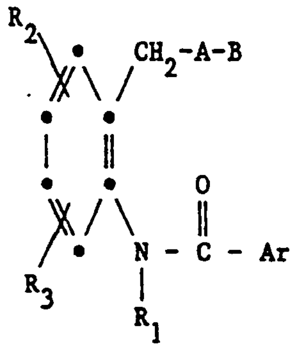

(VIII),

worin Ar, $R_1$, $R_2$, $R_3$, A und B die unter Formel I angegebene Bedeutung haben, cyclisiert, oder

5) zur Herstellung einer Verbindung der Formel I, worin A Alkenylen bedeutet, unter den Bedingungen einer Wittig-Reaktion eine Verbindung der Formel Va

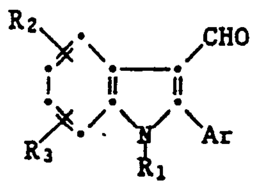

(Va),

die einem 3-Formylderivat einer Verbindung der Formel V entspricht, mit dem Ylid einer Verbindung der Formel XII

$$R_5—A'—B \quad (XII)$$

worin B die unter Formel I angegebene Bedeutung hat, A' Alkylen wie oben für A in Verbindungen der Formel I definiert, jedoch mit um 1 Kohlenstoffatom verkürzter Kettenlänge, ist und $R_5$ einen Dialkylphosphono- oder Triarylphosphonium-Rest bedeutet, umsetzt oder

6) zur Herstellung einer Verbindung der Formel I, worin A Niederalkyl-(thio oder oxy)-niederalkylen oder Niederalkylen-(thio oder oxy)-phenylen bedeutet, eine Verbindung der Formel Vb

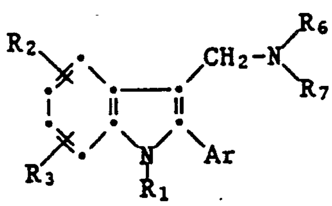

(Vb),

worin Ar, $R_1$, $R_2$ und $R_3$ die unter Formel I angegebene Bedeutung haben und $R_6$ und $R_7$ jeweils Niederalkyl bedeuten — also ein 3-(di-substituiertes Aminomethyl)-Derivat einer Verbindung der Formel V — mit einer Verbindung der Formel XIII

$$R_5'—A''—B \quad (XIII)$$

oder einem reaktiven Alkalimetall- oder Ammonium-Derivat davon, worin B wie unter Formel I angegeben definiert ist, $R_5'$ Hydroxy oder Thiol bedeutet und A'' für Niederalkylen oder Phenylen steht; oder mit einem Lacton oder Thiolacton einer Verbindung der Formel XIII, worin $R_5'$ Hydroxy oder Thiol bedeutet, A'' Niederalkylen ist und B für Carboxy steht, umsetzt, oder

7) eine Verbindung der Formel Ia

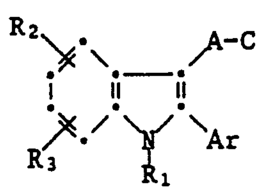

(Ia),

worin A, Ar, $R_1$, $R_2$ und $R_3$ die unter Formel I angegebene Bedeutung haben und C eine von B verschiedene und in B überführbare Gruppe, wie Trialkoxymethyl, verestertes Hydroxymethyl, veräthertes

Hydroxymethyl, Halogenmethyl, 2-Oxazolinyl, Dihydro-2-oxazolinyl, Niederalkanoyloxymethyl, Acetyl, Methyl, Carboxycarbonyl, Trihalogenacetyl, Di-niederalkoxymethyl, Alkylendioxymethyl, Vinyl, Alkinyl, verestertes Carboxy oder amidiertes Carboxy, bedeutet, gegebenenfalls unter Verlängerung der Kette A im Rahmen ihrer Definition, oder eine Verbindung der Formel I* oder Ia*, die derjenigen der Formel I beziehungsweise Ia gleicht, worin jedoch abweichend A für Alkylen mit 1 oder 2 Kohlenstoffatomen steht, unter Verlängerung der Alkylenkette A zu Alkylen mit einer solchen Zahl von Kohlenstoffatomen wie sie unter Formel I definiert ist, in eine Verbindung der Formel I umwandelt; wobei störende reaktionsfähige Gruppen im Molekül gegebenenfalls vorübergehend geschützt sind, und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Anti-poden aufspaltet.

**Patentansprüche für den Vertragsstaat: AT**

1. Verfahren zur Herstellung von 2-(Pyridyl und Imidazolyl)-Indolen der Formel I

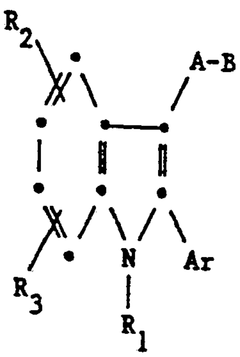

(I),

worin

$R_1$ Wasserstoff oder Niederalkyl bedeutet, Ar jeweils für unsubstituiertes oder durch Niederalkyl, Carboxy, Niederalkoxycarbonyl oder Carbamoyl substituiertes 3-Pyridyl oder 1-Imidazolyl steht, $R_2$ und $R_3$ unabhängig voneinander Wasserstoff, Niederalkyl, Halogen, Trifluormethyl, Hydroxy, Niederalkoxy, Carboxyniederalkyl, Niederalkoxycarbonyl-niederalkyl, Carboxy, Niederalkoxycarbonyl oder Niederalkyl-(thio, sulfinyl oder sulfonyl) bedeuten, oder $R_2$ und $R_3$ zusammen, an benachbarten Kohlenstoffatomen, Niederalkylendioxy sind;

A Alkylen mit 3 bis 12 Kohlenstoffatomen, wobei die Anzahl der zwischen dem Indolkern und der Gruppe B stehenden Kohlenstoffatome 3 bis 12 beträgt, Alkenylen oder Alkinylen mit jeweils 2 bis 12 Kohlenstoffatomen, Niederalkylenphenylen-nieder-(alkylen oder alkenylen), Niederalkylen-phenylen, Niederalkylen-(thio oder oxy)-niederalkylen, Niederalkylen-(thio oder oxy)-phenylen oder Niederalkylen-phenylen-(thio oder oxy)-niederalkylen bedeutet und

B für Carboxy, verestertes Carboxy in Form von pharmazeutisch verwendbaren Estern, Carbamoyl, Mono- oder Diniederalkylcarbamoyl, Hydroxymethyl, Cyan, Hydroxycarbamoyl, 5-Tetrazolyl oder Formyl steht: ihre Imidazolyl- oder Pyridyl-N-Oxiden, wobei die mit "nieder" bezeichneten Gruppen höchstens 7 Kohlenstoffatome enthalten, und ihre Salzen, dadurch gekennzeichnet, dass man

1) eine Verbindung der Formel IV

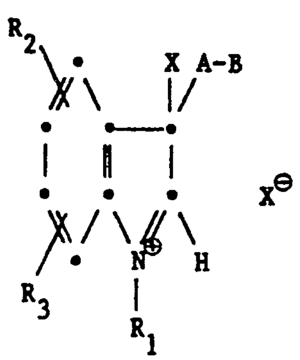

(IV),

worin $R_1$, $R_2$, $R_3$, A und B die unter Formel I angegebene Bedeutung haben und X für Halogen steht, mit einer Verbindung der Formel Ar—H oder einem reaktiven metallierten Derivat davon, worin Ar die unter

Formel I angegebene Bedeutung hat, umsetzt, und, falls gewünscht, eine erhaltene Verbindung der Formel I, worin $R_1$ Wasserstoff ist, mit einem Alkylierungsmittel in eine Verbindung der Formel I überführt, worin $R_1$ Niederalkyl bedeutet, oder

2) eine Verbindung der Formel V

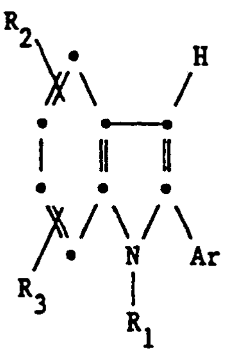

(V),

worin $R_1$, $R_2$, $R_3$ und Ar die unter Formel I angegebene Bedeutung haben, als reaktives metallorganisches Derivat mit einem reaktiven funktionellen Derivat einer Verbindung der Formel VI

HO—A—B (VI),

worin A und B die unter Formel I angegebene Bedeutung haben, umsetzt, und, falls gewünscht, eine erhaltene Verbindung der Formel I, worin $R_1$ Wasserstoff ist, mit einem Alkylierungsmittel in eine Verbindung der Formel I umsetzt, worin $R_1$ Niederalkyl ist, oder

3) eine Verbindung der Formel VII

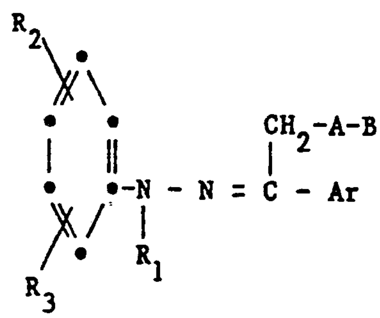

(VII),

worin $R_1$, $R_2$, $R_3$, A und B die unter Formel I angegebene Bedeutung haben und Ar für unsubstituiertes oder wie unter Formel I angegeben substituiertes 3-Pyridyl steht, ringschliesst, oder

4) eine Verbindung der Formel VIII

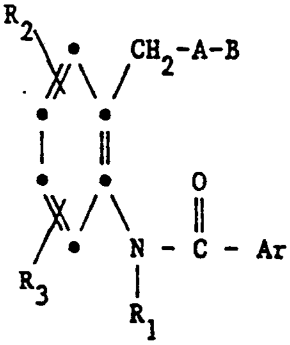

(VIII),

worin Ar, $R_1$, $R_2$, $R_3$, A und B die unter Formel I angegebene Bedeutung haben, cyclisiert, oder

5) zur Herstellung einer Verbindung der Formel I, worin A Alkenylen bedeutet, unter den Bedingungen einer Wittig-Reaktion eine Verbindung der Formel Va

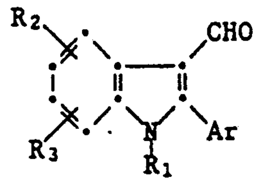

(Va),

die einem 3-Formylderivat einer Verbindung der Formel V entspricht, mit dem Ylid einer Verbindung der Formel XII

$$R_5—A'—B \qquad (XII)$$

worin B die unter Formel I angegebene Bedeutung hat, A' Alkylen wie oben für A in Verbindungen der Formel I definiert, jedoch mit um 1 Kohlenstoffatom verkürzter Kettenlänge, ist und $R_5$ einen Dialkylphosphono- oder Triarylphosphonium-Rest bedeutet, umsetzt oder

6) zur Herstellung einer Verbindung der Formel I, worin A Niederalkyl-(thio oder oxy)-niederalkylen oder Niederalkylen-(thio oder oxy)-phenylen bedeutet, eine Verbindung der Formel Vb

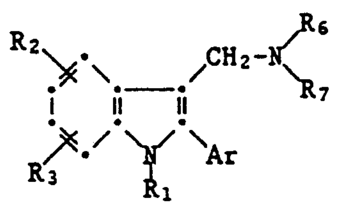

(Vb),

worin Ar, $R_1$, $R_2$ und $R_3$ die unter Formel I angegebene Bedeutung haben und $R_6$ und $R_7$ jeweils Niederalkyl bedeuten — also ein 3-(di-substituiertes Aminomethyl)-Derivat einer Verbindung der Formel V — mit einer Verbindung der Formel XIII

$$R_5'—A''—B \qquad (XIII)$$

oder einem reaktiven Alkalimetall- oder Ammonium-Derivat davon, worin B wie unter Formel I angegeben definiert ist, $R_5'$ Hydroxy oder Thiol bedeutet und A'' für Niederalkylen oder Phenylen steht; oder mit einem Lacton oder Thiolacton einer Verbindung der Formel XIII, worin $R_5'$ Hydroxy oder thiol bedeutet, A'' Niederalkylen ist und B für Carboxy steht, umsetzt, oder

7) eine Verbindung der Formel Ia

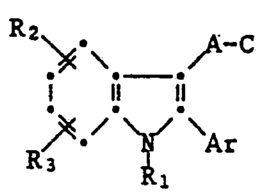

(Ia),

worin A, Ar, $R_1$, $R_2$ und $R_3$ die unter Formel I angegebene Bedeutung haben und C eine von B verschiedene und in B überführbare Gruppe, wie Trialkoxymethyl, verestertes Hydroxymethyl, veräthertes Hydroxymethyl, Halogenmethyl, 2-Oxazolinyl, Dihydro-2-oxazolinyl, Niederalkanoyloxymethyl, Acetyl, Methyl, Carboxycarbonyl, Trihalogenacetyl, Di-niederalkoxymethyl, Alkylendioxymethyl, Vinyl, Alkinyl, verestertes Carboxy oder amidiertes Carboxy, bedeutet, gegebenenfalls unter Verlängerung der Kette A im Rahmen ihrer Definition, oder eine Verbindung der Formel I* oder Ia*, die derjenigen der Formel I beziehungsweise Ia gleicht, worin jedoch abweichend A für Alkylen mit 1 oder 2 Kohlenstoffatomen steht, unter Verlängerung der Alkylenkette A zu Alkylen mit einer solchen Zahl von Kohlenstoffatomen wie sie unter Formel I definiert ist, in eine Verbindung der Formel I umwandelt; wobei störende reaktionsfähige Gruppen im Molekül gegebenenfalls vorübergehend geschützt sind, und/oder, wenn erwünscht, eine erhaltene Verbindung der Formel I in eine andere Verbindung der Erfindung umwandelt, und/oder, wenn erwünscht, eine erhaltene freie Verbindung der Formel I in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Anti-poden aufspaltet.

2. Verfahren nach Anspruch 1, dadurch gekennzeichnet, dass man Verbindungen der Formel I, worin $R_1$ Wasserstoff oder Niederalkyl bedeutet, Ar jeweils unsubstituiertes oder durch Niederalkyl substituiertes 3-Pyridyl oder 1-Imidazolyl bedeutet, $R_2$ und $R_3$ unabhängig voneinander Wasserstoff, Niederalkyl, Halogen, Trifluormethyl, Hydroxy, Niederalkoxy oder Niederalkylthio bedeuten, oder $R_2$ und $R_3$ zusammen, an benachbarten Kohlenstoffatomen, Niederalkylendioxy sind;

A für Alkylen mit 4 bis 12 Kohlenstoffatomen, Nieder-(alkylenphenylen, alkylen-thio-phenylen oder alkylen-oxy-phenylen) mit 7 bis 10 Kohlenstoffatomen steht;

B Carboxy, Niederalkoxycarbonyl, Carbamoyl, Cyan, Hydroxycarbamoyl, 5-Tetrazolyl oder Hydroxymethyl bedeutet; wobei die mit "nieder" bezeichneten Gruppen höchstens 7 Kohlenstoffatome enthalten, und ihre Salze herstellt.

3. Verfahren nach Anspruch 1, dadurch gekennzeichnet, dass man Verbindungen der Formel II

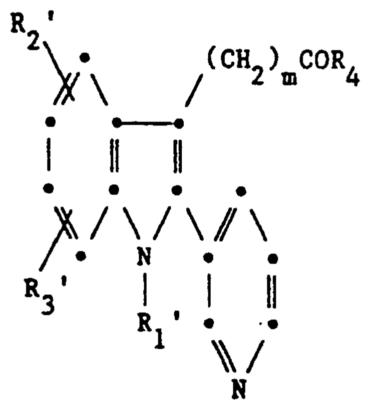

(II),

worin $R_1'$ Wasserstoff oder Niederalkyl bedeutet, $R_2'$ und $R_3'$ unabhängig voneinander für Wasserstoff, Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 4 bis 12 steht und $R_4$ Hydroxy, Niederalkoxy oder Amino bedeutet; wobei die mit "nieder" bezeichneten Gruppen höchstens 7 Kohlenstoffatome enthalten, und ihre pharmazeutisch verwendbaren herstellt.

4. Verfahren nach Anspruch 3, dadurch gekennzeichnet, dass man Verbindungen der Formel II, worin $R_1'$ Wasserstoff oder Niederalkyl bedeutet, $R_2'$ für Wasserstoff oder Halogen steht, $R_3'$ Wasserstoff ist, m für eine ganze Zahl von 4 bis 8 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze herstellt.

5. Verfahren nach Anspruch 1, dadurch gekennzeichnet, dass man Verbindungen der Formel III

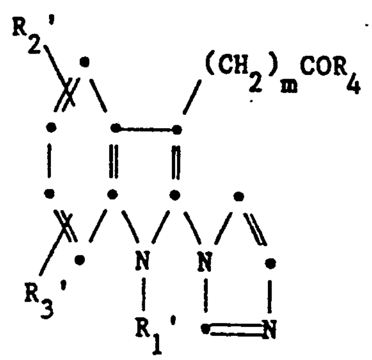

(III),

worin $R_1'$ Wasserstoff oder Niederalkyl bedeutet, $R_2'$ und $R_3'$ unabhängig voneinander für Wasserstoff, Niederalkyl, Halogen, Trifluormethyl, Hydroxy, Niederalkylthio oder Niederalkoxy stehen, oder $R_2'$ und $R_3'$ zusammen, an benachbarten Kohlenstoffatomen, Methylendioxy bedeuten, m für eine ganze Zahl von 3 bis 12 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze herstellt.

6. Verfahren nach Anspruch 5, dadurch gekennzeichnet, dass man Verbindungen der Formel III, worin $R_1'$ Wasserstoff oder Niederalkyl bedeutet, $R_2'$ für Wasserstoff oder Halogen steht, $R_3'$ Wasserstoff ist, m für eine ganze Zahl von 4 bis 8 steht und $R_4$ Hydroxy, Niederalkoxy oder Amino bedeutet, wobei die mit "nieder" bezeichneten Gruppen höchstens 7 Kohlenstoffatome enthalten, und ihre pharmazeutisch verwendbaren Salze herstellt.

7. Verfahren nach Anspruch 1, dadurch gekennzeichnet, dass man 3-(5-Carboxypentyl)-2-(3-pyridyl)-indol und pharmazeutisch verwendbare Salze davon herstellt.

8. Verfahren nach Anspruch 1, dadurch gekennzeichnet, dass man 3-(5-Carboxypentyl)-1-methyl-2-(3-pyridyl)-indol und pharmazeutisch verwendbare Salze davon herstellt.

9. Verfahren nach Anspruch 1, dadurch gekennzeichnet, dass man 5-Hydroxy-2-(3-pyridyl)-3-(5-carboxypentyl)-indol und pharmazeutisch verwendbare Salze davon herstellt.

10. Verfahren nach Anspruch 1, dadurch gekennzeichnet, dass man 3-(5-Carboxypentyl)-5-chlor-2-(3-pyridyl)-indol und pharmazeutisch verwendbare Salze davon herstellt.

11. Verfahren nach Anspruch 1, dadurch gekennzeichnet, dass man (5-Carboxypentyl)-5-chlor-1-methyl-2-(3-pyridyl)-indol und pharmazeutisch verwendbare Salze davon herstellt.

12. Verfahren nach Anspruch 1, dadurch gekennzeichnet, dass man 2-(1-Imidazolyl)-3-(3-carboxypropyl)-indol und pharmazeutisch verwendbare Salze davon herstellt.

13. Verfahren nach Anspruch 1, dadurch gekennzeichnet, dass man 2-(1-Imidazolyl)-3-(5-carboxypentyl)-indol und pharmazeutisch verwendbare Salze davon herstellt.

14. Verfahren zur Herstellung von Verbindungen der Formel I gemäss Anspruch 1, dadurch gekennzeichnet, dass man

1) eine Verbindung der Formel IV

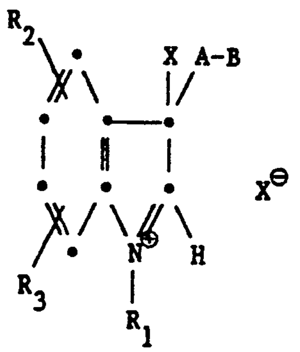

(IV),

worin $R_1$, $R_2$, $R_3$, A und B die unter Formel I angegebene Bedeutung haben und X für Halogen steht, mit einer Verbindung der Formel Ar—H oder einem reaktiven metallierten Derivat davon, worin Ar die unter Formel I angegebene Bedeutung hat, umsetzt, und, falls gewünscht, eine erhaltene Verbindung der Formel I, worin $R_1$ Wasserstoff ist, mit einem Alkylierungsmittel in eine Verbindung der Formel I überführt, worin $R_1$ Niederalkyl bedeutet, oder

2) eine Verbindung der Formel V

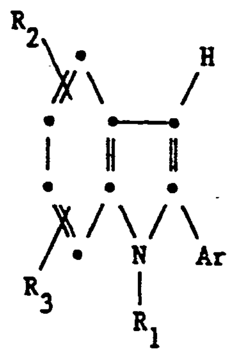

(V),

worin $R_1$, $R_2$, $R_3$ und Ar die unter Formel I angegebene Bedeutung haben, als reaktives metallorganisches Derivat mit einem reaktiven funktionellen Derivat einer Verbindung der Formel VI

$$HO—A—B \qquad (VI),$$

worin A und B die unter Formel I angegebene Bedeutung haben, umsetzt, wobei störende reaktionsfähige Gruppen im Molekül gegebenenfalls vorübergehend geschützt sind, uund, falls gewünscht, eine erhaltene Verbindung der Formel I, worin $R_1$ Wasserstoff ist, mit einem Alkylierungsmittel in eine Verbindung der Formel I umsetzt, worin $R_1$ Niederalkyl ist, oder

3) eine Verbindung der Formel VII

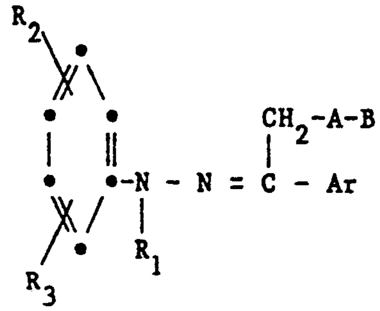

(VII),

worin $R_1$, $R_2$, $R_3$, A und B die unter Formel I angegebene Bedeutung haben und Ar für unsubstituiertes oder wie unter Formel I angegeben substituiertes 3-Pyridyl steht, ringschliesst, oder

4) eine Verbindung der Formel VIII

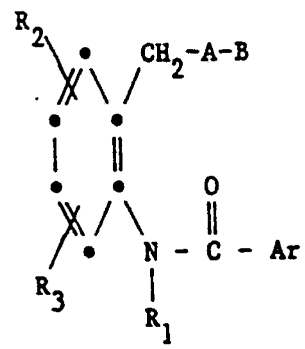

(VIII),

worin Ar, $R_1$, $R_2$, $R_3$, A und B die unter Formel I angegebene Bedeutung haben, und Ar unsubstituiertes oder wie unter Formel I definiert substituiertes 3-Pyridyl bedeutet, cyclisiert, oder

5) eine Verbindung Ia

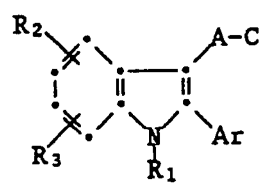

(Ia),

worin A, Ar, $R_1$, $R_2$ und $R_3$ die unter Formel I angegebene Bedeutung haben und C eine von B verschiedene und in B überführbare Gruppe, wie Trialkoxymethyl, verestertes Hydroxymethyl, veräthertes Hydroxymethyl, Halogenmethyl, 2-Oxazolinyl, Dihydro-2-oxazolinyl, Niederalkanoyloxymethyl, Acetyl, Methyl, Carboxycarbonyl, Trihalogenacetyl, Di-niederalkoxymethyl, Alkylendioxymethyl, Vinyl, Alkinyl, verestertes Carboxy oder amidiertes Carboxy, bedeutet, in eine Verbindung der Formel I umwandelt; und eine gemäss den oben beschriebenen Verfahren erhaltent Verbindung der Formel I in eine andere Verbindung der Erfindung umwandelt, und eine gemäss den oben beschreibenen Verfahren erhaltene freie Verbindung der Formel I in ein Salz oder ein erhaltenes Salz in die freie Verbindung überführt, und, wenn erwünscht, ein optisches oder geometrisches Isomeres aus einer Mischung von Isomeren der erhaltenen Verbindung der Formel I isoliert.

15. Verfahren zur Herstellung von pharmazeutischen Präparaten, dadurch gekennzeichnet, dass man eine nach dem Verfahren gemäss Anspruch 1 erhaltene Verbindung der Formel I oder ein pharmazeutisch verwendbares Salz einer solchen Verbindung mit einem pharmazeutisch verwendbaren Trägermaterial mischt.

16. Verfahren zur Herstellung von pharmazeutischen Präparaten, dadurch gekennzeichnet, dass man eine nach dem Verfahren gemäss Anspruch 14 erhaltene Verbindung der Formel I oder ein pharmazeutisch verwendbares Salz einer solchen Verbindung mit einem pharmazeutisch verwendbaren Trägermaterial mischt.

**Revendications pour les Etats contractants: BE CH LI DE FR GB IT LU NL SE**

1. 2-(pyridyl et imidazolyl)-indoles de formule I

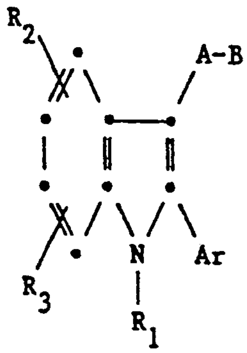

(I),

où $R_1$ représente un hydrogène ou un alcoyle inférieur, Ar représente à chaque fois un 3-pyridyle ou l-imidazolyle non substitué ou substitué par un alcoyle inférieur, carboxy, alcoxy inférieur-carbonyle ou carbamoyle, $R_2$ et $R_3$ représentent indépendamment l'un de l'autre un hydrogène, alcoyle inférieur, halogène, trifluorométhyle, hydroxy, alcoxy inférieur, carboxyalcoyle inférieur, alcoxy inférieur carbonyle-alcoyle inférieur, carboxy, alcoxy inférieur-carbonyle ou alcoyle inférieur-(thio, sulfinyle ou sulfonyle), ou $R_2$ et $R_3$ ensemble, sur les atomes de carbone voisins, représentent un alcoylène inférieur-dioxy;

A représente un alcoylène en $C_{3-12}$, où le nombre des atomes de carbone situés entre le noyau indole et le groupe B s'élève à 3 à 12, un alcénylène ou un alcynylène avec à chaque fois de 2 à 12 atomes de carbone, un alcoyle inférieur phénylène-(alcoylène ou alcénylène) inférieur, un alcoylène inférieur -phénylène, un alcoylène inférieur-(thio ou oxy)-alcoylène inférieur, un alcoylène inférieur-(thio ou oxy)-phénylène ou un alcoylène inférieur phénylène -(thio ou oxy)-alcoylène inférieur et B représente un carboxy, carboxy estérifié sous forme d'esters pharmaceutiquement acceptables, un carbamoyle, un mono- ou dialcoyle inférieur carbamoyle, un hydroxyméthyle, un cyano, un hydroxycarbamoyle, un 5-tétrazolyle ou un formyle; leurs imidazolyl- ou pyridyl-N-oxydes où les groupes décrits comme "inférieurs" contiennent au plus 7 atomes de carbone, et leurs sels.

2. Composés de formule I selon la revendication 1, où $R_1$ représente un hydrogène ou un alcoyle inférieur, Ar représente à chaque fois un 3-pyridyle ou un 1-imidazolyle non substitué ou substitué par un alcoyle inférieur, $R_2$ et $R_3$ représentent indépendamment l'un de l'autre un hydrogène, alcoyle inférieur, halogène, trifuorométhyle, hydroxy, alcoxy inférieur ou alcoyle inférieur thio ou $R_2$ et $R_3$ ensemble, sur des atomes de carbone voisins, représentent un alcoylène inférieur dioxy;

A représente un alcoylène en $C_{4-12}$, un (alcoylèn-phénylène, alcoylène-thio-phénylène ou alcoylènoxy-phénylène) inférieur en $C_{7-10}$;

B représente un carboxy, alcoxy inférieur carbonyle, carbamoyle, cyano, hydroxy carbamoyle, 5-tétrazolyle ou hydroxyméthyle; où les groupes décrits comme "inférieurs" contiennent au plus 7 atomes de carbone, et leurs sels.

3. Composés de formule II selon la revendication 1

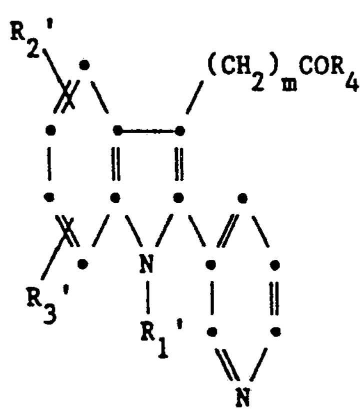

(II),

où $R_1'$ représente un hydrogène ou un alcoyle inférieur, $R_2'$ et $R_3'$ représentent indépendamment l'un de l'autre un hydrogène, alcoyle inférieur, halogène, trifluorométhyle, hydroxy, alcoyle inférieur thio ou alcoxy inférieur, ou $R_2'$ et $R_3'$ ensemble, sur des atomes de carbone voisins, représentent un méthylènedioxy, m représente un nombre entier allant de 4 à 12 et $R_4$ représente un hydroxy, alcoxy inférieur ou amino, où les groupes décrits comme "inférieurs" contiennent au plus 7 atomes de carbone et leurs sels pharmaceutiquement acceptables.

4. Composés de formule II selon la revendication 3, où $R_1'$ représente un hydrogène ou un alcoyle inférieur, $R_2'$ représente un hydrogène ou un halogène, $R_3'$ représente un hydrogène, m représente un nombre entier allant de 4 à 8 et $R_4$ représente un hydroxy, alcoxy inférieur ou amino, où les groupes décrits comme "inférieurs" contiennent au plus 7 atomes de carbone, et leurs sels pharmaceutiquement acceptables.

5. Composés de formule III selon la revendication 1

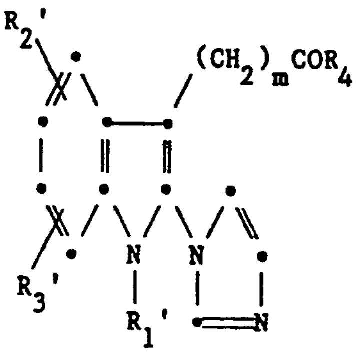

(III),

où $R_1$ représente un hydrogène ou un alcoyle inférieur, $R_2'$ et $R_3'$ représentent indépendamment l'un de l'autre un hydrogène, alcoyle inférieur, halogène, trifluorométhyle, hydroxy, alcoyle inférieur thio ou alcoxy inférieur, ou $R_2'$ et $R_3'$ ensemble, sur des atomes de carbone voisins, représentent un éthylènedioxy, m représente un nombre entier allant de 3 à 12 et $R_4$ représente un hydroxy, alcoxy inférieur ou amino, où les groupes décrits comme "inférieurs" contiennent au plus 7 atomes de carbone, et leurs sels pharmaceutiquement acceptables.

6. Composés de formule III selon la revendication 5, où $R_1'$ représente un hydrogène ou un alcoyle inférieur, $R_2'$ représente un hydrogène ou un halogène, $R_3'$ est un hydrogène, m représente un nombre entier allant de 4 à 8, et $R_4$ représente un hydroxy, alcoxy inférieur ou amino où les groupes décrits comme "inférieurs" contiennent au plus 7 atomes de carbone, et leurs sels pharmaceutiquement acceptables.

7. 3-(5-carboxypentyl)-2-(3-pyridyl)-indole et ses sels pharmaceutiquement acceptables selon la revendication 1.

8. 3-(5-carboxypentyl)-1-méthyl-2-(3-pyridyl)-indole et ses sels pharmaceutiquement acceptables selon la revendication 1.

9. 5-hydroxy-2-(3-pyridyl)-3-(5-carboxypentyl)-indole et ses sels pharmaceutiquement acceptables selon la revendication 1.

10. 3-(5-carboxypentyl)-5-chloro-2-(3-pyridyl)-indole et ses sels pharmaceutiquement acceptables selon la revendication 1.

11. 3-(5-carboxypentyl)-5-chloro-1-méthyl-2-(3-pyridyl)-indole et ses sels pharmaceutiquement acceptables selon la revendication 1.

12. 2-(1-imidazolyl)-3-(3-carboxypropyl)-indole et ses sels pharmaceutiquement acceptables selon la revendication 1.

13. 2-(1-imidazoyl)-3-(5-carboxypentyl)-indole et ses sels pharmaceutiquement acceptables selon la revendication 1.

14. Composé selon la revendication 1 et ses sels pharmaceutiquement acceptables aux fins d'application dans un procédé de traitement thérapeutique du corps humain et animal.

15. Préparations pharmaceutiques contenant des composés de formule I selon la revendication 1 ou leurs sels pharmaceutiquement acceptables.

16. Application de composés de formule I selon la revendication 1 et de leurs sels pharmaceutiquement acceptables à la préparation de préparations pharmaceutiques.

17. Composés de formule I selon la revendication 1 comme inhibiteurs de thromboxane-synthétase.

18. Procédé de préparation de composés de formule I selon la revendication 1, caractérisé en ce que

1) on fait réagir un composé de formule IV

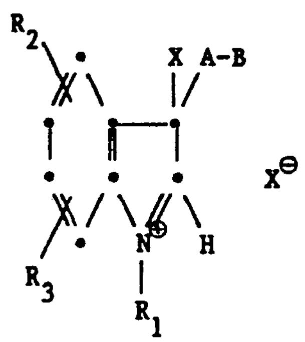

(IV),

où $R_1$, $R_2$, $R_3$, A et B ont la signification donnée sous la formule I et X représente un halogène, avec un

composé de formule Ar-H ou un de ses dérivés métallés réactifs, où Ar a la signification donnée sous la formule I, et, si on le désire, on transforme un composé de formule I obtenu où $R_1$ est un hydrogène, avec un agent alcoylant, en un composé de formule I où $R_1$ représente un alcoyle inférieur ou

2) on fait réagir un composé de formule V

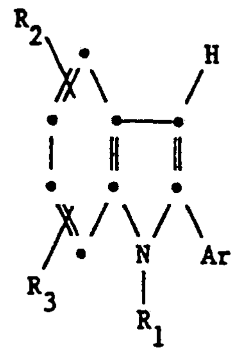

(V),

où $R_1$, $R_2$, $R_3$ et Ar ont la signification donnée sous la formule I comme dérivés organo-métalliques réactifs avec un dérivé fonctionnel réactif d'un composé de formule VI

HO—A—B (VI)

où A et B ont la signification donnée sous la formule I et, si on le désire, on transforme un composé de formule I obtenu où $R_1$ est un hydrogène avec un agent alcoylant en un composé de formule I où $R_1$ est un alcoyle inférieur ou

3) on cyclise un composé de formule VII

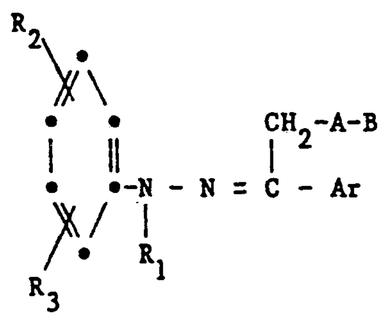

(VII),

où $R_1$, $R_2$, $R_3$, A et B ont la signification donnée sous la formule I et Ar représente un 3-pyridyle non substitué ou substitué comme il est dit sous la formule I, ou

4) on cyclise un composé de formule VIII

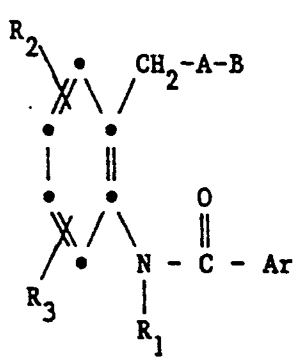

(VIII),

où Ar, $R_1$, $R_2$, $R_3$, A et B ont la signification donnée sous la formule I, ou

5) pour préparer un composé de formule I où A représente un alcénylène, on fait réagir dans les conditions d'une réaction de Wittig un composé de formule Va

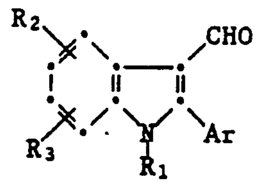

(Va),

qui correspond à un dérivé 3-formyle d'un composé de formule V avec l'ylure d'un composé de formule XII

$$R_5—A'—B \qquad (XII)$$

où B a la signification donnée sous la formule I, A' représente un alcoylène tel que défini ci-dessus pour A dans les composés de formule I, mais avec une longueur de chaîne raccourcie d'un atome de carbone, et $R_5$ représente un radical dialcoyle-phosphono ou triaryl-phosphonium, ou

6) pour préparer un composé de formule I où A représente un alcoyle inférieur-(thio ou oxy)-alcoylène inférieur ou alcoylène inférieur-(thio ou oxy)-phénylène, ou fait réagir un composé de formule Vb

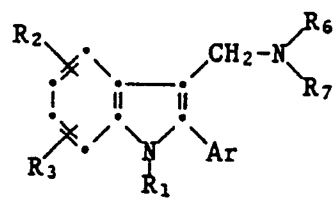

(Vb),

où Ar, $R_1$, $R_2$ et $R_3$ ont la signification donnée sous la formule I et $R_6$ et $R_7$ représentent chacun un alcoyle inférieur — donc un dérivé 3-(aminométhyle disubstitué) d'un composé de formule V — avec un composé de formule XIII

$$R_5—A''B \qquad (XIII)$$

ou un de ses dérivés de métal alcalin ou d'ammonium réactif, où B est défini comme il est dit sous la formule I, $R_5'$ représente un hydroxy ou un thiol et A'' représente un alcoylène inférieur ou phénylène; ou avec une lactone ou thiolactone d'un composé de formule XIII, où $R_5'$ représente un hydroxy ou un thiol, A'' représente un alcoylène inférieur et B un carboxy, ou,

7) on transforme en un composé de formule I un composé de formule Ia

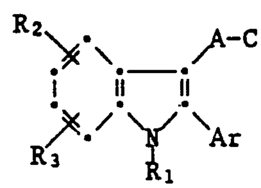

(Ia),

où A, Ar, $R_1$, $R_2$ et $R_3$ ont la signification donnée sous la formule I et C représente un groupe différent de B et transformable en B comme trialcoxyméthyle, hydroxyméthyle estérifié, hydroxyméthyle éthérifié, halogèneméthyle, 2-oxazolinyle, dihydro-2-oxazolinyle, alcanoyloxy, inférieur méthyle, acétyle, méthyle, carboxycarbonyl, trihalogènacétyle, di-alcoxy inférieur méthyle, alcoylènedioxy méthyle, vinyle, alcynyle, carboxy estérifié ou carboxy amidé, éventuellement avec allongement de la chaîne A dans le cadre de sa définition, ou un composé de formule I*, ou Ia* qui équivaut au composé de formule I ou Ia où cependant de manière différente A représente un alcoylène en $C_1$ ou $C_2$, avec allongement de la chaîne alcoylène A pour donner un alcoylène ayant un nombre d'atomes de carbone tel que défini sous la formule I; où les groupes réactifs gênants dans la molécule sont éventuellement temporairement protégés et/ou, si on le désire, on transforme un composé de formule I obtenu en un autre composé de l'invention, et/ou, si on le désire, on transforme un composé de formule I libre obtenu en un sel ou un sel obtenu en le composé libre ou en un autre sel, et/ou si on le désire, on dédouble un mélange d'isomères ou de racémates obtenu pour donner les isomères ou racémates isolés et/ou, sion le désire, on dédouble les racémates obtenus en les antipodes optiques.

**Revendications pour l'Etat contractant: AT**

1. Procédé de préparation de 2-(pyridyl et imidazolyl)-indoles de formule I

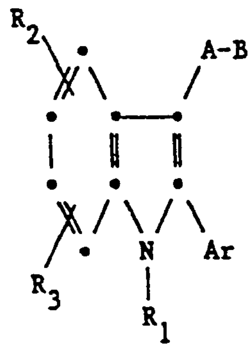

(I),

où $R_1$ représente un hydrogène ou un alcoyle inférieur, Ar représente à chaque fois un 3-pyridyle ou l-imidazolyle non substitué ou substitué par un alcoyle inférieur, carboxy, alcoxy inférieur carbonyle ou carbamoyle, $R_2$ et $R_3$ représentent indépendamment l'un de l'autre un hydrogène, alcoyle inférieur, halogène, trifluorométhyle, hydroxy, alcoxy inférieur, carboxyalcoyle inférieur, alcoxy inférieur carbonyle-alcoyle inférieur, carboxy, alcoxy inférieur carbonyle ou alcoyle inférieur-(thio, sulfinyle ou sulfonyle), ou $R_2$ et $R_3$ ensemble, sur les atomes de carbone voisins, représentent un alcoylène inférieur dioxy;

A représente un alcoylène en $C_{3-12}$, où le nombre des atomes de carbone situés entre le noyau indole et le groupe B s'élève à 3 à 12, un alcénylène ou un alcynylène avec à chaque fois de 2 à 12 atomes de carbone, un alcoyle inférieur phénylène-(alcoylène ou alcénylène) inférieur, un alcoylène inférieur -phénylène, un alcoylène inférieur-(thio ou oxy)-alcoylène inférieur, un alcoylène inférieur-(thio ou oxy)-phénylène ou un alcoylène inférieur phénylène -(thio ou oxy)-alcoylène inférieur et B représente un carboxy, carboxy estérifié sous forme d'esters pharmaceutiquement acceptables, un carbamoyle, un mono- ou dialcoyle inférieur carbamoyle, un hydroxyméthyle, un cyano, un hydroxycarbamoyle, un 5-tétrazolyle ou un formyle; leurs imidazolyl- ou pyridyl-N-oxydes où les groupes décrits comme "inférieurs" contiennent au plus 7 atomes de carbone, et leurs sels, caractérisé en ce que

1) on fait réagir un composé de formule IV

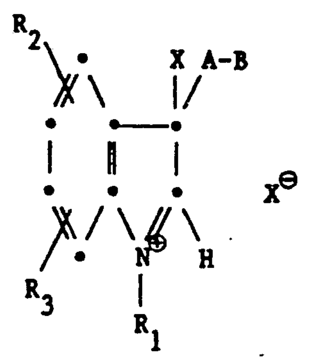

(IV),

où $R_1$, $R_2$, $R_3$, A et B ont la signification donnée sous la formule I et X représente un halogène, avec un composé de formule Ar-H ou un de ses dérivés métallés réactifs, où Ar a la signification donnée sous la formule I, et, si on le désire, on transforme un composé de formule I obtenu où $R_1$ est un hydrogène, avec un agent alcoylant, en un composé de formule I où $R_1$ représente un alcoyle inférieur ou

2) on fait réagir un composé de formule V

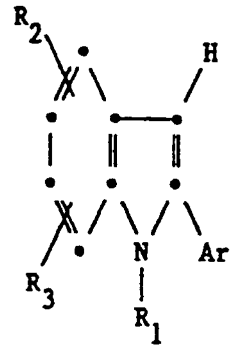

(V),

où $R_1$, $R_2$, $R_3$ et Ar ont la signification donnée sous la formule I comme dérivés organo-métalliques réactifs avec un dérivé fonctionnel réactif d'un composé de formule VI

HO—A—B (VI)

où A et B ont la signification donnée sous la formule I et, si on le désire, on transforme un composé de formule I obtenu où $R_1$ est un hydrogène avec un agent alcoylant en un composé de formule I où $R_1$ est un alcoyle inférieur ou

3) on cyclise un composé de formule VII

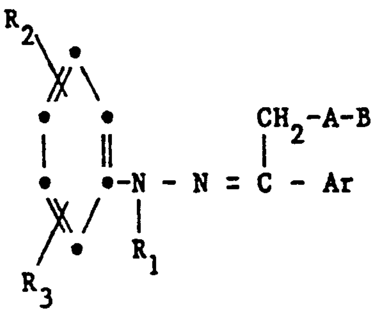

(VII),

où $R_1$, $R_2$, $R_3$, A et B ont la signification donnée sous la formule I et Ar représente un 3-pyridyle non substitué ou substitué comme il est dit sous la formule I, ou

4) on cyclise un composé de formule VIII

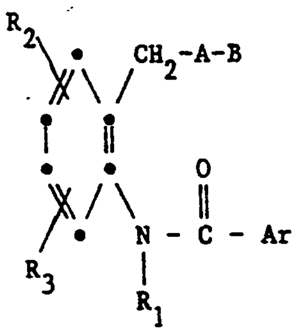

(VIII),

où Ar, $R_1$, $R_2$, $R_3$, A et B ont la signification donnée sous la formule I, ou

5) pour préparer un composé de formule I où A représente un alcénylène, on fait réagir dans les conditions d'une réaction de Wittig un composé de formule Va

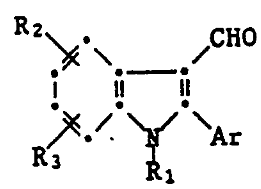

(Va),

qui correspond à un dérivé 3-formyle d'un composé de formule V avec l'ylure d'un composé de formule XII

$R_5$—A'—B (XII)

où B a la signification donnée sous la formule I, A' représente un alcoylène tel que défini ci-dessus pour A

dans les composés de formule I, mais avec une longueur de chaîne raccourcie d'une atome de carbone, et $R_5$ représente un radical dialcoyle-phosphono ou triaryl-phosphonium, ou

6) pour préparer un composé de formule I où A représente un alcoyle inférieur-(thio ou oxy)-alcoylène inférieur ou alcoylène inférieur-(thio ou oxy)-phénylène, ou fait réagir un composé de formule Vb

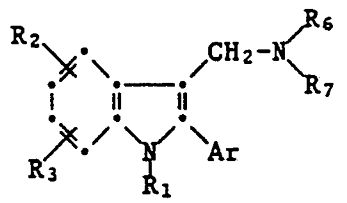
(Vb),

où Ar, $R_1$, $R_2$ et $R_3$ ont la signification donnée sous la formule I et $R_6$ et $R_7$ représentent chacun un alcoyle inférieur — donc un dérivé 3-(aminométhyle disubstitué) d'un composé de formule V — avec un composé de formule XIII

$$R_5'—A''—B \qquad (XIII)$$

ou un de ses dérivés de métal alcalin ou d'ammonium réactif, où B est défini comme il est dit sous la formule I, $R_5'$ représente un hydroxy ou un thiol et A'' représente un alcoylène inférieur ou phénylène; ou avec une lactone ou thiolactone d'un composé de formule XIII, où $R_5'$ représente un hydroxy ou un thiol, A'' représente un alcoylène inférieur et B un carboxy, ou,

7) on transforme en un composé de formule I un composé de formule Ia

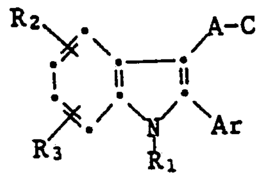
(Ia),

où A, Ar, $R_1$, $R_2$ et $R_3$ ont la signification donnée sous la formule I et C représente un groupe différent de B et transformable en B comme trialcoxyméthyle, hydroxyméthyle estérifié, hydroxyméthyle éthérifié, halogèneméthyle, 2-oxazolinyle, dihydro-2-oxazolinyle, alcanoyloxy, inférieur méthyle, acétyle, méthyle, carboxycarbonyl, trihalogènacétyle, di-alcoxy inférieur méthyle, alcoylènedioxy méthyle, vinyle, alcynyle, carboxy estérifié ou carboxy amidé, éventuellement avec allongement de la chaîne A dans le cadre de sa définition, ou un composé de formule I*, ou Ia* qui équivaut au composé de formule I ou Ia où cependant de manière différente A représente un alcoylène en $C_1$ ou $C_2$, avec allongement de la chaîne alcoylène A pour donner un alcoylène ayant un nombre d'atomes de carbone tel que défini sous la formule I; où les groupes réactifs gênants dans la molécule sont éventuellement temporairement protégés et/ou, si on le désire, on transforme un composé de formule I obtenu en un autre composé de l'invention, et/ou, si on le désire, on transforme un composé de formule I libre obtenu en un sel ou un sel obtenu en le composé libre ou en un autre sel, et/ou si on le désire, on dédouble un mélange d'isomères ou de racémates obtenu pour donner les isomères ou racémates isolés et/ou, sion le désire, on dédouble les racémates obtenus en les antipodes optiques.

2. Procédé selon la revendication 1, caractérisé en ce qu'on prépare les composés de formule I où $R_1$ représente un hydrogène ou un alcoyle inférieur, Ar représente à chaque fois un 3-pyridyle ou un 1-imidazolyle non substitué ou subsitué par un alcoyle inférieur, $R_2$ et $R_3$ représentent indépendamment l'un de l'autre un hydrogène, alcoyle inférieur, halogène, trifluorométhyle, hydroxy, alcoxy inférieur ou alcoyle inférieur-thio ou $R_2$ et $R_3$ ensemble, sur des atomes de carbone voisins, représentent un alcoylène inférieur dioxy;

A représente un alcoylène en $C_{4-12}$, un (alcoylène-phénylène, alcoylène-thio-phénylène ou alcoylèneoxy-phénylène) inférieur ayant de 7 à 10 atomes de carbone;

B représente un carboxy, alcoxy inférieur-carbonyle, carbamoyle, cyano, hydroxycarbamoyle, 5-tétrazolyle ou hydroxyméthyle, où les groupes décrits comme "inférieurs" contiennent au plus 7 atomes de carbone, et leurs sels.

3. Procédé selon la revendication 1, caractérisé en ce qu'on on prépare des composés de formule II

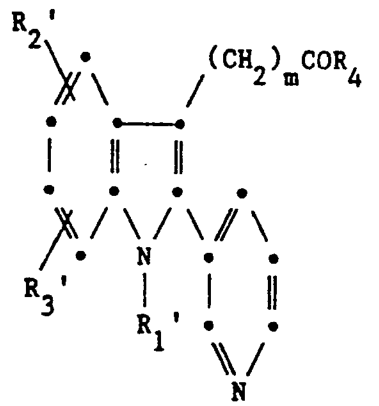

(II),

où $R_1'$ représente un hydrogène ou un alcoyle inférieur, $R_2'$ et $R_3'$ représentent indépendamment l'un de l'autre un hydrogène, alcoyle inférieur, halogène, trifluorométhyle, hydroxy, alcoyle inférieur, thio ou alcoxy inférieur, ou $R_2'$ et $R_3'$ ensemble, sur des atomes de carbone voisins, représentent un méthylènedioxy, m représente un nombre entier allant de 4 à 12 et $R_4$ représente un hydroxy, alcoxy inférieur ou amino, où les groupes décrits comme "inférieurs" contiennent au plus 7 atomes de carbone et leurs sels pharmaceutiquement acceptables.

4. Procédé selon la revendication 3, caractérisé en ce qu'on prépare des composés de formule II où $R_1'$ représente un hydrogène ou un alcoyle inférieur, $R_2'$ représente un hydrogène ou un halogène, $R_3'$ est un hydrogène, m représente un nombre entier allant de 4 à 8 et $R_4$ représente un hydroxy, alcoxy inférieur ou amino, où les groupes décrits comme "inférieurs" contiennent au plus 7 atomes de carbone, et leurs sels pharmaceutiquement acceptables.

5. Procédé selon la revendication 1, caractérisé en ce qu'on prépare des composés de formule III

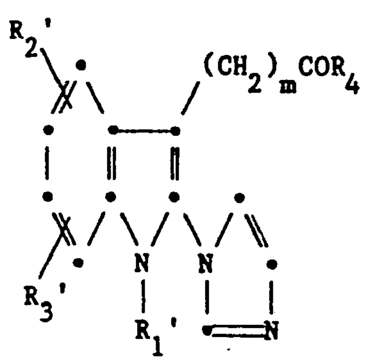

(III),

où $R_1'$ représente un hydrogène ou un alcoyle inférieur, $R_2'$ et $R_3'$ représentent indépendamment l'un de l'autre un hydrogène, alcoyle inférieur, halogène, trifluorométhyle, hydroxy, alcoyle inférieur thio ou alcoxy inférieur, ou $R_2'$ et $R_3'$ ensemble, sur des atomes de carbone voisins, représentent un éthylènedioxy, m représente un nombre entier allant de 3 à 12 et $R_4$ représente un hydroxy, alcoxy inférieur ou amino, où les groupes décrits comme "inférieurs" contiennent au plus 7 atomes de carbone, et leurs sels pharmaceutiquement acceptables.

6. Procédé selon la revendication 5, caractérisé en ce qu'on prépare les composés de formule III où $R_1'$ représente un hydrogène ou un alcoyle inférieur, $R_2'$ représente un hydrogène ou un halogène, $R_3'$ représente un hydrogène, m un nombre entier allant de 4 à 8 et $R_4$ un hydroxy, alcoxy inférieur ou amino, où les groupes décrits comme "inférieurs" contiennent au plus 7 atomes de carbone, et leurs sels pharmaceutiquement acceptables.

7. Procédé selon la revendication 1, caractérisé en ce qu'on prépare le 3-(5-carboxypentyl)-2-(3-pyridyl)-indole et ses sels pharmaceutiquement acceptables.

8. Procédé selon la revendication 1, caractérisé en ce qu'on prépare le 3-(5-carboxypentyl)-1-méthyl-2-(3-pyridyl)-indole et ses sels pharmaceutiquement acceptables

9. Procédé selon la revendication 1, caractérisé en ce qu'on prépare le 5-hydroxy-2-(3-pyridyl)-3-(5-carboxypentyl)-indole et ses sels pharmaceutiquement acceptables.

10. Procédé selon la revendication 1, caractérisé en ce qu'on prépare le 3-(5-carboxypentyl)-5-chloro-2-(3-pyridyl)-indole et ses sels pharmaceutiquement acceptables.

11. Procédé selon la revendication 1, caractérisé en ce qu'on prépare le 3-(5-carboxypentyl)-5-chloro-1-méthyl-2-(3-pyridyl)-indole et ses sels pharmaceutiquement acceptables.

12. Procédé selon la revendication 1, caractérisé en ce qu'on prépare le 2-(1-imidazoyl)-3-(3-carboxypropyl)-indole et ses sels pharmaceutiquement acceptables.

13. Procédé selon la revendication 1, caractérisé en ce qu'on prépare le 2-(1-imidazoyl)-3-(5-carboxypentyl)-indole et ses sels pharmaceutiquement acceptables.

14. Procédé de préparation de composés de formule I selon la revendication I, caractérisé en ce que

1) on fait réagir un composé de formule IV

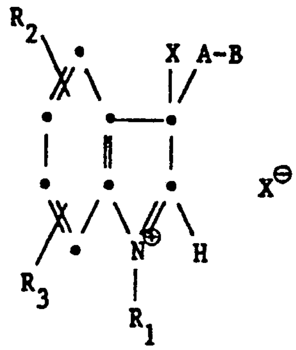

(IV),

où $R_1$, $R_2$, $R_3$, A et B ont la signification donnée sous la formule I et X représente un halogène, avec un composé de formule Ar-H ou un de ses dérivés métallés réactifs, où Ar a la signification donnée sous la formule I, et, si on le désire, on transforme un composé de formule I obtenu où $R_1$ est un hydrogène, avec un agent alcoylant, en un composé de formule I où $R_1$ représente un alcoyle inférieur ou

2) on fait réagir un composé de formule V

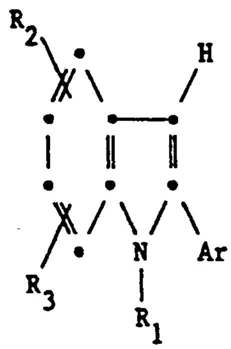

(V),

où $R_1$, $R_2$, $R_3$ et Ar ont la signification donnée sous la formule I comme dérivés organo-métalliques réactifs avec un dérivé fonctionnel réactif d'un composé de formule VI

HO—A—B (VI)

où A et B ont la signification donnée sous la formule I et, si on le désire, on transforme un composé de formule I obtenu où $R_1$ est un hydrogène avec un agent alcoylant en un composé de formule I où $R_1$ est un alcoyle inférieur ou

3) on cyclise un composé de formule VII

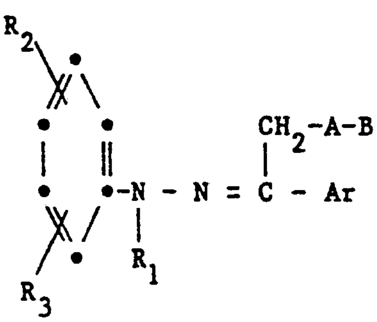

(VII),

où $R_1$, $R_2$, $R_3$, A et B ont la signification donnée sous la formule I et Ar représente un 3-pyridyle non substitué ou substitué comme il est dit sous la formule I, ou

4) on cyclise un composé de formule VIII

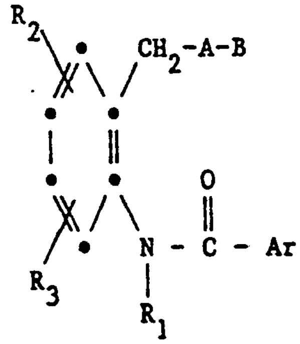

(VIII),

où Ar, $R_1$, $R_2$, $R_3$, A et B ont la signification donnée sous la formule I, ou

5) on transforme en un composé de formule I un composé de formule Ia

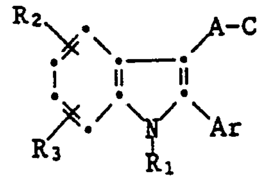

(Ia),

où A, Ar, $R_1$, $R_2$ et $R_3$ ont la signification donnée sous la formule I et C représente un groupe différent de B et transformable en B comme trialcoxyméthyle, hydroxyméthyle estérifié, hydroxyméthyle éthérifié, halogèneméthyle, 2-oxazolinyle, dihydro-2-oxazolinyle, alcanoyloxy, inférieur méthyle, acétyle, méthyle, carboxycarbonyl, trihalogènacétyle, di-alcoxy inférieur méthyle, alcoylènedioxy méthyle, vinyle, alcynyle, carboxy estérifié ou carboxy amidé, éventuellement avec allongement de la chaîne A dans le cadre de sa définition, ou un composé de formule I*, ou Ia* qui équivaut au composé de formule I ou Ia où cependant de manière différente A représente un alcoylène en $C_1$ ou $C_2$, avec allongement de la chaîne alcoylène A pour donner un alcoylène ayant un nombre d'atomes de carbone tel que défini sous la formule I; où les groupes reactifs gênants dans la molécule sont éventuellement temporairement protégés et/ou, si on le désire, on transforme un composé de formule I obtenu en un autre composé de l'invention, et/ou, si on le désire, on transforme un composé de formule I libre obtenu en un sel ou un sel obtenu en le composé libre ou en un autre sel, et/ou si on le désire, on dédouble un mélange d'isomères ou de racémates obtenu pour donner les isomères ou racémates isolés et/ou, si on le désire, on dédouble les racémates obtenus en les antipodes optiques et en ce qu'on transforme un composé libre de formule I obtenu selon le procédé décrit ci-dessus en un sel ou une sel obtenu en le composé libre et, si on le désire, en ce qu'on isole un isomère optique ou géométrique à partir d'un mélange d'isomères du composé de formule I obtenue.

15. Procédé de préparation de préparations pharmaceutiques caractérisé en ce qu'on mélange un composé de formule I obtenu selon le procédé de la revendication 1 ou un sel pharmaceutiquement acceptable d'un tel composé avec un support pharmaceutiquement acceptable.

16. Procédé de préparation de préparations pharmaceutiques, caractérisé en ce qu'on mélange un composé de formule I obtenu selon le procédé de la revendication 14 ou un sel pharmaceutiquement acceptable d'un tel composé avec un support pharmaceutiquement acceptable.

**Claims for the Contracting States: BE CH LI DE FR GB IT LU NL SE**

1. 2-(Pyridyl and imidazolyl)-indoles of the formula I

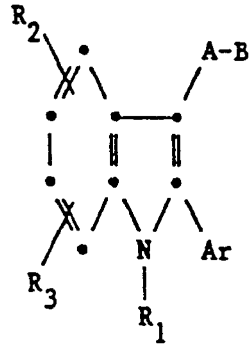

(I),

in which $R_1$ is hydrogen or lower alkyl, Ar is 3-pyridyl or 1-imidazolyl, each unsubstituted or substituted by

lower alkyl, carboxyl, lower alkoxycarbonyl or carbamoyl, $R_2$ and $R_3$ independently of one another are hydrogen, lower alkyl, halogen, trifluoromethyl, hydroxyl, lower alkoxy, carboxy lower alkyl, lower alkoxycarbonyl lower alkyl, carboxyl, lower alkoxycarbonyl or lower alkyl-(thio, sulfinyl or sulfonyl), or $R_2$ and $R_3$ together on adjacent carbon atoms are lower alkylenedioxy;

A is alkylene of 3 to 12 carbon atoms in which the number of the carbon atoms separating the indole nucleus from group B is 3 to 12, alkenylene or alkynylene each of 2 to 12 carbon atoms, lower alkylenephenylenelower (alkylene or alkenylene), lower alkylenephenylene, lower alkylene-(thio or oxy)-lower alkylene, lower alkylene-(thio or oxy)-phenylene or lower alkylenephenylene-(thio or oxy)-lower alkylene, and B is carboxyl, esterfied carboxyl in the form of pharmaceutically acceptable esters, carbamoyl, mono- or di-lower alkylcarbamoyl, hydroxymethyl, cyano, hydroxycarbamoyl, 5-tetrazolyl or formyl; imidazolyl or pyridyl N-oxides thereof, where the groups designated by "lower" contain up to and including 7 carbon atoms, and salts thereof.

2. Compounds of the formula I according to claim 1, in which $R_1$ is hydrogen or lower alkyl, Ar is 3-pyridyl or 1-imidazolyl each unsubstituted or substituted by lower alkyl, $R_2$ and $R_3$ independently of one another are hydrogen, lower alkyl, halogen, trifluoromethyl, hydroxyl, lower alkoxy or lower alkylthio, or $R_2$ and $R_3$ together on adjacent carbon atoms are lower alkylenedioxy;

A is alkylene of 4 to 12 carbon atoms, lower (alkylene-phenylene, alkylene-thio-phenylene or alkylene-oxy-phenylene) of 7 to 10 carbon atoms;

B is carboxyl, lower alkoxycarbonyl, carbamoyl, cyano, hydroxycarbamoyl, 5-tetrazolyl or hydroxymethyl; where the groups designated by "lower" contain up to and including 7 carbon atoms, and salts thereof.

3. Compounds of the formula II according to claim 1

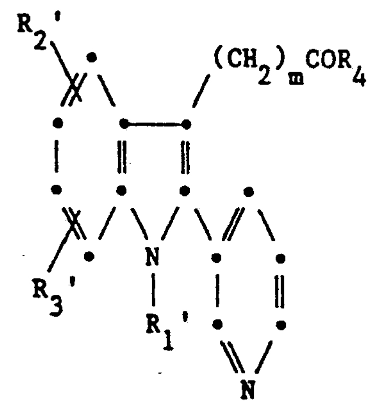

(II),

in which $R_1'$ is hydrogen or lower alkyl, $R_2'$ and $R_3'$ independently of one another are hydrogen, lower alkyl, halogen, trifluoromethyl, hydroxyl, lower alkylthio or lower alkoxy, or $R_2'$ and $R_3'$ together on adjacent carbon atoms are methylenedioxy, m is an integer from 4 to 12 and $R_4$ is hydroxyl, lower alkoxy or amino; where the groups designated by "lower" contain up to and including 7 carbon atoms, and pharmaceutically acceptable salts thereof.

4. Compounds of the formula II according to claim 3, in which $R_1'$ is hydrogen or lower alkyl, $R_2'$ is hydrogen or halogen, $R_3'$ is hydrogen, m is an integer from 4 to 8 and $R_4$ is hydroxyl, lower alkoxy or amino, where the groups designated by "lower" contain up to and including 7 carbon atoms, and pharmaceutically acceptable salts thereof.

5. Compounds of the formula III according to claim 1

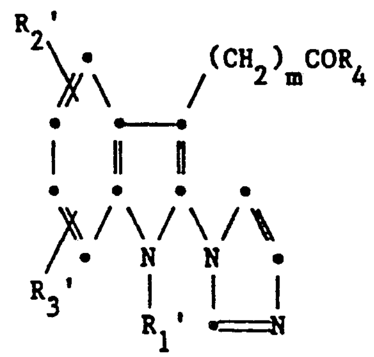

(III),

in which $R_1'$ is hydrogen or lower alkyl, $R_2'$ and $R_3'$ independently of one another are hydrogen, lower alkyl, halogen, trifluoromethyl, hydroxyl, lower alkylthio or lower alkoxy, or $R_2'$ and $R_3'$ together on adjacent carbon atoms are methylendioxy, m is an integer from 3 to 12 and $R_4$ is hydroxyl, lower alkoxy or amino,

where the groups designated by "lower" contain up to and including 7 carbon atoms, and pharmaceutically acceptable salts thereof.

6. Compounds of the formula II according to claim 5, in which $R_1'$ is hydrogen or lower alkyl, $R_2'$ is hydrogen or halogen, $R_3'$ is hydrogen, m is an integer from 4 to 8 and $R_4$ is hydroxyl, lower alkoxy or amino where the groups designated by "lower" contain up to and including 7 carbon atoms, and pharmaceutically acceptable salts thereof.

7. 3-(5-Carboxypentyl)-2-(3-pyridyl)-indole and pharmaceutically acceptable salts thereof according to claim 1.

8. 3-(5-Carboxypentyl)-1-methyl-2-(3-pyridyl)-indole and pharmaceutically acceptable salts thereof according to claim 1.

9. 5-Hydroxy-2-(3-pyridyl)-3-(5-carboxypentyl)-indole and pharmaceutically acceptable salts thereof according to claim 1.

10. 3-(5-Carboxypentyl)-5-chloro-2-(3-pyridyl)-indole and pharmaceutically acceptable salts thereof according to claim 1.

11. 3-(5-Carboxypentyl)-5-chloro-1-methyl-2-(3-pyridyl)-indole and pharmaceutically acceptable salts thereof according to claim 1.

12. 2-(1-Imidazolyl)-3-(3-carboxypropyl)-indole and pharmaceutically acceptable salts thereof according to claim 1.

13. 2-(1-Imidazolyl)-3-(5-carboxypentyl)-indole and pharmaceutically acceptable salts thereof according to claim 1.

14. A compound according to claim 1 and pharmaceutically acceptable salts thereof for use in a method for the therapeutic treatment of the human and animal body.

15. Pharmaceutical preparations containing compounds of the formula I according to claim 1 or pharmaceutically acceptable salts thereof.

16. Use of the compounds of the formula I according to claim 1 and of pharmaceutically acceptable salts thereof for the preparation of pharmaceutical preparations.

17. Compounds of the formula I according to claim 1 as thromboxane synthetase inhibitors.

18. Process for the preparation of compounds of the formula I according to claim 1, which comprises

1) reacting a compound of the formula IV

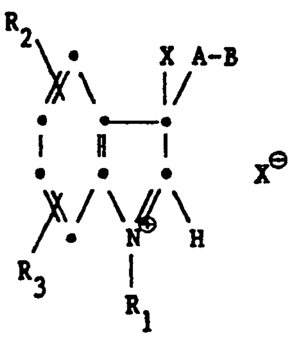

(IV),

in which $R_1$, $R_2$, $R_3$, A and B are as defined under formula I, and X is halogen, with a compound of the formula Ar-H or a reactive metallated derivative thereof, in which Ar is as defined under formula I, and, if desired, converting a resulting compound of the formula I in which $R_1$ is hydrogen by means of an alkylating agent into a compound of the formula I in which $R_1$ is lower alkyl, or

2) reacting a compound of the formula V

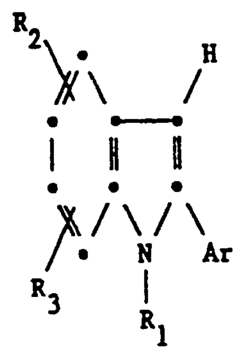

(V),

in which $R_1$, $R_2$, $R_3$ and Ar are as defined under formula I, as a reactive organometallic derivative with a reactive functional derivative of a compound of the formula VI

HO—A—B (VI)

in which A and B are as defined under formula I, and, if desired, converting a resulting compound of the

formula I in which $R_1$ is hydrogen by means of an alkylating agent into a compound of the formula I in which $R_1$ is lower alkyl, or

3) ring-closing a compound of the formula VII

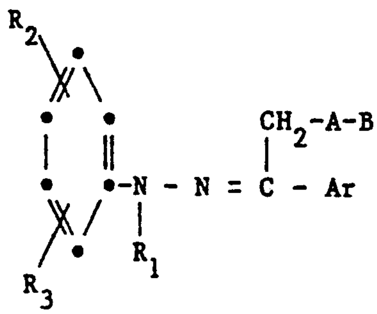

(VII),

in which $R_1$, $R_2$, $R_3$, A and B are as defined under formula I and Ar is 3-pyridyl, unsubstituted or substituted as defined under formula I, or

4) cyclizing a compound of the formula VIII

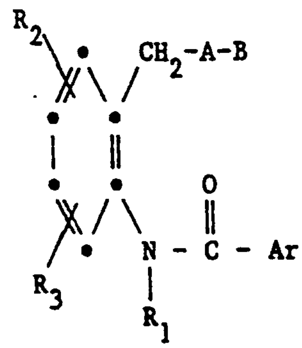

(VIII),

in which Ar, $R_1$, $R_2$, $R_3$, A and B are as defined under formula I, or

5) for the preparation of a compound of the formula I in which A is alkenylene, reaction under the conditions of a Wittig reaction a compound of the formula Va

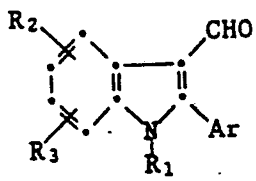

(Va),

which is a 3-formyl derivative of a compound of the formula V, with the ylide of a compound of the formula XII

$$R_5—A'—B \qquad \text{(XII)}$$

in which B is as defined under formula I, A' is alkylene as defined above for A in compounds of the formula I but with the chain length shortened by 1 carbon atom, and $R_5$ is a dialkylphosphono or tiraryIphosphonium radical, or

6) for the preparation of a compound of the formula I, in which A is lower alkyl-(thio or oxy)-lower alkylene or lower alkylene-(thio or oxy)-phenylene, reacting a compound of the formula Vb

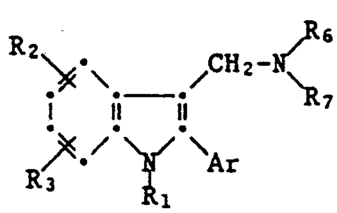

(Vb),

in which Ar, $R_1$, $R_2$ and $R_3$ are as defined under formula I, and $R_6$ and $R_7$ are each lower alkyl — i.e. a 3-(disubstituted aminomethyl) derivative of a compound of the formula V — with a compound of the formula XIII

$$R_5'—A''—B \quad (XIII)$$

or a reactive alkali metal or ammonium derivative thereof, in which B is as defined under formula I, $R_5'$ is hydroxyl or thiol and A'' is lower alkylene or phenylene; or with a lactone or thiolactone of a compound of the formula XIII, in which $R_5'$ is hydroxyl or thiol, A'' is lower alkylene and B is carboxyl, or

7) converting a compound of the formula Ia

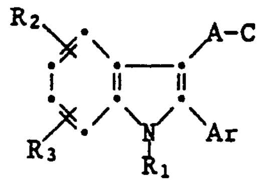

(Ia),

in which A, Ar, $R_1$, $R_2$ and $R_3$ are as defined under formula I and C is a group differing from B and convertible into B, such as trialkoxymethyl, esterified hydroxymethyl, etherified hydroxymethyl, halomethyl, 2-oxazolinyl, dihydro-2-oxazolinyl, lower alkanoyloxymethyl, acetyl, methyl, carboxycarbonyl, trihaloacetyl, di-lower alkoxymethyl, alkylenedioxymethyl, vinyl, alkynyl, esterfied carboxyl or amidated carboxyl, if appropriate with extension of the chain A within the scope of its definition, or a compound of the formula I* or Ia* which is identical to that of the formula I or Ia respectively, but in which A is alkylene of 1 or 2 carbon atoms, with extension of the alkylene chain A to alkylene having the number of carbon atoms as defined under formula I, into a compound of the formula I; interfering reactive groups in the molecule being optionally temporarily protected, and/or, if desired, converting a resulting compound of the formula I into another compound of the invention, and/or, if desired, converting a resulting free compound of the formula I into a salt or a resulting salt into the free compound or into another salt, and/or, if desired, resolving a resulting mixture of isomers or racemates into the individual isomers of racemates, and/or, if desired, resolving racemates obtained into the optical antipodes.

**Claims for the Contracting State: AT**

1. Process for the preparation of 2-(pyridyl and imidazolyl)-indoles of the formula I

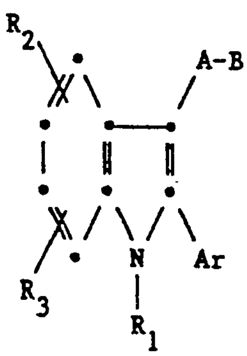

(I),

in which $R_1$ is hydrogen or lower alkyl, Ar is 3-pyridyl or 1-imidazolyl, each unsubstituted or substituted by lower alkyl, carboxyl, lower alkoxycarbonyl or carbamoyl, $R_2$ and $R_3$ independently of one another are hydrogen, lower alkyl, halogen, trifluoromethyl, hydroxyl, lower alkoxy, carboxy lower alkyl, lower alkoxycarbonyl lower alkyl, carboxyl, lower alkoxycarbonyl or lower alkyl-(thio, sulfinyl or sulfonyl), or $R_2$ and $R_3$ together on adjacent carbon atoms are lower alkylenedioxy;

A is alkylene of 3 to 12 carbon atoms in which the number of the carbon atoms separating the indole nucleus from group B is 3 to 12, alkenylene or alkynylene each of 2 to 12 carbon atoms, lower alkylenephenylenelower (alkylene or alkenylene), lower alkylenephenylene, lower alkylene-(thio or oxy)-lower alkylene, lower alkylene-(thio or oxy)-phenylene or lower alkylenephenylene-(thio or oxy)-lower alkylene, and B is carboxyl, esterfied carboxyl in the form of pharmaceutically acceptable eters, carbamoyl, mono- or di-lower alkylcarbamoyl, hydroxymethyl, cyano, hydroxycarbamoyl, 5-tetrazolyl or formyl; imidazolyl or pyridyl N-oxides thereof, where the groups designated by "lower" contain up to and including 7 carbon atoms, and salts thereof, which comprises

1) reacting a compound of the formula IV

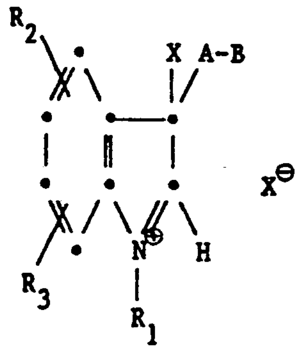

(IV),

in which $R_1$, $R_2$, $R_3$, A and B are as defined under formula I, and X is halogen, with a compound of the formula Ar-H or a reactive metallated derivative thereof, in which Ar is as defined under formula I, and, if desired, converting a resulting compound of the formula I in which $R_1$ is hydrogen by means of an alkylating agent into a compound of the formula I in which $R_1$ is lower alkyl, or

2) reacting a compound of the formula V

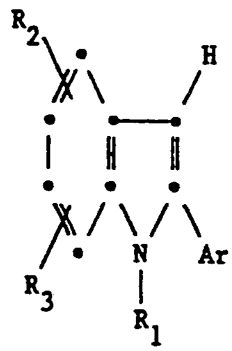

(V),

in which $R_1$, $R_2$, $R_3$ and Ar are as defined under formula I, as a reactive organometallic derivative with a reactive functional derivative of a compound of the formula VI

HO—A—B (VI)

in which A and B are as defined under formula I, and, if desired, converting a resulting compound of the formula I in which $R_1$ is hydrogen by means of an alkylating agent into a compound of the formula I in which $R_1$ is lower alkyl, or

3) ring-closing a compound of the formula VII

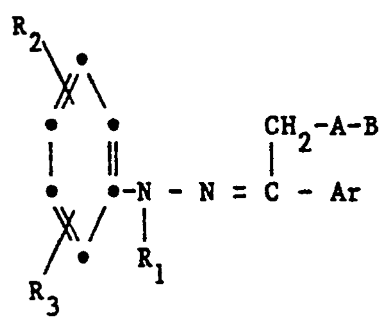

(VII),

in which $R_1$, $R_2$, $R_3$, A and B are as defined under formula I and Ar is 3-pyridyl, unsubstituted or substituted as defined under formula I, or

4) cyclizing a compound of the formula VIII

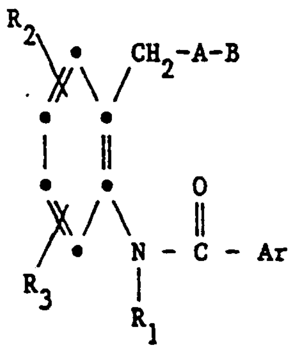

(VIII),

in which Ar, $R_1$, $R_2$, $R_3$, A and B are as defined under formula I, or

5) for the preparation of a compound of the formula I in which A is alkenylene, reaction under the conditions of a Wittig reaction a compound of the formula Va

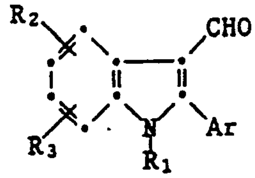

(Va),

which is a 3-formyl derivative of a compound of the formula V, with the ylide of a compound of the formula XII

$$R_5—A'—B \qquad (XII)$$

in which B is as defined under formula I, A′ is alkylene as defined above for A in compounds of the formula I but with the chain length shortened by 1 carbon atom, and $R_5$ is a dialkylphosphono or triarylphosphonium radical, or

6) for the preparation of a compound of the formula I, in which A is lower alkyl-(thio or oxy)-lower alkylene or lower alkylene-(thio or oxy)-phenylene, reacting a compound of the formula Vb

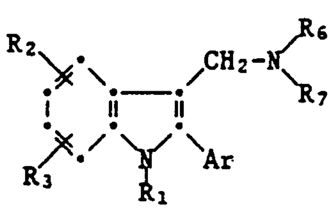

(Vb),

in which Ar, $R_1$, $R_2$ and $R_3$ are as defined under formula I, and $R_6$ and $R_7$ are each lower alkyl — i.e. a 3-(disubstituted aminomethyl) derivative of a compound of the formula V — with a compound of the formula XIII

$$R_5'—A''—B \qquad (XIII)$$

or a reactive alkali metal or ammonium derivative thereof, in which B is as defined under formula I, $R_5'$ is hydroxyl or thiol and A″ is lower alkylene or phenylene; or with a lactone or thiolactone of a compound of the formula XIII, in which $R_5'$ is hydroxyl or thiol, A″ is lower alkylene and B is carboxyl, or

7) converting a compound of the formula Ia

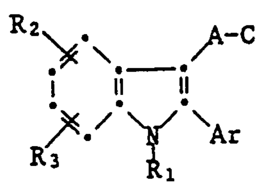

(Ia),

in which A, Ar, $R_1$, $R_2$ and $R_3$ are as defined under formula I and C is a group differing from B and convertible

into B, such as trialkoxymethyl, esterified hydroxymethyl, etherified hydroxymethyl, halomethyl, 2-oxazolinyl, dihydro-2-oxazolinyl, lower alkanoyloxymethyl, acetyl, methyl, carboxycarbonyl, trihaloacetyl, di-lower alkoxymethyl, alkylenedioxymethyl, vinyl, alkynyl, esterfied carboxyl or amidated carboxyl, if appropriate with extension of the chain A within the scope of its definition, or a compound of the formula I* or Ia* which is identical to that of the formula I or Ia respectively, but in which A is alkylene of 1 or 2 carbon atoms, with extension of the alkylene chain A to alkylene having the number of carbon atoms as defined under formula I, into a compound of the formula I; interfering reactive groups in the molecule being optionally temporarily protected, and/or, if desired, converting a resulting compound of the formula I into another compound of the invention, and/or, if desired, converting a resulting free compound of the formula I into a salt or a resulting salt into the free compound or into another salt, and/or, if desired, resolving a resulting mixture of isomers or racemates into the individual isomers of racemates, and/or, if desired, resolving racemates obtained into the optical antipodes.

2. Process according to claim 1, which comprises preparing compounds of the formula I, in which $R_1$ is hydrogen or lower alkyl, Ar is 3-pyridyl or 1-imidazolyl each unsubstituted or substituted by lower alkyl, $R_2$ and $R_3$ independently of one another are hydrogen, lower alkyl, halogen, trifluoromethyl, hydroxyl, lower alkoxy or lower alkylthio, or $R_2$ and $R_3$ together on adjacent carbon atoms are lower alkylenedioxy;

A is alkylene of 4 to 12 carbon atoms, lower (alkylene-phenylene, alkylene-thio-phenylene or alkylene-oxy-phenylene) of 7 to 10 carbons atoms;

B is carboxyl, lower alkoxycarbonyl, carbamoyl, cyano, hydroxycarbamoyl, 5-tetrazolyl or hydroxymethyl; where the groups designated by "lower" contain up to and including 7 carbon atoms, and salts thereof.

3. Process according to claim 1, which comprises preparing compounds of the formula II

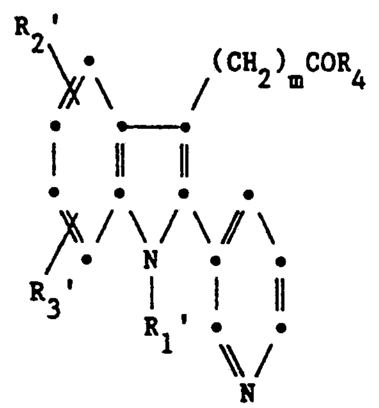

(II),

in which $R_1'$ is hydrogen or lower alkyl, $R_2'$ and $R_3'$ independently of one another are hydrogen, lower alkyl, halogen, trifluoromethyl, hydroxyl, lower alkylthio or lower alkoxy, or $R_2'$ and $R_3'$ together on adjacent carbon atoms are methylenedioxy, m is an integer from 4 to 12 and $R_4$ is hydroxyl, lower alkoxy or amino; where the groups designated by "lower" contain up to and including 7 carbon atoms, and pharmaceutically acceptable salts thereof.

4. Process according to claim 3, which comprises preparing compounds of the formula II, in which $R_1'$ is hydrogen or lower alkyl, $R_2'$ is hydrogen or halogen, $R_3'$ is hydrogen, m is an integer from 4 to 8 and $R_4$ is hydroxyl, lower alkoxy or amino, where the groups designated by "lower" contain up to and including 7 carbon atoms, and pharmaceutically acceptable salts thereof.

5. Process according to claim 1, which comprises preparing compounds of the formula III

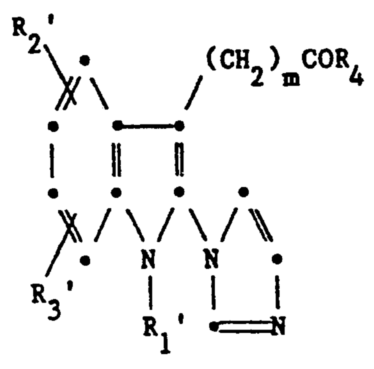

(III),

in which $R_1'$ is hydrogen or lower alkyl, $R_2'$ and $R_3'$ independently of one another are hydrogen, lower alkyl, halogen, trifluoromethyl, hydroxyl, lower alkylthio or lower alkoxy, or $R_2'$ and $R_3'$ together on adjacent

carbon atoms are methylenedioxy, m is an integer from 3 to 12 and $R_4$ is hydroxyl, lower alkoxy or amino, where the groups designated by "lower" contain up to and including 7 carbon atoms, and pharmaceutically acceptable salts thereof.

6. Process according to claim 5, which comprises preparing compounds of the formula III, in which $R_1'$ is hydrogen or lower alkyl, $R_2'$ is hydrogen or halogen, $R_3'$ is hydrogen, m is an integer from 4 to 8 and $R_4$ is hydroxyl, lower alkoxy or amino where the groups designated by "lower" contain up to and including 7 carbon atoms, and pharmaceutically acceptable salts thereof.

7. Process according to claim 1, which comprises preparing 3-(5-carboxypentyl)-2-(3-pyridyl)-indole and pharmaceutically acceptable salts thereof.

8. Process according to claim 1, which comprises preparing 3-(5-carboxypentyl)-1-methyl-2-(3-pyridyl)-indole and pharmaceutically acceptable salts thereof.

9. Process according to claim 1, which comprises preparing 5-hydroxy-2-(3-pyridyl)-3-(5-carboxypentyl)-indole and pharmaceutically acceptable salts thereof.

10. Process according to claim 1, which comprises preparing 3-(5-carboxypentyl)-5-chloro-2-(3-pyridyl)-indole and pharmaceutically acceptable salts thereof.

11. Process according to claim 1, which comprises preparing 3-(5-carboxypentyl)-5-chloro-1-methyl-2-(3-pyridyl)-indole and pharmaceutically acceptable salts thereof.

12. Process according to claim 1, which comprises preparing 2-(1-imidazolyl)-3-(3-carboxypropyl)-indole and pharmaceutically acceptable salts thereof.

13. Process according to claim 1, which comprises preparing 2-(1-imidazolyl)-3-(5-carboxypentyl)-indole and pharmaceutically acceptable salts thereof.

14. Process for the preparation of compounds of the formula I according to claim 1, which comprises

1) reacting a compound of the formula IV

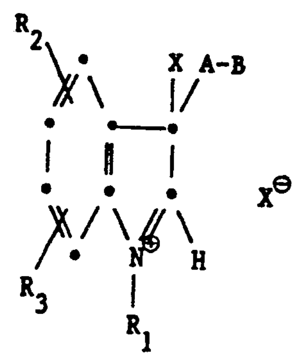

(IV),

in which $R_1$, $R_2$, $R_3$, A and B are as defined under formula I, and X is halogen, with a compound of the formula Ar-H or a reactive metallated derivative thereof, in which Ar is as defined under formula I, and, if desired, converting a resulting compound of the formula I in which $R_1$ is hydrogen by means of an alkylating agent into a compound of the formula I in which $R_1$ is lower alkyl, or

2) reacting a compound of the formula V

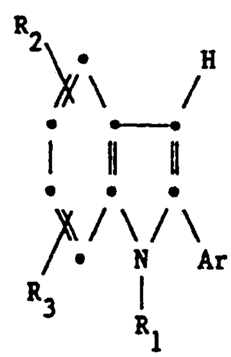

(V),

in which $R_1$, $R_2$, $R_3$ and Ar are as defined under formula I, as a reactive organometallic derivative with a reactive functional derivative of a compound of the formula VI

$$HO—A—B \qquad (VI)$$

in which A and B are as defined under formula I, interfering reactive groups in the molecule being optionally temporarily protected, and, if desired, converting a resulting compound of the formula I in which

$R_1$ is hydrogen by means of an alkylating agent into a compound of the formula I in which $R_1$ is lower alkyl, or

3) ring-closing a compound of the formula VII

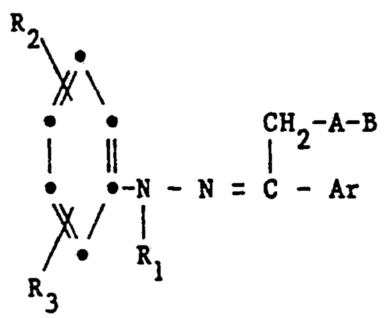

(VII),

in which $R_1$, $R_2$, $R_3$, A and B are as defined under formula I and Ar is 3-pyridyl, unsubstituted or substituted as defined under formula I, or

4) cyclizing a compound of the formula VIII

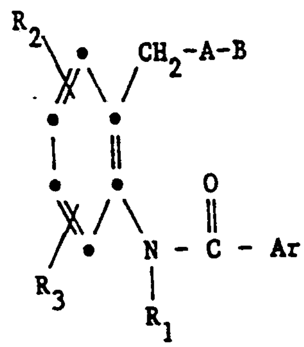

(VIII),

in which Ar, $R_1$, $R_2$, $R_3$, A and B are as defined under formula I and Ar is 3-pyridyl, unsubstituted or substituted as defined under formula I, or

5) converting a compound of the formula Ia

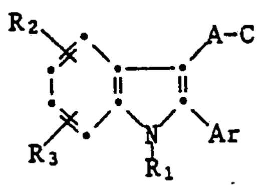

(Ia),

in which A, Ar, $R_1$, $R_2$ and $R_3$ are as defined under formula I and C is a group differing from B and convertible into B, such as trialkoxymethyl, esterified hydroxymethyl, etherified hydroxymethyl, halomethyl, 2-oxazolinyl, dihydro-2-oxazolinyl, lower alkanoyloxymethyl, acetyl, methyl, carboxycarbonyl, trihaloacetyl, di-lower alkoxymethyl, alkylenedioxymethyl, vinyl, alkynyl, esterified carboxyl or amidated carboxyl, into the compound of the formula I; and converting a compound of the formula I obtained according to the process described above into another compound of the invention, and a free compound of the formula I obtained according to the process described above into a salt or a resulting salt into the free compound, and, if desired, isolating an optical or geometrical isomer from a mixture of isomers of the resulting compound of the formula I.

15. Process for the preparation of pharmaceutical preparations, which comprises mixing a compound of the formula I obtained in accordance with the process according to claim 1 or a pharmaceutically acceptable salt of such a compound with a pharmaceutically acceptable carrier.

16. Process for the preparation of pharmaceutical preparations, which comprises mixing a compound of the formula I obtained in accordance with the process according to claim 14 or a pharmaceutically acceptable salt of such a compound with a pharmaceutically acceptable carrier.